(19) Europäisches Patentamt
European Patent Office
Office européen des brevets

(11) EP 2 992 032 B1

(12) EUROPEAN PATENT SPECIFICATION

(45) Date of publication and mention of the grant of the patent:
**06.12.2017 Bulletin 2017/49**

(21) Application number: **14721328.4**

(22) Date of filing: **29.04.2014**

(51) Int Cl.:
***C08G 64/30*** (2006.01) ***C07D 317/36*** (2006.01)

(86) International application number:
**PCT/EP2014/058716**

(87) International publication number:
**WO 2014/177558 (06.11.2014 Gazette 2014/45)**

(54) **PROCESS FOR PREPARING POLYCARBONATES BY POLYMERIZATION OF FIVE-MEMBERED-RING CYCLIC CARBONATES**

VERFAHREN ZUR HERSTELLUNG VON POLYCARBONATEN DURCH POLYMERISATION VON CYCLISCHEN CARBONATEN MIT FÜNFGLIEDRIGEM RING

PROCÉDÉ DE PRÉPARATION DE POLYCARBONATES PAR POLYMÉRISATION DE CARBONATES CYCLIQUES À NOYAU PENTAGONAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.04.2013 EP 13290095**

(43) Date of publication of application:
**09.03.2016 Bulletin 2016/10**

(73) Proprietors:
- **Total Research & Technology Feluy**
  **7181 Seneffe (BE)**
- **Centre National de la Recherche Scientifique (C.N.R.S.)**
  **75016 Paris (FR)**

(72) Inventors:
- **CARPENTIER, Jean-françois**
  **F-35690 Acigne (FR)**
- **GUILLAUME, Sophie**
  **F-35500 Vitré (FR)**
- **GUERIN, William**
  **F-35000 Rennes (FR)**

(74) Representative: **Raboin, Jean-Christophe et al**
**Total Research & Technology Feluy**
**Zone Industrielle C**
**7181 Seneffe (BE)**

(56) References cited:
**WO-A1-2012/038240 WO-A1-2012/160042**
**JP-A- 2011 084 512 US-A- 5 003 084**
**US-A- 5 733 860 US-A1- 2012 101 233**

- **GUANG-PENG WU ET AL: "Enhanced Asymmetric Induction for the Copolymerization of CO 2 and Cyclohexene Oxide with Unsymmetric Enantiopure SalenCo(III) Complexes: Synthesis of Crystalline CO 2 -Based Polycarbonate", JOURNAL OF THE AMERICAN CHEMICAL SOCIETY, vol. 134, no. 12, 28 March 2012 (2012-03-28), pages 5682-5688, XP055078716, ISSN: 0002-7863, DOI: 10.1021/ja300667y**
- **DATABASE CA [Online] CHEMICAL ABSTRACTS SERVICE, COLUMBUS, OHIO, US; LV, XIAOBING ET AL: "Crystallizable syndiotactic carbon dioxide-based polycarbonate material and its preparation method", XP002712690, retrieved from STN Database accession no. 155:616247 & CN 102 229 745 A (DALIAN UNIVERSITY OF TECHNOLOGY, PEOP. REP. CHINA) 2 November 2011 (2011-11-02)**
- **DATABASE CA [Online] CHEMICAL ABSTRACTS SERVICE, COLUMBUS, OHIO, US; KUSUNOKI, JUNYA ET AL: "Temporarily fixing agents for organic substrates, and semiconductor devices and their manufacture", XP002712691, retrieved from STN Database accession no. 155:382111 -& JP 2011 168663 A (SUMITOMO BAKELITE CO., LTD., JAPAN) 1 September 2011 (2011-09-01)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PIERRE BRIGNOU ET AL: "Polycarbonates Derived from Green Acids: Ring-Opening Polymerization of Seven-Membered Cyclic Carbonates", MACROMOLECULES, vol. 43, no. 19, 12 October 2010 (2010-10-12), pages 8007-8017, XP055132404, ISSN: 0024-9297, DOI: 10.1021/ma1014098**
- **MILOUD BOUYAHYI ET AL: "Exploring Electronic versus Steric Effects in Stereoselective Ring-Opening Polymerization of Lactide and [beta]-Butyrolactone with Amino-alkoxy-bis(phenolate)-Yttrium Complexes", CHEMISTRY - A EUROPEAN JOURNAL, vol. 17, no. 6, 7 February 2011 (2011-02-07), pages 1872-1883, XP055132414, ISSN: 0947-6539, DOI: 10.1002/chem.201002779**
- **JOHN W. KRAMER ET AL: "Polymerization of Enantiopure Monomers Using Syndiospecific Catalysts: A New Approach To Sequence Control in Polymer Synthesis", JOURNAL OF THE AMERICAN CHEMICAL SOCIETY, vol. 131, no. 44, 11 November 2009 (2009-11-11), pages 16042-16044, XP055132411, ISSN: 0002-7863, DOI: 10.1021/ja9075327**


Remarks:

The file contains technical information submitted after the application was filed and not included in this specification

## Description

### FIELD OF THE INVENTION

The invention relates to a process for the preparation of polycarbonates or copolymers thereof. In particular the invention relates to a process for the preparation of polycarbonates, or copolymers thereof, by ring opening polymerization of five-membered ring cyclic carbonates.

### BACKGROUND OF THE INVENTION

Polycarbonates are becoming more important as alternatives to synthetic polymers. There already exist commercially available polycarbonates with excellent electrical insulating properties, transparency, and heat resistance and polycarbonates are present in many products, like adhesives for compact discs, in packaging, and optical lenses, to name a few.

One of the most widely used polycarbonates is bisphenol A polycarbonate (BPA-PC), which has interesting thermal and mechanical properties but has recently been reported as being a potential hazard in food / human contact applications. Other commercially available polycarbonates such as propylene polycarbonate (PPC), ethylene polycarbonate (PEC), poly(butylene carbonate) (PBC), or poly(trimethylene carbonate) (PTMC), while presenting particular thermo-mechanical properties, still do not provide a durable and attractive alternative, especially as commodity polymers for packaging or as elastomers.

One alternative to bisphenol A polycarbonate is poly(cyclohexene carbonate) (PCHC). PCHC has a melting temperature of 115 °C, lower than that of BPA-PC (150 °C) and a degradation temperature of ca. 310 °C, similar to that of BPA-PC (300 °C). It has an elongation at break ($\varepsilon_r$) of 2% and a Young's modulus (E) of 3500 MPa, significantly higher than that of BPA-PC (2400 MPa). Due to these properties, PCHC is a promising replacer for BPA-PC.

Since the late 90s different groups have synthesized PCHC by copolymerizing $CO_2$ with cyclohexene oxide. This synthesis presents problems in controlling the stereochemistry and the chemoselectivity of the PCHC, such that an ether-free polycarbonate sequence is difficult to obtain.

Consequently, it is an object of the present invention to provide an improved process for the polymerization of five-membered cyclic carbonates. It is also an object of the present invention to provide an improved process for the copolymerization of five-membered cyclic carbonates with cyclic esters. It is an object of the present invention to provide novel polycarbonates, particularly poly(cyclic carbonates), more particularly PCHC, with improved mechanical properties.

### SUMMARY OF THE INVENTION

The present inventors have now found that any one of the objects above can be attained by using the processes as presently claimed.

According to a first aspect of the present invention, a process for preparing a polycarbonate or a copolymer thereof is provided. The process for preparing a polycarbonate or a copolymer thereof comprises the step of homo- or copolymerizing in the presence of at least one catalyst, one or more compounds of formula (I), stereoisomers, racemics, or mixtures thereof; optionally with one or more cyclic esters, stereoisomers, racemics, or mixtures thereof;

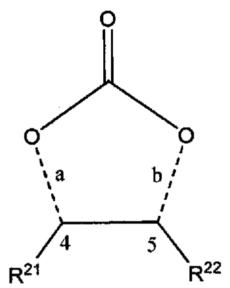 (I)

wherein

each dotted bond "**a**" and "**b**" independently represents a solid bond, a wedged bond, or a hashed wedged bond; each $\mathbf{R^{21}}$ and $\mathbf{R^{22}}$ are, independently, selected from $C_{1\text{-}6}$alkyl, $C_{3\text{-}6}$cycloalkyl, and $C_{6\text{-}10}$aryl; or $\mathbf{R^{21}}$ and $\mathbf{R^{22}}$ taken together with the carbon atoms 4 and 5 of the cyclic carbonate form a ring selected from $C_{3\text{-}9}$cycloalkylene, $C_{5\text{-}9}$cycloalkenylene, and $C_{3\text{-}12}$arylene, said ring being optionally substituted with one or more substituents each

independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

Preferably, the present invention provides a process for preparing a polycarbonate or copolymer thereof, said process comprising copolymerizing in the presence of at least one catalyst, one or more compounds of formula (I), stereoisomers, racemics, or mixtures thereof; with one or more cyclic esters, stereoisomers, racemics, or mixtures thereof;

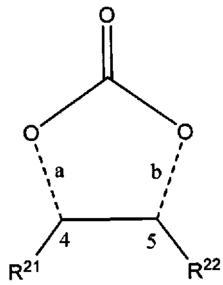
(I)

wherein

each dotted bond "**a**" and "**b**" independently represents a solid bond, a wedged bond, or a hashed wedged bond; and **$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{3-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; wherein two of said one or more substituents can join to form a ring fused to said $C_{3-9}$cycloalkylene, $C_{5-9}$cycloalkenylene, or $C_{6-12}$arylene; wherein said ring formed by said two substituents is selected from the group comprising $C_{3-9}$cycloalkyl, $C_{5-9}$cycloalkenyl, $C_{3-12}$aryl, and heterocyclyl, wherein each group can be unsubstituted or substituted with one or more $R^{40}$, wherein each **$R^{40}$** is independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

More preferably, the present invention provides a process for preparing a polycarbonate or copolymer thereof, said process comprising homo- or copolymerizing in the presence of at least one catalyst, one or more compounds of formula (I), stereoisomers, racemics, or mixtures thereof; optionally with one or more cyclic esters, stereoisomers, racemics, or mixtures thereof;

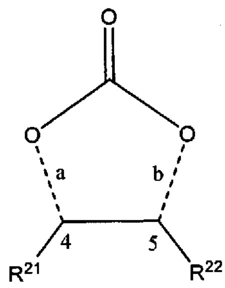
(I)

wherein

each dotted bond "**a**" and "**b**" independently represents a solid bond, a wedged bond, or a hashed wedged bond; and **$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

According to a second aspect, the present invention further encompasses a process for the synthesis of a compound of formula (I) as defined above, a stereoisomer or racemate thereof, comprising the step of contacting a

compound of formula (V), a stereoisomer, or racemate thereof; with at least one reagent selected from the group consisting of an alkyl- or cycloalkyl-chloroformate derivative of formula (VI); an urea; phosgene or a derivative/substitute of phosgene, e.g. triphosgene; a cyclic carbonate; and carbon dioxide;

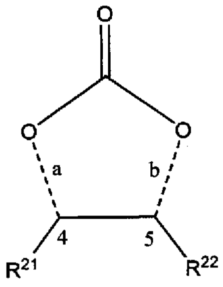 (I)

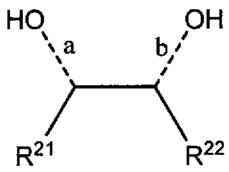 (V) 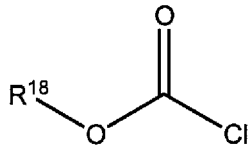 (VI),

wherein

each dotted bond "**a**" and "**b**" independently represents a solid bond, a wedged bond, or a hashed wedged bond; $\mathbf{R^{21}}$ and $\mathbf{R^{22}}$ are each independently selected from $C_{1-6}$alkyl, $C_{3-6}$cycloalkyl, and $C_{6-10}$aryl; or $\mathbf{R^{21}}$ and $\mathbf{R^{22}}$ taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and $\mathbf{R^{18}}$ is $C_{1-6}$alkyl or $C_{3-6}$cycloalkyl.

Preferably, the present invention further provides a process for the synthesis of a compound of formula (I) a stereoisomer or racemate thereof, comprising the step of contacting a compound of formula (V), a stereoisomer, or racemate thereof; with at least one reagent selected from the group consisting of an alkyl- or cycloalkyl-chloroformate of formula (VI); an urea; a phosgene or derivative thereof; a cyclic carbonate; and carbon dioxide;

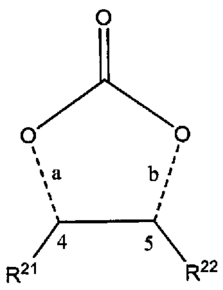 (I)

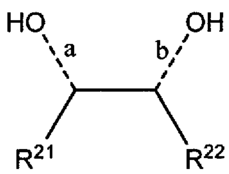 (V) 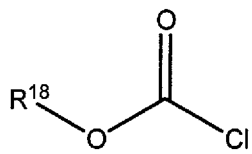 (VI),

wherein

each dotted bond "**a**" and "**b**" independently represents a solid bond, a wedged bond, or a hashed wedged bond;

**$R^{21}$** and **$R^{22}$** taken together with the carbon atoms to which they are attached form a ring selected from $C_{3-9}$cycloalkylene, $C_{5-9}$cycloalkenylene, and $C_{3-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and

**$R^{18}$** is $C_{1-6}$alkyl, or $C_{3-6}$cycloalkyl.

According to a third aspect, the present invention also encompasses a polycarbonate or copolymer thereof comprising a recurring unit of formula (VII1) and/or (VII2),

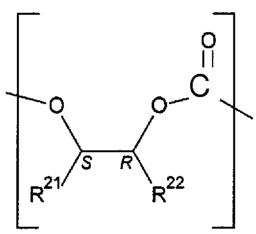
(VII1)
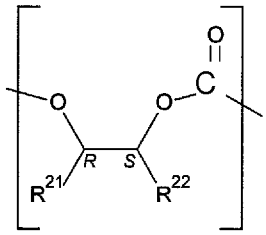
(VII2)

wherein

**$R^{21}$** and **$R^{22}$** are each independently selected from $C_{1-6}$alkyl, $C_{3-6}$cycloalkyl, and $C_{6-10}$aryl; or **$R^{21}$** and **$R^{22}$** taken together with the carbon atoms 4 and 5 to which they are attached form a ring selected from $C_{3-9}$cycloalkylene, $C_{5-9}$cycloalkenylene, and $C_{3-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

Preferably, the present invention also encompasses a polycarbonate or copolymer thereof comprising a recurring unit of formula (VII1) and/or (VII2),

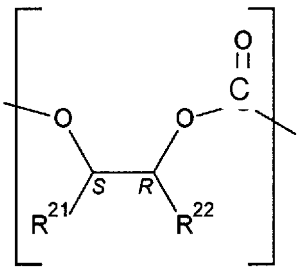
(VII1)
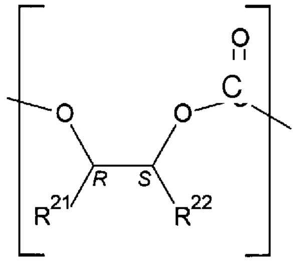
(VII2)

wherein

**$R^{21}$** and **$R^{22}$** taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

According to a fourth aspect, the present invention also encompasses a polycarbonate or copolymer thereof comprising recurring unit of formula (VII3) or (VII4),

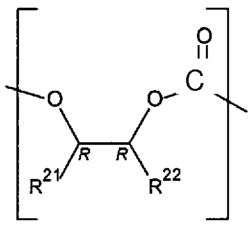
(VII3)
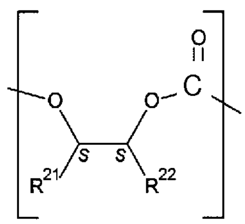
(VII4)

wherein

**R$^{21}$** and **R$^{22}$** are each independently selected from $C_{1-6}$alkyl, $C_{3-6}$cycloalkyl, and $C_{6-10}$aryl; or **R$^{21}$** and **R$^{22}$** taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino;
wherein said polycarbonate or copolymer thereof is characterized by a $^{13}C\{^1H\}$ NMR spectrum comprising only one signal present in the areas ranging from $\delta$ 150 to 155 ppm.

Preferably, the present invention also encompasses a polycarbonate or copolymer thereof comprising recurring units of formula (VII3) or of formula (VII4),

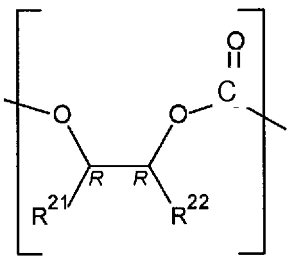
(VII3)
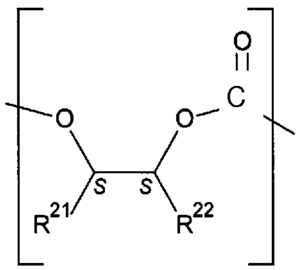
(VII4)

wherein

**R$^{21}$** and **R$^{22}$** taken together with the carbon atoms 4 and 5 to which they are attached form a ring selected from $C_{3-9}$cycloalkylene, $C_{5-9}$cycloalkenylene, and $C_{3-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and
wherein said polycarbonate or copolymer thereof is characterized by a $^{13}C\{^1H\}$ NMR spectrum comprising only one signal present in the carbonyl areas ranging from $\delta$ 150 to 155 ppm; preferably said polycarbonate or copolymer thereof is characterized by a high resolution (100 MHz or higher) $^{13}C\{^1H\}$ NMR spectrum comprising only one sharp resonance present in the carbonyl areas ranging from $\delta$ 150 to 155 ppm.

In an embodiment, said polycarbonate or copolymer thereof according to the fourth aspect of the invention also comprises less than 2% of monomer having at least one chiral center of inverse configuration compared to that of the enantiopure monomer. This percentage can be measured, after hydrolysis of the precipitated polymer, by chiral chromatography of the vicinal diol resulting from the hydrolysis.
In a fifth aspect, the present invention also encompasses an article comprising a polycarbonate or copolymer thereof according to the third or fourth aspect of the invention, or a polycarbonate prepared according to the first aspect of the invention.
The processes of the present invention allow the production of polycarbonates and copolymers thereof with controlled tacticity, and improved properties such as high molecular mass and narrow molecular mass distribution, and original thermal features, in an efficient and economical way. In addition, the processes of the present invention allow the preparation of polycarbonates and copolymers thereof in a one-pot and one-step method.
The independent and dependent claims set out particular and preferred features of the invention. Features from the dependent claims may be combined with features of the independent or other dependent claims as appropriate.
The above and other characteristics, features and advantages of the present invention will become apparent from the following detailed descriptions, taken in conjunction with the accompanying drawings, which illustrate, by way of examples, the principles of the invention. The reference figures quoted below refer to the attached drawings.

**BRIEF DESCRIPTION OF THE DRAWINGS**

Figure 1 represents the formula as well as the $^1H$ NMR spectrum (400 MHz, $CDCl_3$, 25 °C) of a PCHC synthesized from *trans-rac*-CHC in the presence of [(NNO)ZnEt]/BnOH in toluene at 100 °C (Example 1, Table 1, entry 8; $CDCl_3$).

Figure 2 represents the formula as well as the $^{13}C\{^1H\}$ NMR spectrum (100 MHz, $CDCl_3$, 25 °C) of a PCHC synthesized from *trans-rac*-CHC in the presence of [(NNO)ZnEt]/BnOH in toluene at 100 °C (PCHC of Example 1, Table 1, entry 5).

Figure 3 represents a graph plotting the differential scanning calorimetry (DSC) trace of a PCHC sample synthesized from *trans-rac*-CHC (PCHC of Example 1, Table 1, entry 3).

Figure 4 represents the $^1H$ NMR spectrum (400 MHz, $CDCl_3$, 25 °C) of a copolymer PLLA-co-PCHC synthesized from *trans-rac*-CHC and L-LA in the presence of a [(NNO)ZnEt]/BnOH system in toluene at 100 °C (with presence of residual $CH_2Cl_2$ and MeOH following precipitation of the polymer).

Figure 5 represents a graph plotting the DSC trace of a sample of the copolymer PLLA-co-PCHC with a Mn of 15,760 g/mol.

Figure 6 represents the formula as well as the $^{13}C\{^1H\}$ NMR spectrum (100 MHz, $CDCl_3$, 25 °C) of a PCHC synthesized from (*R*,*R*)-CHC in the presence of [(NNO)ZnEt]/BnOH in toluene at 60 °C (PCHC of Example 2, Table 2, entry 1)

Figure 7 represents a graph plotting the differential scanning calorimetry (DSC) trace of a PCHC sample (PCHC of Example 2, Table 2, entry 2).

Figure 8 section a) represents part of a $^{13}C\{^1H\}$ NMR spectrum (100 MHz, $CDCl_3$, 25°C) of a PCHC synthesized by ROP of rac-CHC according to an embodiment of the invention (Table 4, entry 1'); section b) represents part of $^{13}C\{^1H\}$ NMR spectrum (100 MHz, $CDCl_3$, 25°C) of a PCHC synthesized by ROP of *rac*-CHC, according to an embodiment of the invention (Table 4, entries 5 and 6, the latter corresponding to the darker line).

Figure 9 represents the formula as well as the $^1H$ NMR spectrum (500 MHz, $CD_2Cl_2$, 25°C) of a copolymer PLLA-*co*-PCHC according to an embodiment of the invention (Table 5, entry 8).

Figure 10 represents the formula as well as the $^{13}C\{^1H\}$ NMR spectrum (100 MHz, $CD_2Cl_2$, 25˚C) of a copolymer PLLA-*co*-PCHC according to an embodiment of the invention (Table 5, entry 8); wherein x stands for unreacted CHC, and * stands for residual solvent resonances.

Figure 11 represents the formula as well as the $^1H$ NMR spectrum (500 MHz, $CDCl_3$, 25˚C) of a PCHC-*b*-PLLA copolymer according to an embodiment of the invention (Table 6, entry 2).

Figure 12 represents the formula as well as the $^{13}C\{^1H\}$ NMR spectrum (100 MHz, $CDCl_3$, 25˚C) of a PCHC-*b*-PLLA copolymer according to an embodiment of the invention (Table 6, entry 2), wherein * stands for residual solvent resonances.

Figure 13 represents the formula as well as the $^1H$ NMR spectrum (400 MHz, $CDCl_3$, 25˚C) of the crude product from the sequential copolymerization *rac*-CHC:LLA (1:1) according to an embodiment of the invention (Table 6, entry 2').

Figure 14 represents the formula as well as the $^1H$ NMR spectrum (400 MHz, $CDCl_3$, 25˚C) of the PCHC-*b*-PLLA copolymer obtained from precipitation of the crude reaction mixture in Example 8 (produced from the sequential copolymerization *rac*-CHC:LLA (1:1) described in Table 6, entry 2') in cold methanol.

Figure 15 represents the formula as well as the $^1H$ NMR spectrum (400 MHz, $CDCl_3$, 25˚C) of the PLLA-*b*-PCHC copolymer described in Example 8 (obtained by sequential copolymerization of L-LA and *rac*-CHC, where the L-LA was added before the addition of *rac*-CHC), wherein * stands for residual solvent resonances.

Figure 16 represents the formula as well as the $^{13}C\{^1H\}$ NMR spectrum (100 MHz, $CD_2Cl_2$, 25˚C) of the PLLA-*b*-PCHC copolymer described in Example 8 (obtained by sequential copolymerization of L-LA and *rac*-CHC, where the L-LA was added before the addition of *rac*-CHC), wherein * stands for residual solvent resonances.

Figure 17 represents the formula as well as the $^1H$ NMR spectrum (400 MHz, $CD_2Cl_2$, 25˚C) of a PCHC-*b*-PLLA copolymer according to an embodiment of the invention (Table 8, entry 2), wherein * stands for residual solvent resonances. 13 1

Figure 18 represents the formula as well as the C{ H} NMR spectrum (100 MHz, $CD_2Cl_2$, 25°C) of a PLLA-*b*-PCHC copolymer according to an embodiment of the invention (Table 8, entry 2), wherein * stands for residual solvent resonances.

Figure 19 represents the formula as well as the $^1$H NMR spectrum (500 MHz, $CDCl_3$, 25°C) of a PCHC-*co*-PTMC copolymer according to an embodiment of the invention (Table 9, entry 2), wherein * stands for residual solvent resonances.

Figure 20 represents the formula as well as the $^{13}$C{$^1$H} NMR spectrum (100 MHz, $CDCl_3$, 25°C) of a PCHC-*co*-PTMC copolymer according to an embodiment of the invention (Table 9, entry 2), wherein * stands for residual solvent resonances.

Figure 21 represents the formula as well as the $^{13}$C{$^1$H} NMR spectrum (100 MHz, $CDCl_3$, 25°C) of a PCHC-*co*-PTMC copolymer according to an embodiment of the invention (Table 10, entry 2), wherein * stands for residual solvent resonances.

Figure 22 represents the formula as well as the $^1$H NMR spectrum (500 MHz, $CDCl_3$, 25°C) of a PCHC-*co*-PTMC copolymer according to an embodiment of the invention (Table 11, entry 2), wherein * stands for residual solvent resonances.

Figure 23 represents the formula as well as the $^{13}$C{$^1$H} NMR spectrum (100 MHz, $CDCl_3$, 25°C) of a PCHC-*co*-PTMC copolymer according to an embodiment of the invention (Table 11, entry 2), wherein * stands for residual solvent resonances.

## DETAILED DESCRIPTION OF THE INVENTION

Before the present process of the invention is described, it is to be understood that this invention is not limited to particular processes, components, or devices described, as such processes, components, and devices may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

When describing the present invention, the terms used are to be construed in accordance with the following definitions, unless a context dictates otherwise.

Whenever the term "substituted" is used in the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture.

The term "$C_{1-20}$alkyl", as a group or part of a group, refers to a hydrocarbyl radical of Formula $C_nH_{2n+1}$ wherein n is a number ranging from 1 to 20. Generally, the alkyl groups comprise from 1 to 20 carbon atoms, preferably from 1 to 12 carbon atoms, preferably from 1 to 10 carbon atoms, preferably from 1 to 6 carbon atoms, more preferably 1, 2, 3, 4, 5, 6 carbon atoms. Alkyl groups may be linear, or branched and may be substituted as indicated herein. When a subscript is used herein following a carbon atom, the subscript refers to the number of carbon atoms that the named group may contain. Thus, for example, $C_{1-20}$alkyl groups include all linear, or branched alkyl groups having 1 to 20 carbon atoms, and thus includes for example methyl, ethyl, *n*-propyl, *i*-propyl, 2-methyl-ethyl, butyl and its isomers (e.g. *n*-butyl, *i*-butyl and *t*-butyl); pentyl and its isomers, hexyl and its isomers, heptyl and its isomers, octyl and its isomers, nonyl and its isomers, decyl and its isomers, undecyl and its isomers, dodecyl and its isomers, tridecyl and its isomers, tetradecyl and its isomers, pentadecyl and its isomers, hexadecyl and its isomers, heptadecyl and its isomers, octadecyl and its isomers, nonadecyl and its isomers, icosyl and its isomers, and the like. For example, $C_{1-10}$alkyl includes all linear, or branched alkyl groups having 1 to 10 carbon atoms, and thus includes for example methyl, ethyl, *n*-propyl, *i*-propyl, 2-methyl-ethyl, butyl and its isomers (e.g. *n*-butyl, *i*-butyl and *t*-butyl); pentyl and its isomers, hexyl and its isomers, heptyl and its isomers, octyl and its isomers, nonyl and its isomers, decyl and its isomers and the like. For example, $C_{1-6}$alkyl includes all linear, or branched alkyl groups having 1 to 6 carbon atoms, and thus includes for example methyl, ethyl, *n*-propyl, *i*-propyl, 2-methyl-ethyl, butyl and its isomers (e.g. *n*-butyl, *i*-butyl and *t*-butyl); pentyl and its isomers, hexyl and its isomers. When the suffix "ene" is used in conjunction with an alkyl group, i.e. "alkylene", this is intended to mean the alkyl group as defined herein having two single bonds as points of attachment to other groups.

The term "$C_{1-6}$alkoxy" or "$C_{1-6}$alkyloxy", as a group or part of a group, refers to a group having the Formula $-OR^a$ wherein $R^a$ is $C_{1-6}$alkyl. Non-limiting examples of suitable $C_{1-6}$alkoxy include methoxy, ethoxy, propoxy, isopropoxy, butoxy, isobutoxy, sec-butoxy, tert-butoxy, pentyloxy and hexyloxy.

The term "$C_{3-9}$cycloalkyl", as a group or part of a group, refers to a cyclic alkyl group, that is to say, a monovalent, saturated, hydrocarbyl group having 1 or more cyclic structure, and comprising from 3 to 9 carbon atoms, more preferably from 3 to 8 carbon atoms, more preferably from 3 to 6 carbon atoms, still more preferably from 5 to 6 carbon atoms. Cycloalkyl includes all saturated hydrocarbon groups containing 1 or more rings, including monocyclic or bicyclic groups. The further rings of multi-ring cycloalkyls may be either fused, bridged and/or joined through one or more spiro atoms. Examples of $C_{3-6}$cycloalkyl groups include but are not limited to cyclopropyl, cyclobutyl, cyclopentyl, cyclohexyl. When the suffix "ene" is used in conjunction with a cycloalkyl group, i.e. cycloalkylene, this is intended to mean the cycloalkyl group as defined herein having two single bonds as points of attachment to other groups.

The term "$C_{5-9}$cycloalkenyl", as a group or part of a group, refers to a group derived from a cycloalkyl group, as defined above, comprising from 5 to 9 carbon atoms, and comprising one or more double bonds. For example, this could be the group cyclopentenyl, cyclohexenyl, cyclopenta-1,3-dienyl, cycloheptenyl, cyclooctenyl, cycloocta-1,4-dienyl. When the suffix "ene" is used in conjunction with a cycloalkenyl group, i.e. cycloalkenylene, this is intended to mean the cycloalkenyl group as defined herein having two single bonds as points of attachment to other groups.

The term "$C_{6-30}$aryl", as a group or part of a group, refers to a polyunsaturated, aromatic hydrocarbyl group having a single ring (i.e. phenyl) or multiple aromatic rings fused together (e.g. naphthalene), or linked covalently, typically containing 6 to 30 atoms; wherein at least one ring is aromatic. Examples of suitable aryl include $C_{6-12}$aryl, more preferably $C_{6-10}$aryl. Non-limiting examples of $C_{6-12}$aryl comprise phenyl, biphenylyl, biphenylenyl, or 1-or 2-naphthanelyl. When the suffix "ene" is used in conjunction with an aryl group, this is intended to mean the aryl group as defined herein having two single bonds as points of attachment to other groups.

The term "$C_{6-30}$aryl$C_{1-20}$alkyl", as a group or part of a group, means a $C_{1-20}$alkyl as defined herein, wherein at least one hydrogen atom is replaced by at least one $C_{6-30}$aryl as defined herein. Preferred $C_{6-30}$aryl$C_{1-20}$alkyl includes $C_{6-12}$aryl$C_{1-6}$alkyl. Non-limiting examples of $C_{6-12}$aryl$C_{1-6}$alkyl group include benzyl, phenethyl, dibenzylmethyl, methylphenylmethyl, 3-(2-naphthyl)-butyl, and the like.

The term "halo" or "halogen", as a group or part of a group, is generic for fluoro, chloro, bromo or iodo.

The term "halo$C_{1-6}$alkyl", as a group or part of a group, refers to a $C_{1-6}$alkyl group having the meaning as defined above wherein one or more hydrogens are replaced with a halogen as defined above. Non-limiting examples of such halo$C_{1-6}$alkyl radicals include chloromethyl, 1-bromoethyl, fluoromethyl, difluoromethyl, trifluoromethyl, 1,1,1-trifluoroethyl and the like.

The term "halo$C_{1-6}$alkoxy", as a group or part of a group, refers to a group of Formula $-O-R^b$ wherein $R^b$ is halo$C_{1-6}$alkyl as defined herein. Non-limiting examples of suitable halo$C_{1-6}$alkoxy include fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy, 2,2,2-trichloroethoxy, trichloromethoxy, 2-bromoethoxy, pentafluoroethyl, 3,3,3-trichloropropoxy, 4,4,4-trichlorobutoxy.

The term "hydroxyl" or "hydroxy", as a group or part of a group, refers to the group -OH.

The term "$C_{1-6}$alkylthio", as a group or part of a group, refers to a group having the Formula -$SR^a$ wherein $R^a$ is $C_{1-6}$alkyl. Non-limiting examples of $C_{1-6}$alkylthio groups include methylthio (-$SCH_3$), ethylthio (-$SCH_2CH_3$), n-propylthio, isopropylthio, n-butylthio, isobutylthio, sec-butylthio, tert-butylthio, and the like.

The term "amino" refers to the group -$NH_2$.

The term "mono- or di-$C_{1-6}$alkylamino", as a group or part of a group, refers to a group of formula -N($R^c$)($R^d$) wherein $R^c$ and $R^d$ are each independently selected from hydrogen, or $C_{1-6}$alkyl, wherein at least one of $R^c$ or $R^d$ is $C_{1-6}$alkyl. Thus, alkylamino include mono-alkyl amino group (e.g. mono-$C_{1-6}$alkylamino group such as methylamino and ethylamino), and di-alkylamino group (e.g. di-$C_{1-6}$alkylamino group such as dimethylamino and diethylamino). Non-limiting examples of suitable alkylamino groups include *n*-propylamino, isopropylamino, *n*-butylamino, *i*-butylamino, *sec*-butylamino, *t*-butylamino, pentylamino, *n*-hexylamino, di-*n*-propylamino, di-*i*-propylamino, ethylmethylamino, methyl-*n*-propylamino, methyl-*i*-propylamino, *n*-butylmethylamino, *i*-butylmethylamino, *t*-butylmethylamino, ethyl-*n*-propylamino, ethyl-*i*-propylamino, *n*-butylethylamino, i-butylethylamino, *t*-butylethylamino, di-*n*-butylamino, di-*i*-butylamino, methylpentylamino, methylhexylamino, ethylpentylamino, ethylhexylamino, propylpentylamino, propylhexylamino, and the like.

The term "carboxy" or "carboxyl", as a group or part of a group, refers to the group -$CO_2H$.

The term "$C_{1-6}$alkoxycarbonyl", as a group or part of a group, refers to a carboxy group linked to a $C_{1-6}$alkyl radical i.e. to form -C(=O)$OR^a$, wherein $R^a$ is as defined above for $C_{1-6}$alkyl.

The term "$C_{1-6}$alkylcarbonyloxy", as a group or part of a group, refers to a group of Formula -O-C(=O)$R^a$ wherein $R^a$ is as defined above for $C_{1-6}$alkyl.

The term "heterocyclyl" , as a group or part of a group, refers to a saturated, unsaturated or aromatic ring system of 4 to 18 atoms including at least one N, O, S, or P. Heterocyclyl thus include heteroaryl groups. Heterocyclyl as used herein includes by way of example and not limitation the heterocycles described in Paquette, Leo A. "Principles of Modern Heterocyclic Chemistry" (W.A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; Katritzky, Alan R., Rees, C.W. and Scriven, E. "Comprehensive Heterocyclic Chemistry" (Pergamon Press, 1996); and J. Am. Chem. Soc. (1960) 82:5566. In a preferred embodiment, heterocyclyl is selected from the group comprising saturated, unsaturated and aromatic ring systems comprising 4 to 10 atoms including at least one N, O, or S. In a preferred embodiment, heterocyclyl is selected from the group comprising saturated, unsaturated and aromatic ring systems comprising 5 to 9 atoms including at least one N, O, or S. In a preferred embodiment, heterocyclyl is selected from the group comprising saturated, unsaturated and aromatic ring systems comprising 5 to 9 atoms including at least one N, or O. Heterocyclyl thus include heteroaryl groups. In a particular embodiment, heterocyclyl is selected from the group comprising 1,3-dioxanyl, 1,4-dioxanyl; pyridyl, dihydroypyridyl, tetrahydropyridyl (piperidyl), thiazolyl, tetrahydrothiophenyl, sulfur oxidized tetrahydrothiophenyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, bis-tetrahydrofuranyl, tetrahydropyranyl, bis-tetrahydropyranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2-dithiazinyl, thianthrenyl, pyranyl, isobenzofuranyl, chromenyl, xanthenyl, phenoxathinyl, 2H-pyrrolyl, isothiazolyl, isoxazolyl, pyrazinyl, pyridazinyl, indolizinyl, isoindolyl, 3H-indolyl, 1H-indazoly, purinyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, benzothienyl, benzothiazolyl and isatinoyl; preferably heterocyclyl is selected from the group comprising 1,3-dioxanyl, 1,4-dioxanyl; pyridyl, dihydroypyridyl, tetrahydropyridyl (piperidyl), thiazolyl, tetrahydrothiophenyl, furanyl, thienyl, pyrrolyl, pyrazolyl, imidazolyl, tetrazolyl, benzofuranyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, bis-tetrahydrofuranyl, tetrahydropyranyl, bis-tetrahydropyranyl, tetrahydroquinolinyl, tetrahydroisoquinolinyl, decahydroquinolinyl, octahydroisoquinolinyl, azocinyl, triazinyl, thianthrenyl, pyranyl, isobenzofuranyl, chromenyl, xanthenyl, phenoxathinyl, 2H-pyrrolyl, isothiazolyl, isoxazolyl, pyrazinyl, pyridazinyl, indolizinyl, isoindolyl, 3H-indolyl, 1H-indazoly, purinyl, 4H-quinolizinyl, phthalazinyl, naphthyridinyl, quinoxalinyl, quinazolinyl, cinnolinyl, pteridinyl, pyrimidinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, benzothienyl, benzothiazolyl and isatinoyl. The term "heteroaryl", as a group or part of a group, refers to an aromatic ring system of 5 to 18 atoms including at least one N, O, S, or P and thus refers to aromatic heterocycles. Examples of heteroaryl include but are not limited to pyridyl, pyridazinyl, pyrimidinyl, pyrazinyl, s-triazinyl, oxazolyl, imidazolyl, thiazolyl, isoxazolyl, pyrazolyl, isothiazolyl, furyl, thienyl, and pyrrolyl. The term "non-aromatic heterocyclyl", as a group or part of a group, refers to a saturated or unsaturated non-aromatic ring system of 4 to 18 atoms including at least one N, O, S, or P.

Preferred statements (features) and embodiments of the processes and polycarbonates of this invention are set herein below. Each statements and embodiments of the invention so defined may be combined with any other statement and/or embodiments unless clearly indicated to the contrary. In particular, any feature indicated as being preferred or advantageous may be combined with any other feature or features indicated as being preferred or advantageous.

Numbered statements of this invention are:

1. A process for preparing a polycarbonate or copolymer thereof, said process comprising homo- or copolymerizing in the presence of at least one catalyst, one or more compounds of formula (I), stereoisomers, racemics, or mixtures thereof; optionally with one or more cyclic esters, stereoisomers, racemics, or mixtures thereof;

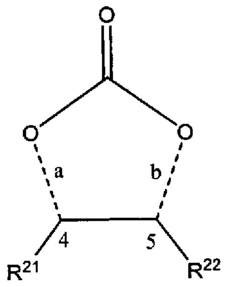
(I)

wherein

each dotted bond "a" and "b" independently represents a solid bond, a wedged bond, or a hashed wedged bond; each **$R^{21}$** and **$R^{22}$** are, independently, selected from $C_{1-6}$alkyl, $C_{3-6}$cycloalkyl, and $C_{6-10}$aryl; or **$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

2. The process according to claim 1, wherein the catalyst is an organometallic catalyst of formula (II) or (III),

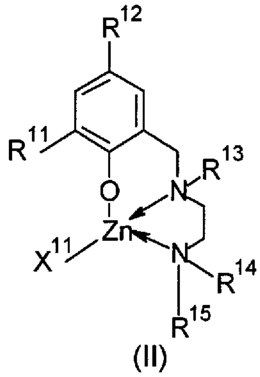
(II)

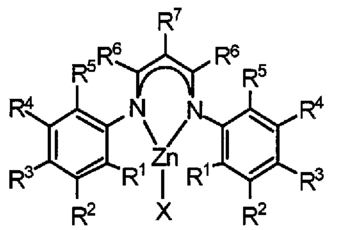
(III)

wherein

**$R^1$, $R^2$, $R^3$, $R^4$, $R^5$, $R^6$**, and **$R^7$** are each independently selected from the group consisting of hydrogen, optionally substituted $C_{1-12}$alkyl, and an inert functional group, and wherein two or more of said groups can be linked together to form one or more rings;
**X** is $-N(SiR^{27}{}_3)_2$, $C_{1-12}$alkyl, $C_{1-12}$alkoxy, $-NR^9R^{10}$ or $-BH_4$;
each **$R^{27}$** is independently selected from hydrogen and $C_{1-6}$alkyl;
each **$R^9$** and **$R^{10}$** is independently selected from hydrogen and $C_{1-6}$alkyl;
**$R^{11}$** and **$R^{12}$** are each independently $C_{1-10}$alkyl;
**$R^{13}$**, **$R^{14}$**, and **$R^{15}$** are each independently $C_{1-10}$alkyl, or
**$R^{13}$** and **$R^{14}$** are covalently bound to each other and are each a methylene and **$R^{15}$** is $C_{1-10}$alkyl;

**$X^{11}$** is selected from $C_{1-10}$alkyl, $-OR^{16}$, and $-N(SiR^{17}{}_3)_2$;
**$R^{16}$** is $C_{1-10}$alkyl; and
each **$R^{17}$** is independently selected from hydrogen and $C_{1-6}$alkyl.

3. The process according to any one of statements 1 to 2, wherein the catalyst is selected from [(NNO)ZnEt], [BDI]Zn($N(SiMe_3)_2$), [BDI]Zn(Et), and {[BDI]Zn($OR^{30}$)}$_2$, wherein **$R^{30}$** is $C_{1-6}$alkyl; preferably the catalyst is [(NNO)ZnEt].

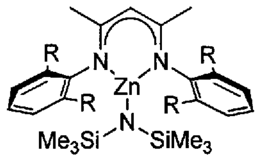

R = $^{i}$Pr

**(BDI)Zn($NTMS_2$)**

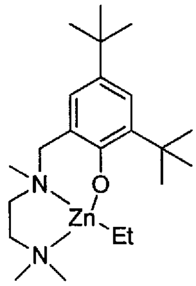

**(NNO)ZnEt**

4. The process according to statement 1, wherein the catalyst is selected from complexes of formulae $M(OSO_2CF_3)_m$, $M(N(OSO_2CF_3)_2)_m$, $M(R^{23}C(O)CR^{23}{}_2C(O)R^{23})_m$, and $(R^{24}CO_2)_mM$, wherein **M** is a metal of Group 2, 3, including the lanthanide series, or Group 12, 13, 15, wherein each **$R^{23}$** is independently an optionally substituted $C_{1-12}$alkyl, wherein each **$R^{24}$** is independently a perfluorinated $C_{1-12}$alkyl or an aryl, and wherein m is the valence of M.
5. The process according to any one of statements 1 to 4, wherein the process is performed in the presence of a compound of formula (IV),

$R^8$-OH (IV)

wherein **$R^8$** is selected from the group consisting of $C_{1-20}$alkyl, $C_{6-30}$aryl, and $C_{6-30}$aryl$C_{1-20}$alkyl optionally substituted by one or more substituents selected from the group consisting of halogen, hydroxyl, and $C_{1-6}$alkyl.
6. The process according to any one of statements 1 to 5, wherein the cyclic ester is selected from the group comprising lactide, glycolide, β-butyrolactone, δ-valerolactone, γ-butyrolactone, γ-valerolactone, 4-methyldihydro-2(3H)-furanone, alpha-methyl-gamma-butyrolactone, and ε-caprolactone, and mixture thereof.
7. The process according to any one of statements 1 to 6, wherein the process is performed in the presence or absence (in bulk) of solvent.
8. The process according to statement 7, wherein the solvent is selected from an aliphatic or aromatic hydrocarbon, an ether, and an halogenated solvent.
9. A process for the synthesis of a compound of formula (I) a stereoisomer or racemate thereof, comprising the step of contacting a compound of formula (V), a stereoisomer, or racemate thereof; with at least one reagent selected from the group consisting of an alkyl- or cycloalkyl-chloroformate of formula (VI); an urea; a phosgene or derivative thereof; a cyclic carbonate; and carbon dioxide;

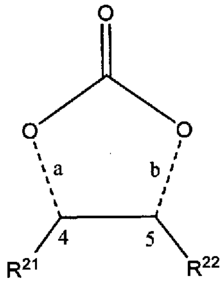

(I)

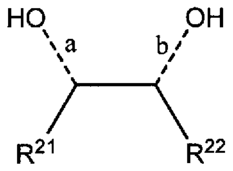

(V)

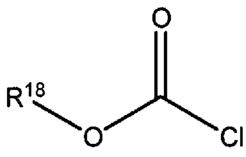

(VI),

wherein
each dotted bond "**a**" and "**b**" independently represents a solid bond, a wedged bond, or a hashed wedged bond;
each **R$^{21}$** and **R$^{22}$** are, independently, selected from $C_{1-6}$alkyl, $C_{3-6}$cycloalkyl, and $C_{6-10}$aryl; or
**R$^{21}$** and **R$^{22}$** taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and
**R$^{18}$** is $C_{1-6}$alkyl, or $C_{3-6}$cycloalkyl.

1. A polycarbonate or copolymer thereof comprising a recurring unit of formula (VII1) and/or (VII2),

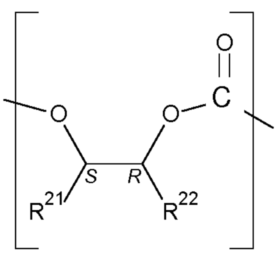

(VII1)

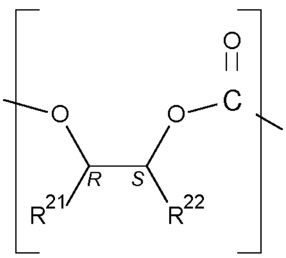

(VII2)

wherein
**R$^{21}$** and **R$^{22}$** taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.
2. The polycarbonate or copolymer thereof according to statement 10, comprising recurring units each independently represented by the following formula (VIIA) or (VIIB) or (VIIC);

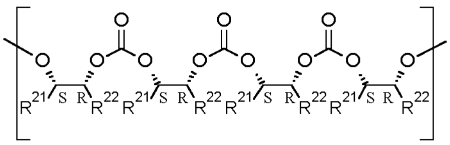

(VIIA)

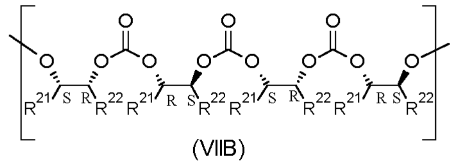

(VIIB)

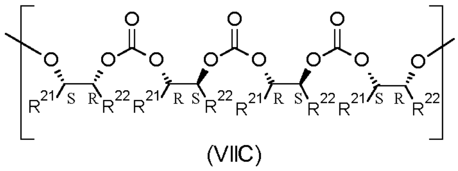

(VIIC)

wherein $R^{21}$ and $R^{22}$ have the same meaning as that defined in statement 10.

12. The polycarbonate or copolymer thereof according to statement 10 or 11, comprising recurring units each independently represented by the following formula (VIIA1) or (VIIB1) or (VIIC1);

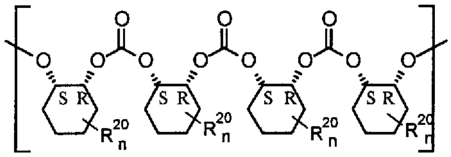

(VIIA1)

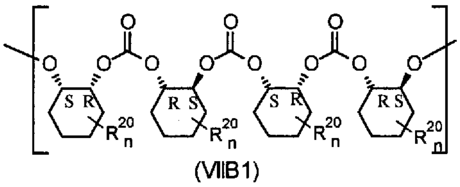

(VIIB1)

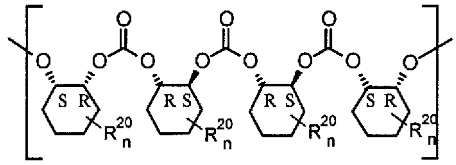

(VIIC1)

each $R^{20}$ is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino;

**n** is an integer selected from 0, 1, 2, 3, or 4.

13. The polycarbonate or copolymer thereof according to statement 10 or 11, comprising recurring units each independently represented by the following formula (VIIA2) or (VIIB2) or (VIIC2);

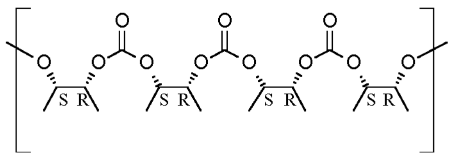

(VIIA2)

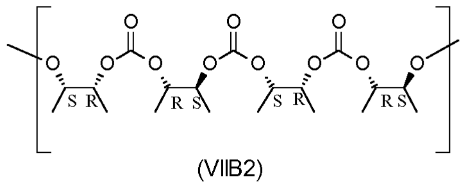

(VIIB2)

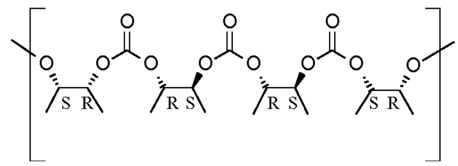

(VIIC2)

3. A polycarbonate or copolymer thereof comprising recurring units of formula (VII3) or of formula (VI14),

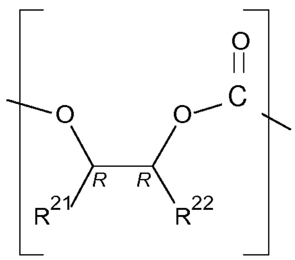

(VII3)

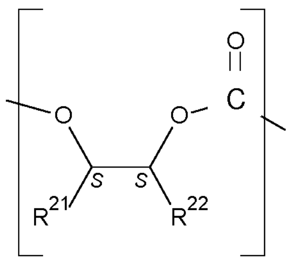

(VII4)

wherein
**$R^{21}$** and **$R^{22}$** taken together with the carbon atoms 4 and 5 to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and
wherein said polycarbonate or copolymer thereof is characterized by a $^{13}C\{^{1}H\}$ NMR spectrum comprising only one signal present in the areas ranging from $\delta$ 150 to 155 ppm.
14. An article comprising a polycarbonate or copolymer thereof according to any one of statements 10 to 14 or a polycarbonate or copolymer thereof prepared according to a process according to any of statements 1 to 8.
15. A process for preparing a polycarbonate or copolymer thereof, said process comprising homo- or copolymerizing in the presence of at least one catalyst, one or more compounds of formula (I), stereoisomers, racemics, or mixtures thereof; optionally with one or more cyclic esters, stereoisomers, racemics, or mixtures thereof;

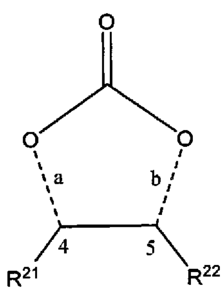

(I)

wherein

each dotted bond "**a**" and "**b**" independently represents a solid bond, a wedged bond, or a hashed wedged bond; and
**$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one

or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

17. A process for preparing a polycarbonate or copolymer thereof, said process comprising copolymerizing in the presence of at least one catalyst, one or more compounds of formula (I), stereoisomers, racemics, or mixtures thereof; with one or more cyclic esters, stereoisomers, racemics, or mixtures thereof;

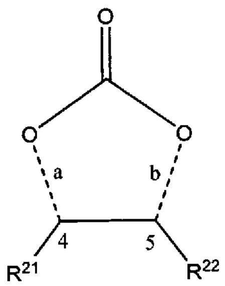
(I)

wherein

each dotted bond "**a**" and "**b**" independently represents a solid bond, a wedged bond, or a hashed wedged bond; and

**$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{3-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; wherein two of said one or more substituents can join to form a ring fused to said $C_{3-9}$cycloalkylene, $C_{5-9}$cycloalkenylene, or $C_{3-12}$arylene; wherein said ring formed by said two substituents is selected from the group comprising $C_{3-9}$cycloalkyl, $C_{5-9}$cycloalkenyl, $C_{6-12}$aryl, and heterocyclyl, wherein each group can be unsubstituted or substituted with one or more $R^{40}$, wherein each **$R^{40}$** is independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

18. The process according to any one of statements 1-3, 5-8, 16-17, wherein the catalyst is an organometallic catalyst of formula (II) or (III),

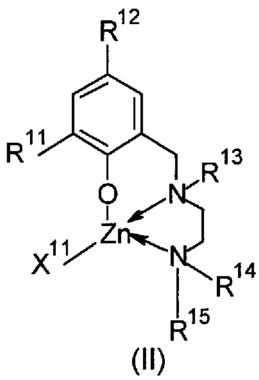
(II)

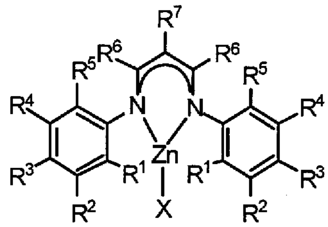
(III)

wherein

**$R^1$**, **$R^2$**, **$R^3$**, **$R^4$**, **$R^5$**, **$R^6$**, and **$R^7$** are each independently selected from the group consisting of hydrogen, optionally substituted $C_{1-12}$alkyl, and an inert functional group, and wherein two or more of said groups can be linked together to form one or more rings;

**X** is $-N(SiR^{27}{}_3)_2$, $C_{1-12}$alkyl, $C_{1-12}$alkoxy, $-NR^9R^{10}$ or $-BH_4$;

each **$R^{27}$** is independently selected from hydrogen and $C_{1-6}$alkyl;

each $\mathbf{R^9}$ and $\mathbf{R^{10}}$ is independently selected from hydrogen and $C_{1-6}$alkyl;
$\mathbf{R^{11}}$ and $\mathbf{R^{12}}$ are each independently $C_{1-10}$alkyl;
$\mathbf{R^{13}}$, $\mathbf{R^{14}}$, and $\mathbf{R^{15}}$ are each independently $C_{1-10}$alkyl, or
$\mathbf{R^{13}}$ and $\mathbf{R^{14}}$ are covalently bound to each other and are each a methylene and $\mathbf{R^{15}}$ is $C_{1-10}$alkyl;
$\mathbf{X^{11}}$ is selected from $C_{1-10}$alkyl, $-OR^{16}$, and $-N(SiR^{17}{}_3)_2$;
$\mathbf{R^{16}}$ is $C_{1-6}$alkyl; and
each $\mathbf{R^{17}}$ is independently selected from hydrogen and $C_{1-6}$alkyl.

19. The process according to any one of statements 1-3, 5-8, 16-18, wherein the catalyst is selected from [(NNO)ZnEt], $[BDI]Zn(N(SiMe_3)_2)$, [BDI]Zn(Et), and $\{[BDI]Zn(OR^{30})\}_2$, wherein $\mathbf{R^{30}}$ is $C_{1-6}$alkyl,

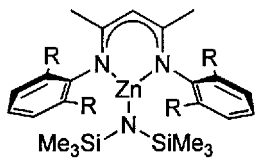

R = $^iPr$

**$(BDI)Zn(NTMS_2)$**

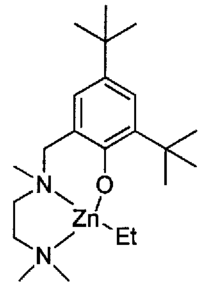

**(NNO)ZnEt** .

20. The process according to any one of statements 1-3, 5-8, 16-19, wherein the catalyst is [(NNO)ZnEt].
21. The process according to any one of statements 1, 4-8, 16-17, wherein the catalyst is selected from complexes of formulae $M(OSO_2CF_3)_m$, $M(N(OSO_2CF_3)_2)_m$, $M(R^{23}C(O)CR^{23}{}_2C(O)R^{23})_m$, and $(R^{24}CO_2)_mM$, wherein **M** is a metal of Group 2, 3, including the lanthanide series, or Group 12, 13, 15, wherein each $\mathbf{R^{23}}$ is independently an optionally substituted $C_{1-12}$alkyl, wherein each $\mathbf{R^{24}}$ is independently a perfluorinated $C_{1-12}$alkyl or an aryl, and wherein **m** is the valence of M.
22. The process according to any one of statements 1, 5-8, 16-17, wherein the catalyst is selected from the group comprising dimeric phosphazene bases, or amines or guanidines, organic acids, sparteine, thiourea-amino derivaties, N-heterocyclic carbenes, 4-(dialkylamino)pyridines, amidines, phosphorus based phospines, phosphazenium derivatives and phosphazenes, metal amides; and compounds of general formula $M^1(Y^1,Y^2, ... Y^p)_q$, in which $\mathbf{M^1}$ is a metal selected from the group comprising the elements of columns 3 to 12 of the periodic table of the elements, and the elements Al, Ga, In, Tl, Ge, Sn, Pb, Sb, Ca, Mg and Bi; whereas $\mathbf{Y^1}$, $\mathbf{Y^2}$, ... $\mathbf{Y^p}$ are each substituents selected from the group comprising alkyl with 1 to 20 carbon atoms, aryl having from 6 to 30 carbon atoms, alkoxy having from 1 to 20 carbon atoms, aryloxy having from 6 to 30 carbon atoms, oxide, carboxylate, and halide groups, and **p** and **q** are integers of from 1 to 6.
23. The process according to any one of statements 1, 5-8, 16-17, 22, wherein the catalyst is selected from the group comprising 1,5,7-triazobicyclo-[4,4,0]dec-5-ene (TBD), 1,8-diazabicyclo[5.4.0]undec-7-ene (DBU), 4-dimethylaminopyridine (DMAP), and *tert*-butylimino-1,3-dimethylperhydro-1,3,2-diazaphosphine (BEMP); $Sn(Oct)_2$, $Al(OiPr)_3$, $Ti(OiPr)_4$, Ti(2-ethylhexanoate)$_4$, Ti(2-ethylhexyloxide)$_4$, $Zr(OiPr)_4$, Bi(neodecanoate)$_3$, (2,4-di-tert-butyl-6-(((2-(dimethylamino)ethyl)(methyl)amino)methyl)phenoxy)(ethoxy)zinc, Zn(lactate)$_2$, $Y[N(SiMe_3)_2]_3$; [Y] + L1; and [Y] + L2, wherein **L1** is a tetradentate ligand of formula L1 and **L2** is a tetradentate ligand of formula L2

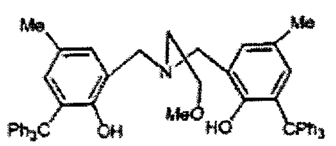

L1

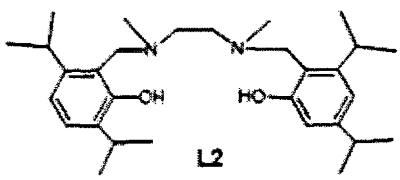

L2 .

24. The process according to any one of statements 1, 5-8, 16-17, wherein the catalyst is selected from the group comprising [(NNO)ZnEt]; $[BDI]Zn(N(SiMe_3)_2)$; 1,5,7-triazobicyclo-[4,4,O]dec-5-ene (TBD); 1,8-diazabicyclo[5.4.0]undec-7-ene (DBU); $Y[N(SiMe_3)_2]_3$; [Y] + L1; and [Y] + L2; wherein **L1** is a tetradentate ligand of formula L1 and **L2** is a tetradentate ligand of formula L2

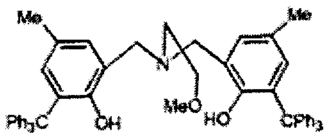
L1

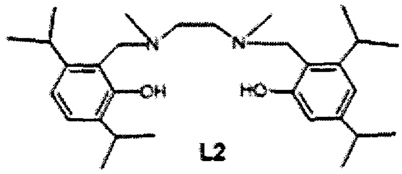
L2
.

25. The process according to any one of statements 1-8, 16-24, wherein the process is performed in the presence of a compound of formula (IV),

$R^8$-OH (IV)

wherein **$R^8$** is selected from the group consisting of $C_{1-20}$alkyl, $C_{6-30}$aryl, and $C_{6-30}$aryl$C_{1-20}$alkyl optionally substituted by one or more substituents selected from the group consisting of halogen, hydroxyl, and $C_{1-6}$alkyl.

26. The process according to any one of statements 1-5, 7-8, 16-25, wherein the cyclic ester is selected from the group comprising lactones, cyclic carbonates, glycolides and lactides, each group can be unsubstituted or substituted with one or more substituents each independently selected from $C_{1-6}$alkyl and $C_{1-6}$alkoxy.

27. The process according to any one of statements 1-5, 7-8, 16-26, wherein the cyclic ester is selected from the group comprising lactide, trimethylene carbonate, glycolide, β-butyrolactone, δ-valerolactone, γ-butyrolactone, γ-valerolactone, 4-methyldihydro-2(3H)-furanone, alpha-methyl-gamma-butyrolactone, and ε-caprolactone, 1,3-dioxolan-2-one, propylene carbonate, 4-methyl-1,3-dioxan-2-one, 1,3-doxepan-2-one, 5-$C_{1-4}$alkoxy-1,3-dioxan-2-one; and mixture thereof, wherein each group can be unsubstituted or substituted with one or more substituents each independently selected from $C_{1-6}$alkyl and $C_{1-6}$alkoxy. For example said cyclic ester can be selected from the group comprising lactide, trimethylene carbonate, glycolide, β-butyrolactone, δ-valerolactone, γ-butyrolactone, γ-valerolactone, 4-methyldihydro-2(3H)-furanone, alpha-methyl-gamma-butyrolactone, ε-caprolactone, and mixture thereof. For example said cyclic ester can be selected from the group comprising lactide, glycolide, β-butyrolactone, δ-valerolactone, γ-butyrolactone, γ-valerolactone, 4-methyldihydro-2(3H)-furanone, alpha-methyl-gamma-butyrolactone, ε-caprolactone, and mixture thereof. For example said cyclic ester is a lactide, such as L-lactide (*S*,*S*-lactide), D-lactide (*R*,*R*-lactide), or meso-(*S*,*R*)-lactide. For example, said cyclic ester is a cyclic carbonate, such as trimethylene carbonate.

28. The process according to any one of statements 1-7, 16-27, wherein the process is performed in the presence or absence (in bulk) of solvent.

29. The process according to any one of statements 1-7, 16-28, wherein the process is performed in the presence of solvent.

30. The process according to any one of statements 1-7, 16-28, wherein the process is performed in the presence of solvent selected from an aliphatic or aromatic hydrocarbon, an ether, an halogenated solvent and a $C_{1-6}$alkyl carbonate.

31. The process according to any one of statements 1-8, 16-30, wherein **$R^{21}$** and **$R^{22}$** taken together with the carbon atoms 4 and 5 of the cyclic carbonate form a ring selected from $C_{5-7}$cycloalkylene, and $C_{15-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino, wherein two of said one or more substituents can join to form a ring fused to said $C_{5-7}$cycloalkylene, or $C_{6-10}$arylene ring.

32. The process according to any one of statements 1-8, 16-31, wherein **$R^{21}$** and **$R^{22}$** taken together with the carbon atoms 4 and 5 of the cyclic carbonate form a ring selected from $C_{6-7}$cycloalkylene, and $C_{6-10}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-3}$alkylcarbonyloxy, amino; or mono- or di-$C_{1-6}$alkylamino; wherein two of said one or more substituents can join to form a $C_{3-6}$cycloalkyl fused to said $C_{6-7}$cycloalkylene, or $C_{6-10}$arylene ring.

33. The process according to any one of statements 1-8, 16-33, wherein **$R^{21}$** and **$R^{22}$** taken together with the carbon atoms 4 and 5 of the cyclic carbonate form a cyclohexylene; optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino, for example one or more substituents each independently selected from halo, hydroxyl, $C_{1-6}$alkyl, $C_{1-6}$alkoxy, or $C_{1-6}$alkylthio; wherein two of said substituents can join to form a $C_{3-6}$cycloalkyl fused to said cyclohexylene ring.

34. The process according to any one of statements 1-8, 16-31, wherein **$R^{21}$** and **$R^{22}$** taken together with the carbon atoms 4 and 5 of the cyclic carbonate form a ring selected from $C_{3-9}$cycloalkylene, and $C_{6-12}$arylene, said ring being optionally substituted with one or more substituents each independently selected from halo, hydroxyl, $C_{1-6}$alkyl,

halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

35. The process according to any one of statements 1-8, 16-32, 34, wherein **$R^{21}$** and **$R^{22}$** taken together with the carbon atoms 4 and 5 of the cyclic carbonate form a ring selected from $C_{3-6}$cycloalkylene, and $C_{6-10}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

36. The process according to any one of statements 1-8, 16-35, wherein **$R^{21}$** and **$R^{22}$** taken together with the carbon atoms 4 and 5 of the cyclic carbonate form $C_{3-6}$cycloalkylene, preferably a $C_{4-6}$cycloalkylene, preferably a $C_{5-6}$cycloalkylene, preferably a cyclohexylene; optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; for example one or more substituents each independently selected from halo, hydroxyl, $C_{1-6}$alkyl, $C_{1-6}$alkoxy, or $C_{1-6}$alkylthio.

37. The process according to any one of statements 1-8, 16-30, wherein the compound of formula (I) has structural formula (IA)

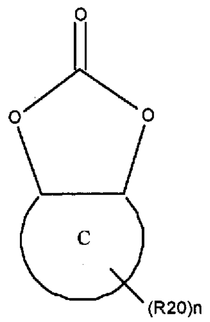

(IA)

wherein

the ring **C** is selected from $C_{3-9}$cycloalkylene, $C_{5-9}$cycloalkenylene, or $C_{6-12}$arylene; preferably $C_{4-6}$cycloalkylene, or $C_{6-10}$arylene; more preferably $C_{5-6}$cycloalkylene;

each **$R^{20}$** is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-10}$alkyl; $C_{1-10}$alkoxy, or $C_{1-6}$alkylthio; preferably $C_{1-6}$alkyl; more preferably $C_{1-4}$alkyl; optionally wherein two $R^{20}$ can join to form a ring fused to ring C, wherein said ring formed by two $R^{20}$ can be selected from the group comprising $C_{3-9}$cycloalkyl, $C_{5-9}$cycloalkenyl, $C_{3-12}$aryl, and heterocyclyl, preferably $C_{5-6}$cycloalkyl, or $C_{6-10}$aryl, and wherein said ring formed by two $R^{20}$ can be unsubstituted or substituted with one or more $R^{40}$, wherein each **$R^{40}$** is independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino;

**n** is an integer selected from 0, 1, 2, 3, or 4; preferably 0, 1, 2, or 3, preferably 0, 1, or 2, more preferably 0 or 1, yet more preferably 0.

38. The process according to any one of statements 1-8, 16-30, 37, wherein the compound of formula (I) has structural formula (IA1), (IA2), (IA3), or (IA4), wherein

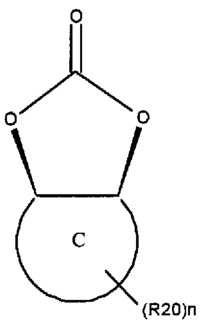
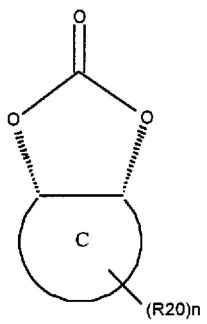
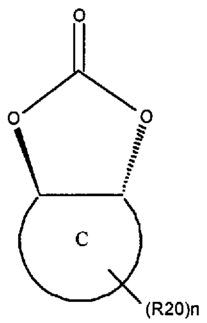
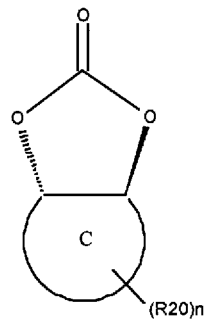

(IA1) (IA2) (IA3) (IA4)

the ring **C** is selected from $C_{3-9}$cycloalkylene, $C_{5-9}$cycloalkenylene, or $C_{6-12}$arylene; preferably $C_{4-6}$cycloalkylene, or $C_{6-10}$arylene; more preferably $C_{5-6}$cycloalkylene; preferably hexylene, each $\mathbf{R^{20}}$ is independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-10}$alkyl; $C_{1-10}$alkoxy, or $C_{1-6}$alkylthio; preferably $C_{1-6}$alkyl; more preferably $C_{1-4}$alkyl; optionally wherein two $R^{20}$ can join to form a ring fused to ring C, wherein said ring formed by two $R^{20}$ can be selected from the group comprising $C_{3-9}$cycloalkyl, $C_{5-9}$cycloalkenyl, $C_{6-12}$aryl, and heterocyclyl, preferably $C_{5-6}$cycloalkyl, or $C_{6-10}$aryl, and wherein said ring formed by two $R^{20}$ can be unsubstituted or substituted with one or more $R^{40}$, wherein each $\mathbf{R^{40}}$ is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-6}$alkyl; $C_{1-10}$alkoxy, or $C_{1-6}$alkylthio; preferably $C_{1-6}$alkyl; more preferably $C_{1-4}$alkyl;

**n** is an integer selected from 0, 1, 2, 3, or 4; preferably 0, 1, 2, or 3, preferably 0, 1, or 2, more preferably 0 or 1, yet more preferably 0.

39. The process according to any one of statements 1-8, 16-30, 37-38, wherein the compound of formula (I) is selected from the compounds of formula (IAa), (IA1a), (IA2a) (IA3a), (IA4a), (IC);

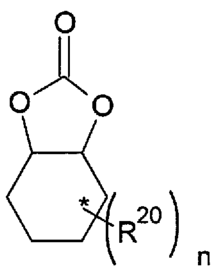
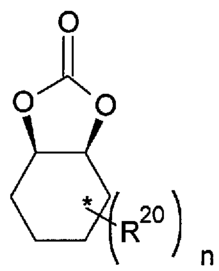
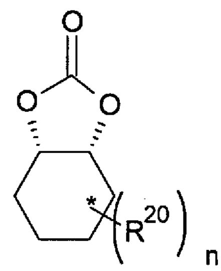
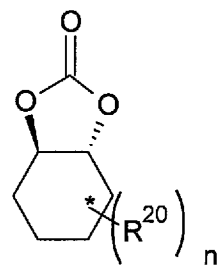

(IAa) (IA1a) (IA2a) (IA3a)

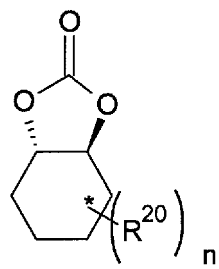
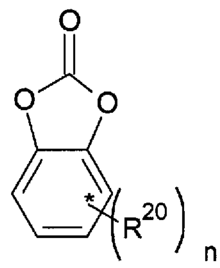

(IA4a) (IC)

wherein $\mathbf{R^{20}}$ is selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio; more preferably halo, hydroxyl, $C_{1-6}$alkyl, $C_{1-6}$alkoxy, $C_{1-6}$alkylthio; optionally wherein two $R^{20}$ can join to form a ring

fused to ring C, wherein said ring formed by two $R^{20}$ can be selected from the group comprising $C_{3-9}$cycloalkyl, $C_{5-9}$cycloalkenyl, $C_{6-12}$aryl, and heterocyclyl, preferably $C_{5-6}$cycloalkyl, or $C_{6-10}$aryl, and wherein said ring formed by two $R^{20}$ can be unsubstituted or substituted with one or more $R^{40}$, wherein each $\mathbf{R^{40}}$ is independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-10}$alkyl; $C_{1-10}$alkoxy, or $C_{1-6}$alkylthio; preferably $C_{1-6}$alkyl; more preferably $C_{1-4}$alkyl; and
**n** is an integer selected from 0, 1, 2, 3, or 4; preferably 0, 1, 2, or 3, preferably 0, 1, or 2, more preferably 0 or 1, yet more preferably 0.

40. A process for the synthesis of a compound of formula (I) a stereoisomer or racemate thereof, comprising the step of contacting a compound of formula (V), a stereoisomer, or racemate thereof; with at least one reagent selected from the group consisting of an alkyl- or cycloalkyl-chloroformate of formula (VI); an urea; a phosgene or derivative thereof; a cyclic carbonate; and carbon dioxide;

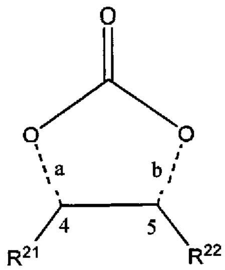
(I)

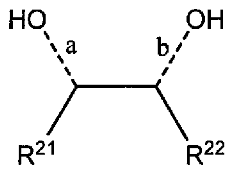
(V)
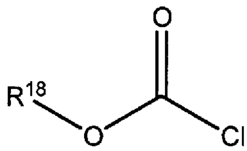
(VI),

wherein

each dotted bond "**a**" and "**b**" independently represents a solid bond, a wedged bond, or a hashed wedged bond;
$\mathbf{R^{21}}$ and $\mathbf{R^{22}}$ taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and
$\mathbf{R^{18}}$ is $C_{1-6}$alkyl, or $C_{3-6}$cycloalkyl.

41. A polycarbonate or copolymer thereof comprising a recurring unit of formula (VII1) and/or (VII2),

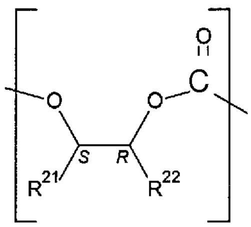
(VII1)
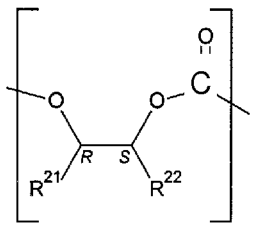
(VII2)

wherein
$\mathbf{R^{21}}$ and $\mathbf{R^{22}}$ taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.
42. The polycarbonate or copolymer thereof according to statement 41, comprising recurring units each independently represented by the following formula (VIIA) or (VIIB) or (VIIC);

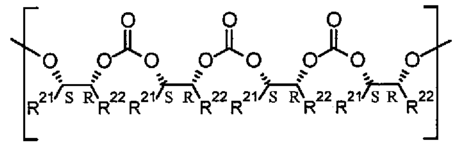

(VIIA)

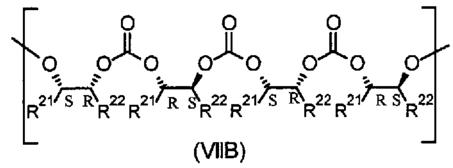

(VIIB)

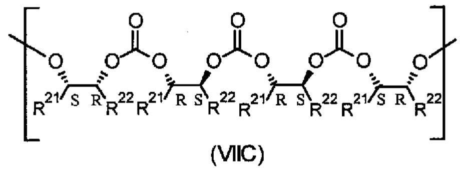

(VIIC)

wherein $R^{21}$ and $R^{22}$ have the same meaning as that defined in statement 41.

43. The polycarbonate or copolymer thereof according to any one of statements 41 or 42, comprising recurring units each independently represented by the following formula (VIIA1) or (VIIB1) or (VIIC1);

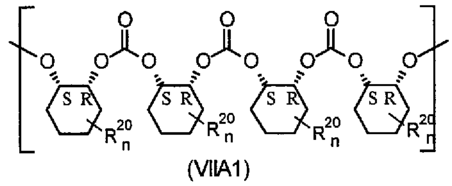

(VIIA1)

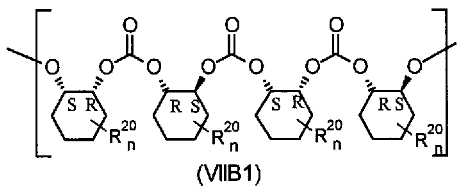

(VIIB1)

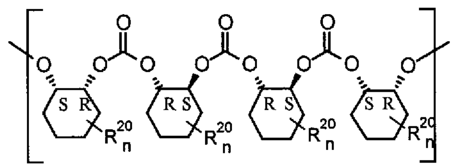

(VIIC1)

each $R^{20}$ is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino;

**n** is an integer selected from 0, 1, 2, 3, or 4.

44. A polycarbonate or copolymer thereof comprising recurring units of formula (VII3) or of formula (VII4),

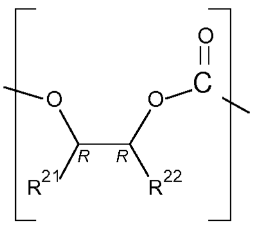
(VII3)
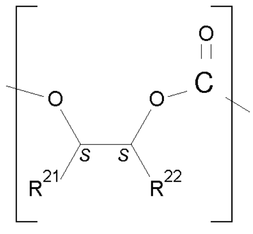
(VII4)

wherein

> $R^{21}$ and $R^{22}$ taken together with the carbon atoms 4 and 5 to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and
> wherein said polycarbonate or copolymer thereof is characterized by a $^{13}C\{^1H\}$ NMR spectrum comprising only one signal present in the areas ranging from δ 150 to 155 ppm, preferably said polycarbonate or copolymer thereof is characterized by a high resolution (100 MHz or higher) $^{13}C\{^1H\}$ NMR spectrum comprising only one sharp resonance present in the carbonyl areas ranging from δ 150 to 155 ppm.

> 6. An article comprising a polycarbonate or copolymer thereof according to any one of statements 11-14, 41-44 or a polycarbonate or copolymer thereof prepared according to a process according to any of one of statements 1-9, 16-39.

The present invention provides a process for preparing a polycarbonate, said process comprising homo- or copolymerizing in the presence of at least one catalyst, one or more compounds of formula (I), stereoisomers, racemics, or mixtures thereof; optionally with one or more cyclic esters, stereoisomers, racemics, or mixtures thereof;
wherein

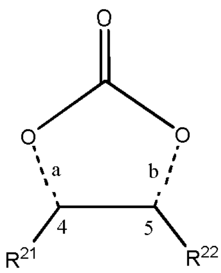
(I)

each dotted bond "**a**" and "**b**" independently represents a solid bond (-), a wedged bond (▬), or a hashed wedged bond (⋯⋯);
**$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms 4 and 5 of the cyclic carbonate form a ring selected from $C_{3-9}$cycloalkylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms 4 and 5 of the cyclic carbonate form a ring selected from $C_{3-6}$cycloalkylene, and $C_{6-10}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms 4 and 5 of the cyclic carbonate form $C_{3-6}$cycloalkylene, preferably a $C_{4-6}$cycloalkylene, preferably a $C_{5-6}$cycloalkylene, preferably a cyclohexylene; optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-

$C_{1-6}$alkylamino; for example one or more substituents each independently selected from halo, hydroxyl, $C_{1-6}$alkyl, $C_{1-6}$alkoxy, or $C_{1-6}$alkylthio.

In a preferred embodiment, **$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

Preferably, the present invention provides a process for preparing a polycarbonate or copolymer thereof, said process comprising copolymerizing in the presence of at least one catalyst, one or more compounds of formula (I), stereoisomers, racemics, or mixtures thereof; with one or more cyclic esters, stereoisomers, racemics, or mixtures thereof;

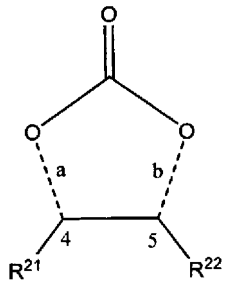

(I)

each dotted bond "**a**" and "**b**" independently represents a solid bond (-), a wedged bond (▬), or a hashed wedged bond (⋯⋯);

**$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino, wherein two of said one or more substituents can join to form a ring fused to said $C_{3-9}$cycloalkylene, $C_{5-9}$cycloalkenylene, or $C_{3-12}$arylene; wherein said ring formed by said two substituents is selected from the group comprising $C_{3-9}$cycloalkyl, $C_{5-9}$cycloalkenyl, $C_{6-12}$aryl, and heterocyclyl, wherein each group can be unsubstituted or substituted with one or more $R^{40}$, wherein each **$R^{40}$** is independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-3}$alkylcarbonyloxy, amino; or mono- or di-$C_{1-6}$alkylamino; preferably each **$R^{40}$** is independently selected from halo, hydroxyl, $C_{1-10}$alkyl; $C_{1-10}$alkoxy, or $C_{1-6}$alkylthio; preferably **$R^{40}$** is $C_{1-6}$alkyl; more preferably $C_{1-4}$alkyl. Preferably, **$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino. Preferably **$R^{21}$** and **$R^{22}$** taken together with the carbon atoms 4 and 5 of the cyclic carbonate form a ring selected from $C_{5-7}$cycloalkylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino, wherein two of said one or more substituents can join to form a ring fused to said $C_{5-7}$cycloalkylene, or $C_{6-10}$arylene ring; preferably **$R^{21}$** and **$R^{22}$** taken together with the carbon atoms 4 and 5 of the cyclic carbonate form a ring selected from $C_{6-7}$cycloalkylene, and $C_{6-10}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; wherein two of said one or more substituents can join to form a $C_{3-6}$cycloalkyl fused to said $C_{6-7}$cycloalkylene, or $C_{6-10}$arylene ring; preferably **$R^{21}$** and **$R^{22}$** taken together with the carbon atoms 4 and 5 of the cyclic carbonate form a cyclohexylene; optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino, for example one or more substituents each independently selected from halo, hydroxyl, $C_{1-6}$alkyl, $C_{1-6}$alkoxy, or $C_{1-6}$alkylthio; wherein two of said substituents can join to form a $C_{3-6}$cycloalkyl fused to said cyclohexylene ring.

In one embodiment, **$R^{21}$** and **$R^{22}$** taken together with the carbon atoms 4 and 5 of the cyclic carbonate form

cyclohexylene, phenylene, or cyclopropylene, each optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms 4 and 5 of the cyclic carbonate form a cyclohexylene, or a phenylene, preferably a cyclohexylene.

In an embodiment, the compound of formula (I) can have preferably structural formula (IA);

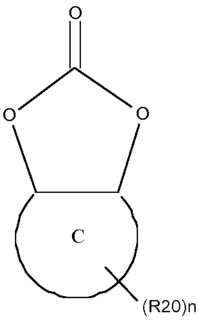

)

wherein

the ring **C** is selected from $C_{3-9}$cycloalkylene, $C_{5-9}$cycloalkenylene, or $C_{6-12}$arylene; preferably $C_{4-6}$cycloalkylene, or $C_{6-10}$arylene; more preferably $C_{5-6}$cycloalkylene;

each **$R^{20}$** is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-10}$alkyl; $C_{1-10}$alkoxy, or $C_{1-6}$alkylthio; preferably $C_{1-6}$alkyl; more preferably $C_{1-4}$alkyl; optionally wherein two **$R^{20}$** can join to form a ring fused to ring C, wherein said ring formed by two **$R^{20}$** can be selected from the group comprising $C_{3-9}$cycloalkyl, $C_{5-9}$cycloalkenyl, $C_{6-12}$aryl, and heterocyclyl, preferably $C_{5-6}$cycloalkyl, or $C_{6-10}$aryl, and wherein said ring formed by two **$R^{20}$** can be unsubstituted or substituted with one or more $R^{40}$, wherein each **$R^{40}$** is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-10}$alkyl; $C_{1-10}$alkoxy, or $C_{1-6}$alkylthio; preferably $C_{1-6}$alkyl; more preferably $C_{1-4}$alkyl;

and

**n** is an integer selected from 0, 1, 2, 3, or 4; preferably 0, 1, 2, or 3, preferably 0, 1, or 2, more preferably 0 or 1, yet more preferably 0.

In some embodiments, compound of formula (I) can have one of the structural formula (IA), (IA1), (IA2), (IA3), (IA4), preferably (IA), (IA1), (IA2), (IA3), (IA4),

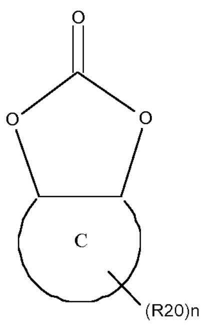

(IA)

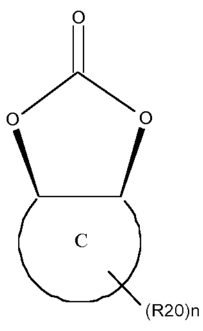

(IA1)

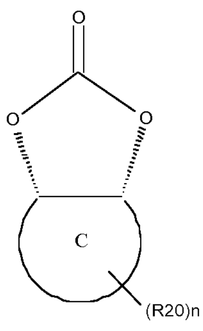

(IA2)

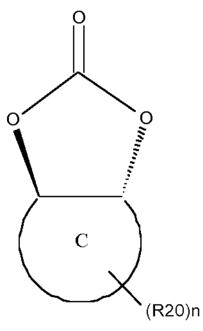

(IA3)

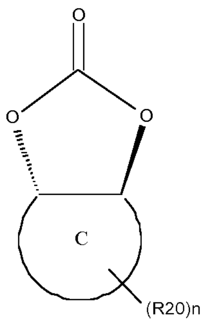

(IA4)

wherein

the ring **C** is selected from $C_{3-9}$cycloalkylene, $C_{5-9}$cycloalkenylene, or $C_{6-12}$arylene; preferably $C_{4-6}$cycloalkylene, or $C_{6-10}$arylene; more preferably $C_{5-6}$cycloalkylene;

each $\mathbf{R^{20}}$ is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-10}$alkyl; $C_{1-6}$alkylthio, or $C_{1-10}$alkoxy, preferably $C_{1-6}$alkyl, more preferably $C_{1-4}$alkyl; and

**n** is an integer selected from 0, 1, 2, 3, or 4; preferably 0, 1, 2, or 3, preferably 0, 1, or 2, more preferably 0 or 1, yet more preferably 0.

Optionally two $R^{20}$ can join to form a ring fused to ring C, wherein said ring formed by two $R^{20}$ can be selected from the group comprising $C_{3-9}$cycloalkyl, $C_{5-9}$cycloalkenyl, $C_{6-12}$aryl, and heterocyclyl, preferably $C_{5-6}$cycloalkyl, or $C_{6-10}$aryl, and wherein said ring formed by two $R^{20}$ can be unsubstituted or substituted with one or more $R^{40}$, wherein each $\mathbf{R^{40}}$ is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-10}$alkyl; $C_{1-10}$alkoxy, or $C_{1-6}$alkylthio; preferably $C_{1-6}$alkyl; more preferably $C_{1-4}$alkyl.

In some embodiments, compound of formula (I) can have one of the structural formula (IA), (IA1), (IA2), (IA3), (IA4), wherein the ring **C,** $\mathbf{R^{20}}$ and **n** are as defined herein above.

In some preferred embodiments, compound of formula (I) can have one of the structural formula (IA), (IA1), (IA2), (IA3), (IA4), wherein the ring **C** is selected from preferably $C_{4-6}$cycloalkylene, or $C_{6-10}$arylene; preferably $C_{5-6}$cycloalkylene; and each $\mathbf{R^{20}}$ is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-10}$alkyl; $C_{1-6}$alkylthio, or $C_{1-10}$alkoxy, preferably $C_{1-6}$alkyl, more preferably $C_{1-4}$alkyl; and **n** is an integer selected from 0, 1, 2, 3, or 4; preferably 0, 1, 2, or 3, preferably 0, 1, or 2, more preferably 0 or 1, yet more preferably 0.

In some embodiments, compound of formula (I) can be selected from the following compounds of formula (IAa), (IA1a), (IA2a) when n is not 0, (IA3a), (IA4a),

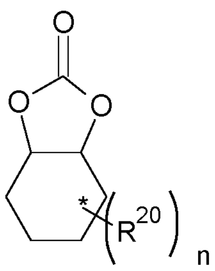

(IAa)

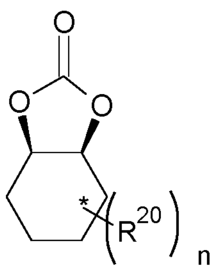

(IA1a)

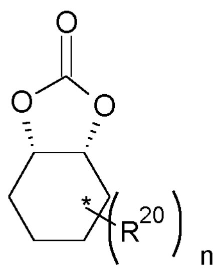

(IA2a)

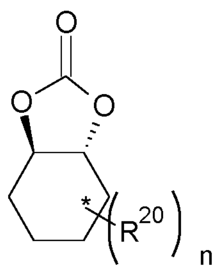

(IA3a)

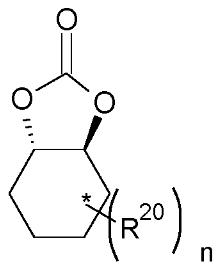

(IA4a)

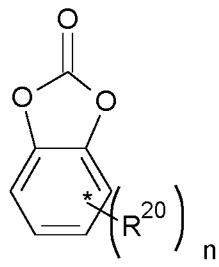

(IC)

wherein **$R^{20}$** is selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio; more preferably halo, hydroxyl, $C_{1-6}$alkyl, $C_{1-6}$alkoxy, $C_{1-6}$alkylthio; and

**n** is an integer selected from 0, 1, 2, 3, or 4; preferably 0, 1, 2, or 3, preferably 0, 1, or 2, more preferably 0 or 1, yet more preferably 0.

Optionally two $R^{20}$ can join to form a ring fused to ring C, wherein said ring formed by two $R^{20}$ can be selected from the group comprising $C_{3-9}$cycloalkyl, $C_{5-9}$cycloalkenyl, $C_{6-12}$aryl, and heterocyclyl, preferably $C_{5-6}$cycloalkyl, or $C_{6-10}$aryl, and wherein said ring formed by two $R^{20}$ can be unsubstituted or substituted with one or more $R^{40}$, wherein each **$R^{40}$** is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-10}$alkyl; $C_{1-10}$alkoxy, or $C_{1-6}$alkylthio; preferably $C_{1-6}$alkyl; more preferably $C_{1-4}$alkyl.

In some embodiments, compound of formula (I) can be selected from the following compounds of formula (IAa), (IA1a), (IA2a), (IA3a), (IA4a), (IC), wherein **$R^{20}$** and **n** are as defined herein above.

In some embodiments, the polymerization of the present invention can occur with the same compound of formula (I), or with two or more compounds of formula (I) differing in structural formula, in which case a copolymerization takes place. In some embodiments, the polymerization of the present invention can occur with two or more of the same compounds of formula (I) differing only in stereochemistry.

In some embodiments, the copolymerization of the present invention can occur with the same compound of formula (I) and one or more cyclic esters. In some embodiments, the copolymerization of the present invention can occur with two or more compounds of formula (I) differing in structural formula, or differing in stereochemistry, and one or more cyclic ester.

In some other embodiments, the copolymerization of the present invention can occur with the same compound of formula (I) and two or more differing cyclic esters.

As used herein, the terms "cyclic ester" refers to cyclic monoesters, cyclic diesters, cyclic triesters, and the like. Preferred are the cyclic monoesters also known as lactones and cyclic carbonates, and the cyclic diesters also known as glycolide and lactides. In an embodiment, the cyclic ester is selected from the group comprising lactones, cyclic carbonates, glycolides and lactides, each group can be unsubstituted or substituted with one or more substituents each independently selected from $C_{1-6}$alkyl, and $C_{1-6}$alkoxy. In an embodiment, the cyclic ester is selected from the group comprising lactones, glycolides and lactides.

Non-limiting examples of suitable cyclic esters can be selected from the group comprising lactide, trimethylene carbonate, glycolide, β-butyrolactone, δ-valerolactone, γ-butyrolactone, γ-valerolactone, 4-methyldihydro-2(3H)-furanone, alpha-methyl-gamma-butyrolactone, ε-caprolactone, 1,3-dioxolan-2-one, propylene carbonate, 4-methyl-1,3-dioxan-2-one, 1,3-doxepan-2-one, 5-$C_{1-4}$alkoxy-1,3-dioxan-2-one; and mixture thereof; each group can be unsubstituted or substituted with one or more substituents each independently selected from $C_{1-6}$alkyl and $C_{1-6}$alkoxy. For example said cyclic esters can be selected from the group comprising lactide, trimethylene carbonate, glycolide, β-butyrotactone, δ-valerolactone, γ-butyrolactone, γ-valerolactone, 4-methyldihydro-2(3H)-furanone, alpha-methyl-gamma-butyrolactone, ε-caprolactone, 4-methyl-1,3-dioxan-2-one, 1,3-doxepan-2-one, and mixture thereof. For example said cyclic esters can be selected from the group comprising lactide, trimethylene carbonate, glycolide, β-butyrolactone, δ-valerolactone, γ-butyrolactone, γ-valerolactone, 4-methyldihydro-2(3H)-furanone, alpha-methyl-gamma-butyrolactone, ε-caprolactone, and mixture thereof. Preferably said cyclic esters can be selected from the group comprising lactide, glycolide, β-butyrolactone, δ-valerolactone, γ-butyrolactone, γ-valerolactone, 4-methyldihydro-2(3H)-furanone, alpha-methyl-gamma-butyrolactone, ε-caprolactone, and mixture thereof. In some embodiment, said cyclic ester is a lactide, such as L-lactide (*S,S*-lactide), D-lactide (*R,R*-lactide), or *meso*-(*S,R*)-lactide. In some embodiment, said cyclic ester is a cyclic carbonate, such as trimethylene carbonate.

The catalyst employed for this process may have general formula $M^1(Y^1,Y^2, ...Y^p)_q$, in which **$M^1$** is a metal selected from the group comprising the elements of columns 3 to 12 of the periodic table of the elements, as well as the elements Al, Ga, In, Tl, Ge, Sn, Pb, Sb, Ca, Mg and Bi; whereas **$Y^1$, $Y^2$, ... $Y^p$** are each substituents selected from the group comprising alkyl with 1 to 20 carbon atoms, aryl having from 6 to 30 carbon atoms, alkoxy having from 1 to 20 carbon atoms, aryloxy having from 6 to 30 carbon atoms, and other oxide, carboxylate, and halide groups as well as elements of group 15 and/or 16 of the periodic table; **p** and **q** are integers of from 1 to 6. As examples of suitable catalysts, we may notably mention the catalysts of Al, Sn, Ti, Zr, Zn, and Bi; preferably an alkoxide or a carboxylate and more preferably $Sn(Oct)_2$, $Al(OiPr)_3$, $Ti(OiPr)_4$, Ti(2-ethylhexanoate)$_4$, Ti(2-ethylhexyloxide)$_4$, $Zr(OiPr)_4$, Bi(neodecanoate)$_3$, (2,4-di-tert-butyl-6-(((2-(dimethylamino)ethyl)(methyl)amino)methyl)phenoxy)(ethoxy)zinc, or Zn(lactate)$_2$.

In one embodiment, the catalyst is an organometallic catalyst of formula (II) or (III),

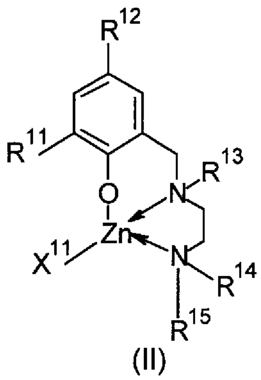
(II)

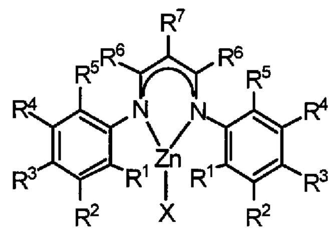
(III)

wherein

**$R^1$, $R^2$, $R^3$, $R^4$, $R^5$, $R^6$,** and **$R^7$** are each independently selected from the group consisting of hydrogen, optionally substituted $C_{1-12}$alkyl, and an inert functional group (e.g., -CN), and wherein two or more of said groups can be linked together to form one or more rings; preferably **$R^1$, $R^2$, $R^3$, $R^4$, $R^5$, $R^6$,** and **$R^7$** are each independently selected from the group consisting of hydrogen, optionally substituted $C_{1-6}$alkyl, and an inert functional group (e.g., -CN), and wherein two or more of said groups can be linked together to form one or more rings; preferably **$R^1$, $R^2$, $R^3$, $R^4$, $R^5$, $R^6$,** and **$R^7$** are each independently selected from the group consisting of hydrogen, or optionally substituted $C_{1-6}$alkyl; preferably **$R^1$, $R^2$, $R^3$, $R^4$, $R^5$, $R^6$,** and **$R^7$** are each independently selected from the group consisting of hydrogen, or optionally substituted $C_{1-4}$alkyl; preferably **$R^2$, $R^3$, $R^4$** and **$R^7$** are each independently H, and **$R^1$, $R^5$,** and **$R^6$,** are each independently $C_{1-6}$alkyl;

**X** is $-N(SiR^{27}{}_3)_2$, $C_{1-12}$alkyl, $C_{1-12}$alkoxy, $-NR^9R^{10}$ or $-BH_4$; preferably **X** is $-N(SIR^{27}{}_3)_2$, $C_{1-6}$alkyl, $C_{1-6}$alkoxy, or $-NR^9R^{10}$; preferably **X** is $-N(SiR^{27}{}_3)_2$, $C_{1-6}$alkyl, $C_{1-3}$alkoxy, or $-NR^9R^{10}$; preferably **X** is $-N(SiR^{27}{}_3)_2$;

each **$R^{27}$** is independently selected from hydrogen and $C_{1-6}$alkyl;

each **$R^9$** and **$R^{10}$** is independently selected from hydrogen and $C_{1-6}$alkyl;

**$R^{11}$** and **$R^{12}$** are each independently $C_{1-6}$alkyl; preferably, **$R^{11}$** and **$R^{12}$** are each independently $C_{1-6}$alkyl; preferably, **$R^{11}$** and **$R^{12}$** are each independently $C_{1-4}$alkyl; for example, **$R^{11}$** and **$R^{12}$** can be each independently selected from the group consisting of methyl, ethyl, *n*-propyl, *i*-propyl, 2-methyl-ethyl, *n*-butyl, *i*-butyl and *t*-butyl; preferably, **$R^{11}$** and **$R^{12}$** can be each independently selected from the group consisting of methyl, ethyl, *i*-propyl and *t*-butyl; for example **$R^{11}$** and **$R^{12}$** can be each independently selected from *i*-propyl or *t*-butyl; preferably, **$R^{11}$** and **$R^{12}$** are *t*-butyl;

**$R^{13}$, $R^{14}$,** and **$R^{15}$** are each independently $C_{1-6}$alkyl; preferably, **$R^{13}$, $R^{14}$** and **$R^{15}$** are each independently $C_{1-6}$alkyl; preferably **$R^{13}$, $R^{14}$** and **$R^{15}$** are each independently $C_{1-4}$alkyl, for example, **$R^{13}$, $R^{14}$** and **$R^{15}$** can be each independently selected from the group consisting of methyl, ethyl, *n*-propyl, *i*-propyl, 2-methyl-ethyl, *n*-butyl, *i*-butyl and *t*-butyl; for example, **$R^{13}$, $R^{14}$** and **$R^{15}$** can be each independently selected from the group consisting of methyl, ethyl, *i*-propyl and *t*-butyl; for example, **$R^{13}$, $R^{14}$** and **$R^{15}$** are each independently selected from methyl or ethyl; preferably, **$R^{13}$, $R^{14}$** and **$R^{15}$** are each independently methyl; or

**$R^{13}$** and **$R^{14}$** are covalently bound to each other and are each a methylene and **$R^{15}$** is $C_{1-10}$alkyl; preferably **$R^{15}$** is $C_{1-6}$alkyl; preferably, **$R^{15}$** is $C_{1-4}$alkyl; for example **$R^{15}$** can be selected from the group consisting of methyl, ethyl, *n*-propyl, *i*-propyl, 2-methyl-ethyl, *n*-butyl, *i*-butyl and *t*-butyl; for example **$R^{15}$** can be selected from the group consisting of methyl, ethyl, *i*-propyl and *t*-butyl; for example **$R^{15}$** can be selected from methyl or ethyl; for example **$R^{15}$** can be methyl;

**$X^{11}$** is selected from $C_{1-6}$alkyl, $-OR^{16}$, and $-N(SiR^{17}{}_3)_2$;

**$R^{16}$** is $C_{1-10}$alkyl; and

each $\mathbf{R^{17}}$ is independently selected from hydrogen and $C_{1-6}$alkyl;

preferably, $\mathbf{X^{11}}$ is selected from $C_{1-6}$alkyl, $-OR^{16}$, or $-N(SiR^{17}{}_3)_2$, $\mathbf{R^{16}}$ is $C_{1-6}$alkyl, and each $\mathbf{R^{17}}$ is independently selected from hydrogen and $C_{1-6}$alkyl; preferably, $\mathbf{X^{11}}$ is selected from $C_{1-4}$alkyl, - $OR^{16}$, or $-N(SiR^{11}{}_3)_2$, $\mathbf{R^{16}}$ is $C_{1-4}$alkyl, and each $\mathbf{R^{11}}$ is independently hydrogen or $C_{1-4}$alkyl; for example $\mathbf{X^{11}}$ can be selected from the group consisting of methyl, ethyl, *n*-propyl, *i*-propyl, 2-methyl-ethyl, *n*-butyl, *i*-butyl and *t*-butyl, or $-OR^{16}$, or $-N(SiR^{17}{}_3)_2$, $\mathbf{R^{16}}$ can be selected from the group consisting of methyl, ethyl, *n*-propyl, *i*-propyl, 2-methyl-ethyl, *n*-butyl, *i*-butyl and *t*-butyl, and each $\mathbf{R^{17}}$ can be independently selected from the group consisting of hydrogen, methyl, ethyl, *n*-propyl, *i*-propyl, 2-methyl-ethyl, *n*-butyl, *i*-butyl and *t*-butyl; preferably, $\mathbf{X^{11}}$ can be selected from the group consisting of methyl, ethyl, *i*-propyl and *n*-butyl, or $-OR^{16}$, $\mathbf{R^{16}}$ can be selected from the group consisting of methyl, ethyl, *i*-propyl and *t*-butyl; preferably, $\mathbf{X^{11}}$ can be selected from $-OR^{16}$, $\mathbf{R^{16}}$ can be selected from the group consisting of methyl, ethyl, *i*-propyl and *t*-butyl; preferably, $\mathbf{X^{11}}$ can be $-OR^{16}$, and $\mathbf{R^{16}}$ is ethyl.

In the present description, an inert functional group is defined as a group containing one or several heteroatoms selected from O, N, S or halogen, that is(are) not reactive in the polymerization system neither as an initiating species nor as a chain transfer agent.

In one embodiment, $\mathbf{R^{11}}$ and $\mathbf{R^{12}}$ are each independently $C_{1-6}$alkyl, preferably $C_{1-4}$alkyl.

In one embodiment, $\mathbf{R^{13}}$**,** $\mathbf{R^{14}}$ and $\mathbf{R^{15}}$ are each independently $C_{1-6}$alkyl, preferably $C_{1-4}$alkyl.

In one embodiment, $\mathbf{X^{11}}$ is selected from $C_{1-6}$alkyl, $-OR^{16}$, or $-N(SiR^{17}{}_3)_2$, $\mathbf{R^{16}}$ is $C_{1-6}$alkyl, and each $\mathbf{R^{17}}$ is independently selected from hydrogen and $C_{1-4}$alkyl.

In one embodiment, the catalyst is selected from [(NNO)ZnEt], $[BDI]Zn(N(SiMe_3)_2)$, [BDI]Zn(Et), and $\{[BDI]Zn(OR^{30})\}_2$, wherein $\mathbf{R^{30}}$ is $C_{1-6}$alkyl; preferably the catalyst is [(NNO)ZnEt].

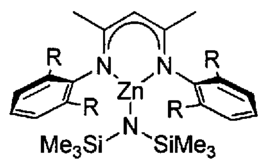

R = $^{i}$Pr

**(BDI)Zn(NTMS$_2$)**

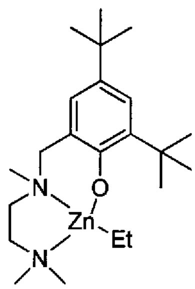

**(NNO)ZnEt**

Suitable catalyst also include metal amides such as bis(trimethylsilyl)amides such as $Y[N(SiMe_3)_2]_3$; [Y] + L1; and [Y] + L2, wherein

**L1** corresponds to the tetradentate ligand depicted below and **L2** corresponds to the tetradentate ligand depicted below

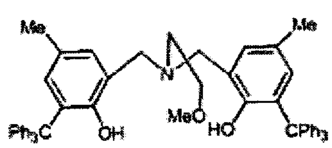

**L1**

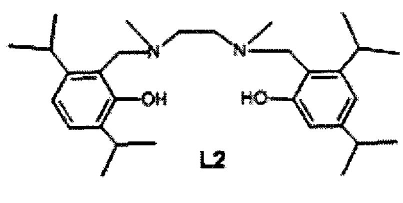

**L2**

In one embodiment, the catalyst is selected from complexes of formulae $M(OSO_2CF_3)_m$, $M(N(OSO_2CF_3)_2)_m$, $M(R^{21}C(O)CR^{23}{}_2C(O)R^{23})_m$, and $(R^{24}CO_2)_mM$, wherein **M** is a metal of Group 2, 3, including the lanthanide series, or Group 12, 13, 15, wherein each $\mathbf{R^{23}}$ is independently an optionally substituted $C_{1-12}$alkyl, wherein each $\mathbf{R^{24}}$ is independently a perfluorinated $C_{1-12}$alkyl or an aryl, and wherein **m** is the valence of M.

In one embodiment, all $\mathbf{R^{23}}$ are the same and are selected from $CH_3$ and $CF_3$, or $\mathbf{R^{24}}$ is selected from $(C_6F_5)$, $(CF_3)$, and $CF_3(CF_2)_P$, wherein **p** is an integer ranging between 1 and 6.

In one embodiment, the catalyst is a Lewis acidic metal salt selected from $Al(OTf)_3$, $Al(NTf_2)_3$, $Mg(OTf)_2$, $Ca(OTf)_2$, $Zn(OTf)_2$, $Sc(OTf)_3$, $Bi(OTf)_3$, $Fe(acac)_3$, $Al(OCOCF_3)_3$, $Al(hfacac)_3$, $Zn(OCOCF_3)_2$, $Zn(BF_4)_2$, $Zn(acac)_2$, $Zn(hfacac)_2$, and $Zr(acac)_4$.

Non-limiting examples of suitable catalyst, include catalyst selected from the group comprising dimeric phosphazene bases, or amines or guanidines, organic acids, sparteine, thiourea-amino derivaties, N-heterocyclic carbenes,

4-(dialkylamino)pyridines, amidines, phosphorus based phospines, phosphazenium derivatives and phosphazenes, strong and weak Bronsted acids, as described in M Fèvre, J Vignolle, Y Gnanou, and D Taton, Organocatalyzed Ring-Opening Polymerizations, 2012 Elsevier B.V.; and in Chem. Soc. Rev., 2013, 42, 2142.

In one embodiment, the catalyst can be selected from 1,8-diazabicyclo[5.4.0]undec-7-ene (DBU), 4-dimethylaminopyridine (DMAP), 1,5,7-triazobicyclo-[4,4,0]dec-5-ene (TBD), and *tert*-butylimino-1,3-dimethylperhydro-1,3,2-diazaphosphine (BEMP); $Y[N(SiMe_3)_2]_3$; [Y] + L1; or [Y] + L2; preferably TBD, BEMP; $Y[N(SiMe_3)_2]_3$; [Y] + L1; or [Y] + L2; preferably TBD, BEMP.

In one embodiment, the process is performed in the presence of a compound of formula (IV),

$R^8$-OH (IV)

wherein $\mathbf{R^8}$ is selected from the group consisting of $C_{1-20}$alkyl, $C_{3-30}$aryl, and $C_{6-30}$aryl$C_{1-20}$alkyl optionally substituted by one or more substituents selected from the group consisting of halogen, hydroxyl, and $C_{1-6}$alkyl. Preferably, $\mathbf{R^8}$ is selected from $C_{3-12}$alkyl, $C_{6-10}$aryl, and $C_{6-10}$aryl$C_{3-12}$alkyl, optionally substituted by one or more substituents, each independently selected from the group consisting of halogen, hydroxyl, and $C_{1-6}$alkyl; preferably, $\mathbf{R^8}$ is selected from $C_{3-12}$alkyl, $C_{6-10}$aryl, and $C_{6-10}$aryl$C_{3-12}$alkyl, optionally substituted by one or more substituents, each independently selected from the group consisting of halogen, hydroxyl and $C_{1-4}$alkyl. The alcohol can be a polyol such as diol, triol or higher functionality polyhydric alcohol. The alcohol may be derived from biomass such as for instance glycerol or 1,3-propanediol or any other sugar-based alcohol such as for example erythritol. The alcohol can be used alone or in combination with another alcohol.

In one embodiment, the compound of formula (IV) is selected from the group comprising 1-octanol, isopropanol, 1,3-propanediol, trimethylolpropane, 2-butanol, 3-buten-2-ol, 1,3-butanediol, 1,4-butanediol, 1,6-hexanediol, 1,7-heptanediol, benzyl alcohol, 4-bromophenol,1,4-benzenedimethanol, and (4-trifluoromethyl)benzyl alcohol; preferably, said compound of formula (IV) is selected from 1-octanol, isopropanol, and 1,4-butanediol.

In one embodiment, the molar ratio of compound of formula (IV) versus the catalyst is of at least 0. For example, the molar ratio of the co-initiator of the polymerization (compound of formula (IV)) to the catalyst can be of from about 0 to about 1000, for example from about 1 to about 1000, for example of from about 1 to about 100, for example of from about 1 to about 5.

In some embodiments, the present process allows to tailor the molecular weight of the polycarbonate by adapting the molar ratio of compound of formula (IV) versus the catalyst.

In an embodiment, the process is performed in the presence or absence (in bulk) of solvent. In one embodiment, the process is performed in the presence of a solvent. In a preferred embodiment, the solvent is selected from an aliphatic or aromatic hydrocarbon, an ether, a halogenated solvent, and a $C_{1-6}$alkyl carbonate. In another embodiment, the present process is performed in bulk.

The process can be performed with or without solvent, or can be performed in a minimum amount of solvent. In an embodiment, said minimum amount of solvent can be the solvent necessary to dissolve the catalyst. The solvent can be an aromatic or aliphatic hydrocarbon, an ether, or an halogenated solvent such as chlorinated solvent.

In an embodiment, the solvent is selected from an alkane such as hexane or heptane; an aromatic hydrocarbon such as toluene; an ether such as tetrahydrofuran (THF); and a chlorinated solvent such dichloromethane; a $C_{1-6}$alkyl carbonate such as dimethyl carbonate; preferably the solvent is an aromatic hydrocarbon, such as toluene.

In one embodiment, the process is performed at a temperature of at least 20 °C. In some embodiments, the process is performed at a temperature of at most 150 °C. For example, the process can be performed, preferably at a temperature of at least 20 °C and at most 150 °C, for example, the process can be performed, preferably at a temperature of at least 40 °C and at most 120 °C, for example at least 50 °C and at most 100 °C, for example at a temperature of at least 50 °C and at most 90 °C, for example at a temperature of between 50 °C and 85 °C, for example at a temperature of between 55 °C and 85 °C.

In an embodiment, the ratio of the compounds of formula (I) to the catalyst to the compound of formula (IV) can be from 25:1:0 to 5000:1:0, from 25:1:1 to 5000:1:50. In an embodiment, the ratio of the cyclic ester to the compounds of formula (I) to the catalyst to the compound of formula (IV) can be for example 100:100:1:1.

In some embodiments, the process can be carried out with unpurified compound of formula (I) optionally with an unpurified cyclic ester monomer. The process can also be carried out with the resulting (co-)polymer being crystallized one or more times in solvent and dried under vacuum before use. The solvent used during crystallization can be the same or different from the solvent used during the polymerization process.

In an embodiment, the process can be performed by contacting said compound of formula (I) optionally with the cyclic ester monomer with the catalyst and optionally a co-initiator, in a reactor equipped with an agitator, for instance a high viscosity agitator.

In an embodiment, the process can be performed by contacting compound of formula (I), the catalyst, optionally the cyclic ester, and optionally the co-initiator, optionally in the presence of a solvent. In some embodiments, the process

can be performed by contacting the catalyst, and the co-initiator, and subsequently adding compound of formula (I), optionally the cyclic ester, optionally in the presence of a solvent.

In an embodiment, the process can be performed by contacting compound of formula (I), optionally the cyclic ester, the catalyst, and optionally the co-initiator, under inert atmosphere, for example in the presence of argon or nitrogen.

The present invention also encompasses a process for the synthesis of a compound of formula (I) as defined in any one of the embodiments herein, a stereoisomer or a racemate thereof, comprising the step of contacting a compound of formula (V), a stereoisomer, or a racemate thereof; with at least one reagent selected from the group comprising an alkyl- or cycloalkyl-chloroformate of formula (VI); an urea; a phosgene or derivative/substitute of phosgene, e.g. triphosgene; a cyclic carbonate; and a carbon dioxide;

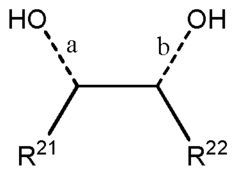 (V) 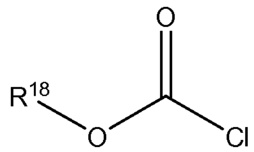 (VI),

wherein

**$R^{18}$** is $C_{1-6}$alkyl or $C_{3-6}$cycloalkyl; and
each **$R^{21}$, $R^{22}$,** and the dotted bonds "a" and "b" are, independently, as defined in any one of the embodiments herein.
In some embodiments, **$R^{18}$** is $C_{1-6}$alkyl; and
**$R^{21}$** and **$R^{22}$** taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms to which they are attached form a ring selected from $C_{5-6}$cycloalkylene, and $C_{6-10}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms to which they are attached form a ring selected from $C_{5-6}$cycloalkylene, and phenylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms to which they are attached form a $C_{5-6}$cycloalkylene, preferably a cyclohexylene; optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; for example one or more substituents each independently selected from halo, hydroxyl, $C_{1-6}$alkyl, $C_{1-6}$alkoxy, or $C_{1-6}$alkylthio.

Preferably, the present invention provides a process for the synthesis of a compound of formula (I) a stereoisomer or racemate thereof, comprising the step of contacting a compound of formula (V), a stereoisomer, or racemate thereof; with at least one reagent selected from the group consisting of an alkyl- or cycloalkyl-chloroformate of formula (VI); an urea; a phosgene or derivative thereof; a cyclic carbonate; and carbon dioxide;

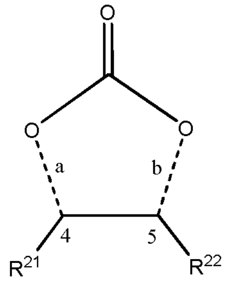
(I)

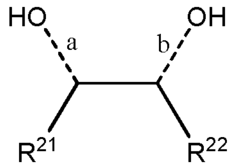
(V)
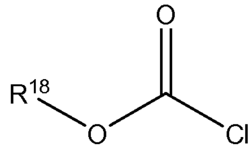
(VI),

wherein

each dotted bond "a" and "b" independently represents a solid bond, a wedged bond, or a hashed wedged bond;
**$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino,
**$R^{18}$** is $C_{1-6}$alkyl, or $C_{3-6}$cycloalkyl, preferably $C_{1-6}$alkyl.

In some preferred embodiments, **$R^{18}$** is $C_{1-4}$alkyl; and **$R^{21}$** and **$R^{22}$** taken together with the carbon atoms to which they are attached form a ring selected from $C_{3-9}$cycloalkylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms to which they are attached form a ring selected from $C_{5-6}$cycloalkylene, and $C_{6-10}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1}$-$_{6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms to which they are attached form a ring selected from $C_{5-6}$cycloalkylene, and phenylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms to which they are attached form $C_{4-6}$cycloalkylene, preferably a cyclohexylene; optionally substituted with one or more substituents each independently selected from halo, hydroxyl, $C_{1-6}$alkyl, halo$C_{1-6}$alkyl, $C_{1-6}$alkoxy, halo$C_{1}$-$_{6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; for example one or more substituents each independently selected from halo, hydroxyl, $C_{1-6}$alkyl, $C_{1-6}$alkoxy, or $C_{1-6}$alkylthio.

In an embodiment, said compound of formula (VI) is a $C_{1-6}$alkyl chloroformate, such as ethyl chloroformate.

In an embodiment, the reaction can be performed in the presence of a suitable base such as a trialkylamine, e.g. triethylamine.

In an embodiment, the reaction can be performed in the presence of a suitable solvent such as tetrahydrofuran, diethylether or other ethers.

The reaction can be performed at a temperature ranging from 0 °C to 60 °C, for example at room temperature.

The present invention also encompasses a polycarbonate or copolymer thereof comprising a recurring unit of formula (VII1) and/or (VII2),

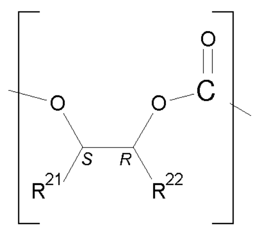
(VII1)
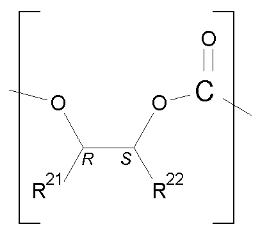
(VII2)

wherein

**$R^{21}$** and **$R^{22}$** taken together with the carbon atoms 4 and 5 to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

In some embodiments, **$R^{21}$** and **$R^{22}$** are each independently selected from $C_{1-6}$alkyl, $C_{3-6}$cycloalkyl, and $C_{6-10}$aryl; preferably $C_{1-6}$alkyl, or $C_{3-6}$cycloalkyl; more preferably $C_{1-6}$alkyl; yet more preferably $C_{1-4}$alkyl; for example **$R^{21}$** and **$R^{22}$** are each independently methyl, ethyl or propyl; preferably **$R^{21}$** is methyl and **$R^{22}$** is methyl.

Preferably, the present invention also encompasses a polycarbonate or copolymer thereof comprising a recurring unit of formula (VII1) and/or (VII2), wherein

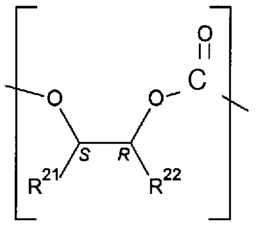
(VII1)
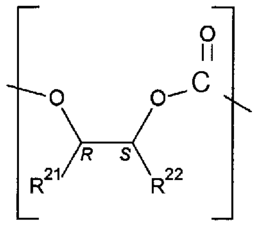
(VII2)

**$R^{21}$** and **$R^{22}$** taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

In some embodiments, **$R^{21}$** and **$R^{22}$** taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms to which they are attached form a ring selected from $C_{3-9}$cycloalkylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{3-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms to which they are attached form a ring selected from $C_{3-6}$cycloalkylene, and $C_{6-10}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably **$R^{21}$** and **$R^{22}$** taken together with the carbon atoms to which they are attached form $C_{3-6}$cycloalkylene, preferably a $C_{4-6}$cycloalkylene, preferably a $C_{5-6}$cycloalkylene, preferably a cyclohexylene; optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; for example one or more substituents each independently selected from halo, hydroxyl, $C_{1-6}$alkyl, $C_{1-6}$alkoxy, or $C_{1-6}$alkylthio.

In some embodiment, said polycarbonate or copolymer thereof comprises recurring units each independently represented by the following formula (VIIA) or (VIIB) or (VIIC);

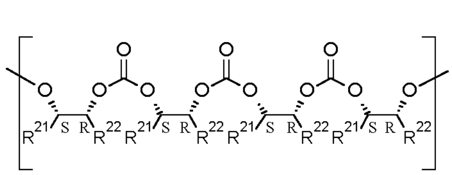
(VIIA)

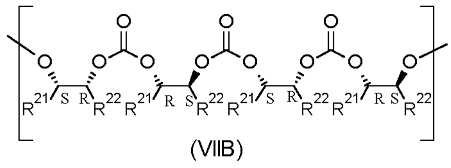
(VIIB)

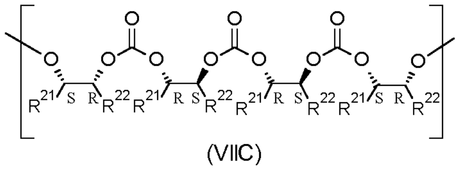

(VIIC)

wherein $R^{21}$ and $R^{22}$ have the same meaning as that defined in any one of the embodiments herein. In some embodiments;

$R^{21}$ and $R^{22}$ taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably $R^{21}$ and $R^{22}$ taken together with the carbon atoms 4 and 5 of the cyclic carbonate form a ring selected from $C_{3-9}$cycloalkylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably $R^{21}$ and $R^{22}$ taken together with the carbon atoms 4 and 5 of the cyclic carbonate form a ring selected from $C_{3-6}$cycloalkylene, and $C_{6-10}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably $R^{21}$ and $R^{22}$ taken together with the carbon atoms 4 and 5 of the cyclic carbonate form $C_{3-6}$cycloalkylene, optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; for example one or more substituents each independently selected from halo, hydroxyl, $C_{1-6}$alkyl, $C_{1-6}$alkoxy, or $C_{1-6}$alkylthio.

In a preferred embodiment, $R^{21}$ and $R^{22}$ taken together with the carbon atoms 4 and 5 of the cyclic carbonate form a cyclohexylene optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

In some embodiment, said polycarbonate or copolymer thereof comprises recurring units each independently represented by the following formula (VIIA1) or (VIIB1) or (VIIC1);

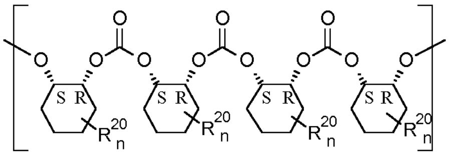

(VIIA1)

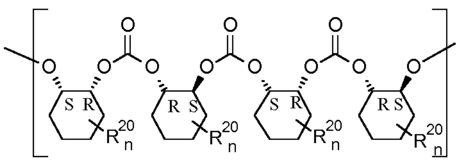

(VIIB1)

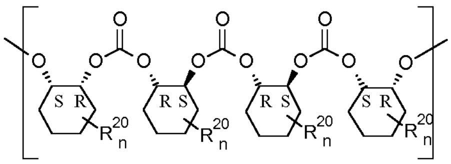

(VIIC1)

wherein **$R^{20}$** is selected from halo, hydroxyl, $C_{1-6}$alkyl, halo$C_{1-6}$alkyl, $C_{1-6}$alkoxy, halo$C_{1-6}$alkoxy, $C_{6}$-$_{12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; preferably halo, hydroxyl, $C_{1-6}$alkyl, halo$C_{1-6}$alkyl, $C_{1-6}$alkoxy, halo$C_{1}$-$_{6}$alkoxy, $C_{6-12}$aryl, $C_{1-6}$alkylthio; more preferably halo, hydroxyl, $C_{1-6}$alkyl, $C_{1-6}$alkoxy, $C_{1-6}$alkylthio; and

**n** is an integer selected from 0, 1, 2, 3, or 4; preferably 0, 1, 2, or 3, preferably 0, 1, or 2, more preferably 0 or 1, yet more preferably 0.

In some embodiment, said polycarbonate or copolymer thereof comprises recurring units each independently represented by the following formula (VIIA2) or (VIIB2) or (VIIC2);

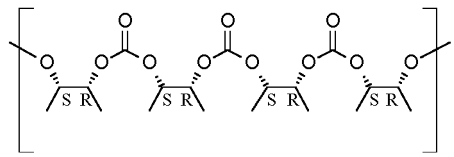

(VIIA2)

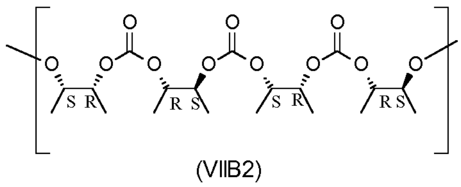

(VIIB2)

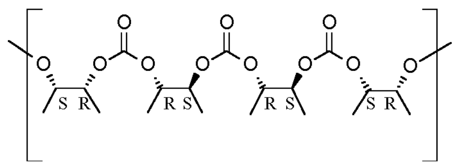

(VIIC2)

The present invention also encompasses a polycarbonate or copolymer thereof comprising recurring unit of formula (VI13) and/or (VI14),

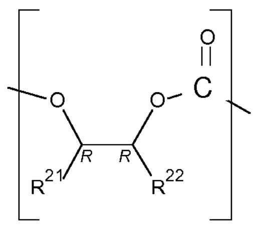

(VII3)

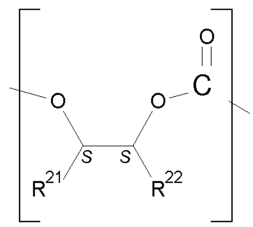

(VII4)

wherein

**$R^{21}$** and **$R^{22}$** taken together with the carbon atoms 4 and 5 to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6}$-$_{12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and

wherein said polycarbonate or copolymer thereof is characterized by a $^{13}C$ NMR spectrum comprising only one signal present in the areas ranging from $\delta$ 150 to 155 ppm. In an embodiment, said polycarbonate or copolymer thereof also comprises less than 2% of monomer having at least one chiral center of inverse configuration compared to the enantiopure monomers. This percentage can be measured after hydrolysis of the polymer, by chiral chromatography of the vicinal diol resulting from the hydrolysis.

Preferably, the present invention also encompasses a polycarbonate or copolymer thereof comprising recurring units of formula (VII3) or of formula (VII4),

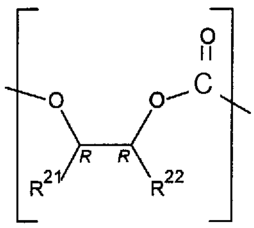 (VII3) 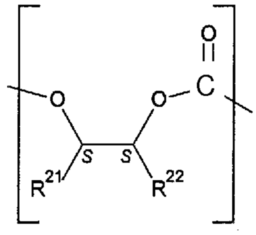 (VII4)

wherein

**$R^{21}$** and **$R^{22}$** taken together with the carbon atoms 4 and 5 to which they are attached form a ring selected from $C_{3-9}$cycloalkylene, $C_{5-9}$cycloalkenylene, and $C_{3-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and

wherein said polycarbonate or copolymer thereof is characterized by a $^{13}C\{^1H\}$ NMR spectrum comprising only one signal present in the areas ranging from $\delta$ 150 to 155 ppm, preferably characterized by a high resolution (100 MHz or higher) $^{13}C\{^1H\}$ NMR spectrum comprising only one sharp resonance present in the carbonyl areas ranging from $\delta$ 150 to 155 ppm. In an embodiment, said polycarbonate or copolymer thereof also comprises less than 2% of monomer having at least one chiral center of inverse configuration compared to the enantiopure monomers. This percentage can be measured after hydrolysis of the polymer, by chiral chromatography of the vicinal diol resulting from the hydrolysis.

Hydrolysis of the polycarbonate can be performed as described by Nozaki et al.(Nozaki, K.; Nakano, K.; Hiyama, T. J. Am. Chem. Soc. 1999, 121, 11008-11009). An example of protocol is described herein wherein, a round-bottomed flask was charged with PCHC (50 mg), tetrahydrofuran (5 mL), methanol (1 mL) and NaOH in $H_2O$ (2 M, 10 mL). The resulting mixture was stirred at room temperature for 24 h. Then, it was neutralized with aqueous HCl (1 M), and concentrated to 4 ml by evaporation. The solution was then extracted with ethyl acetate (4×5 mL). The combined organic layers were dried over $MgSO_4$ and concentrated by evaporation. Further purification of the crude product by silica-gel column chromatography (ethyl acetate as an eluent) gave (1*R*,2*R*)-cyclohexane-1,2-diol.

In Scheme 1 below, there is provided a classification of poly(cyclohexylene carbonate) according to the relative stereochemistry of the carbon at which the main chain enters the cyclohexylene unit (a), the carbon at which the main chain enters and leaves the cyclohexylene unit (b) and adjacent carbons (c).

The term "diad" refers to two adjacent structural units in a polymer molecule. If the diad consists of two identically oriented units, the diad is called a meso diad [m] reflecting similar features as a meso compound. If the diad consists of units oriented in opposition, the diad is called a racemo diad [r] as in a racemic compound.

The term "isotactic triad [mm]" refers to two adjacent meso diads. The term "syndiotactic triad [rr]" refers to two adjacent racemo diads. The term "heterotactic triad [rm]" is composed of a meso diad adjacent to a racemo diad.

The tem "tetrad" refers to three adjacent diads.

The [m] and [r] assignments used herein represent the relative stereochemistry of the carbons of the cycloalkylene units (like cyclohexylene units of the Scheme 1 below) at which the main chain enters (a), not the relative stereochemistry of the two carbons on either side of the carbonate unit (c). Therefore a racemic diad ([r]) represents two entire monomer units that have been incorporated in the opposite stereochemical orientation.

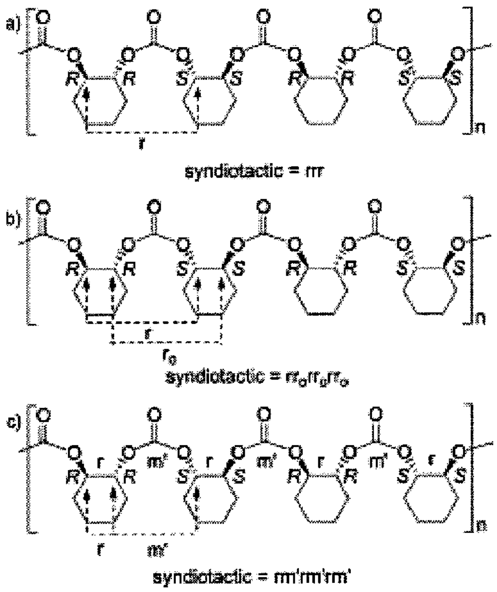


Scheme 1

Scheme 2 describes the different limit microstructures reachable from enantiopure (*R,R*), *rac* (*R,R*/*S,S*) and meso (*R,S*) cyclohexylene carbonate and the corresponding nomenclature considering the absolute configuration at the C4 carbon atom in the cyclohexylene ring.

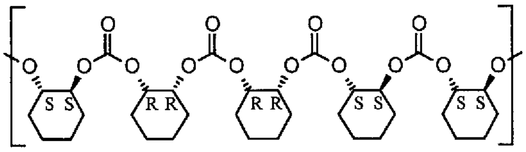

Atactic-rac Polymer

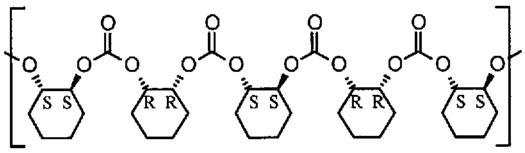

Syndiotactic-rac Polymer

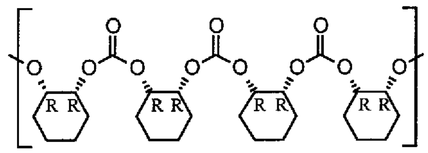

Isotactic-rac Polymer (only the RR chain is shown)

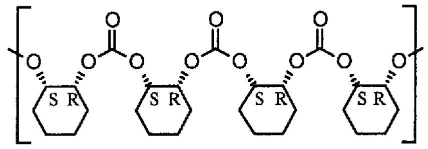

Isotactic-meso Polymer

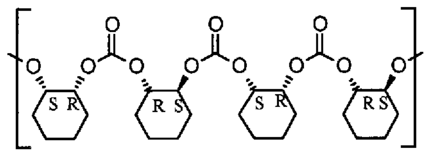

Syndiotactic-meso Polymer

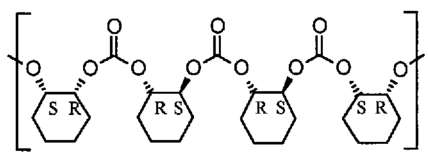

Atactic-meso Polymer

Scheme 2

The present invention thereof encompasses polycarbonate as defined in the third aspect of the invention herein comprising isotactic-meso microstructure, or syndiotactic-meso microstructure, or atactic-meso microstructure.

The process of the present invention allows control of tacticity: using a *trans* enantiopure (*R,R*)-CHC allows to obtain a perfect isotactic polymer; on the other hand, using a *cis* (R,S)-CHC allows to obtain new architectures (as shown above in Scheme 2).

The present invention also encompasses polycarbonates and copolymers thereof obtainable by any one of the processes of the invention. The present invention also encompasses polycarbonates and copolymers thereof comprising at least one recurring unit of formula (VII1), (VII2), (VII3), or (VII4); obtainable by any one of the processes of the invention. The present invention also encompasses polycarbonates and copolymers thereof comprising at least one recurring unit of formula (VII1), (VII2), (VII3), or (VII4) according to the third or fourth aspect of the invention; obtainable by any one of the processes of the invention. In an embodiment, the invention encompasses random (co-) polymers obtainable by the process according to any one of the embodiments presented herein.

In some embodiments, the inventors found that the (co-)polymers prepared with the process of the invention can have high molar mass and narrow molar mass distribution.

The "mass" or "molar mass distribution" or "dispersity" is defined by the ratio $M_w/M_n$ of the weight average molecular weight $M_w$ to the number average molecular weight $M_n$ as determined by Size Exclusion Chromatography.

In an embodiment, the present process allows the preparation of (co-)polymers, with a number average molecular weight $M_n$ which can range between about 250 and about 100,000 g/mol. For example the $M_n$ of the (co-)polymers obtained can range between about 2,500 and about 35,000 g/mol. Specific $M_n$ values of the (co-)polymers obtained are provided in the tables below.

The $M_n$ can be measured by any adequate technique known to the skilled persons for molar mass determination, for instance by chromatography such as size exclusion chromatography (SEC, also referred to as gel permeation chromatography, GPC)) in tetrahydrofuran (THF) at 20 °C calibrated with polystyrene standards.

In an embodiment, the mass molar distribution of the (co-)polymers obtained can range between about 1.10 and about 2.00, preferably below 2.00. Specific $M_w/M_n$ ratios of the (co-)polymers obtained are provided in the tables below.

The process of the present invention allows improved incorporation of compound of formula (I) monomer into

the polycarbonate backbone of the resulting polymer or copolymers thereof. The incorporation is calculated as the molar percentage (mol-%) of compound(s) of formula (I) monomer converted to (co-)polymer as follows: [molar amount of compound(s) of formula (I) in the precipitated (co-)polymer obtained after the process divided by the sum of the molar amounts of compound(s) of formula (I) and optionally cyclic ester(s) in the precipitated (co-)polymer obtained after the process] x 100. The molar amounts of compound(s) of formula (I) and optionally cyclic ester(s) in the precipitated (co-) polymer obtained after the process can be measured by NMR spectroscopy. Improved conversions of compound(s) of formula (I) to (co-)polymers are reported in the tables below at particular operating conditions, employing specific catalytic systems, and specific cyclic esters.

The present invention also encompasses an article comprising a polycarbonate or copolymer thereof according to the invention, or a polycarbonate or copolymer thereof prepared according to any of the process of the invention.

The present invention can be further illustrated by the following examples, although it will be understood that these examples are included merely for purposes of illustration and are not intended to limit the scope of the invention unless otherwise specifically indicated.

## EXAMPLES

### Example 1: Preparation of poly(cyclohexene carbonate) (PCHC) by ring-opening polymerization of *trans racemic* cyclohexene carbonate ((±)*trans*-CHC)

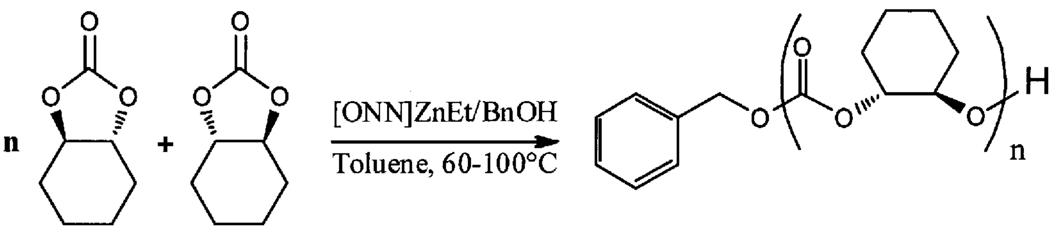


(+/-)trans-CHC

Isotactic-rac enriched PCHC

Scheme 3

Different ring opening polymerizations (ROP) of (±)-*trans*-CHC were performed with various catalysts, and reacting conditions. The conditions and results are listed in Table 1. One combination of specific catalyst and reacting conditions is depicted in Scheme 3 above. The polymerization was performed using the following catalytic systems:

DBU, (BDI)Zn($NTMS_2$), and [(NNO)ZnEt]. DBU was commercially available from Aldrich. DBU was dried over calcium hydride over 24h before being distilled under vacuum and stored under inert atmosphere.

(BDI)Zn($NTMS_2$) was synthesized as described in Chamberlain B. M., Cheng M., Moore D. R., Ovitt T. M., Lobkovsky E. B., Coates G. W.; J. Am; Chem. Soc., 2001, 123, 3229-3230.

[(NNO)ZnEt] was prepared as described in (Williams C. K., Breyfogle L. E., Choi S. K., Nam W., Young V. G., Hillmeyer M. A., Tolman W. B.; J. Am. Chem. Soc., 2003, 125, 11350-11359).

In some cases, these metallic species were combined to an exogenous protic source, typically an alcohol (benzyl alcohol, BnOH). Benzyl alcohol (from Acros) was distilled over Mg turnings under argon atmosphere and kept over activated 3-4 Å molecular sieves. $CDCl_3$ was dried over 3-4 Å molecular sieves.

The polymerization was performed as follows: In a glovebox, at room temperature, the catalyst was added to a flask prior to the addition of toluene (volume calculated to have a final concentration in monomer of 2 mol.$L^{-1}$) and the optional addition of BnOH. The monomer (±)-trans-CHC (prepared as described in Example 3) was then added to the mixture. The mixture was stirred at the listed temperature (Table 1) over an appropriated time period.

Table 1, entries 2-9 were performed in the presence of benzyl alcohol (BnOH) with [(NNO)ZnEt] and PCHCs with a number-average molecular weight (Mn) lower than 22,000 g/mol were synthesized. The molecular weights determined by NMR ($Mn_{NMR}$) were in good agreement with the theoretical values ($Mn_{theo}$). $Mn_{NMR}$ were calculated from the relative intensity of the methylene protons from the benzylic alcohol group (Bn-C*H*$_2$-O-PCHC) compared to the one of the methine protons from the CHC units in the polymer (R-O-(CO)-O-C*H*C*H*-(C4H8)-O-(CO)-O-R).

The values of molar masses measured by SEC, calibrated from polystyrene standards and unadjusted from their difference in their hydrodynamic volume, remained consistent with $Mn_{NMR}$ and $Mn_{theo}$ for PCHCs with masses below 22,000 g/mol. Further experiments were performed at 80 °C (Table 1, entries 7-8) and at 60 °C (Table 1, entry 9). Varying the amount of BnOH equivalent (for example, Table 1, entries 12-13) allows a good tailoring of the molar masses. In addition, since the reaction proceeds by immortal ring-opening, the amounts of catalyst used can be minimized. The molecular weights determined by NMR ($Mn_{NMR}$) and SEC were in perfect agreement with the theoretical values ($Mn_{theo}$).

The reactions were performed also with different zinc catalysts, for example: [(BDI)Zn{N(TMS)$_2$}]. Molecular weights, dispersities, conversions and stereoselectivities obtained from each of the two catalyst systems were found to be similar.

Table 1

| Entry | Catalyst | [±CHC]$_0$:[Cat]$_0$:[BnOH]$_0$ | Temp. (°C) | Time (H) | Conv. CHC (%) | $Mn_{theo}$ (g/mol) | $Mn_{NMR}$ (g/mol) | $Mn_{sec}$ (g/mol) | $\frac{M_W}{M_n}$ |
|---|---|---|---|---|---|---|---|---|---|
| 1 | [(NNO) ZnEt] | 47:1:0 | 100 | 18 | 38 | 2 500 | 22 300 | 19 000 | 1.81 |
| 2 | [(NNO) ZnEt] | 50:1:1 | 100 | 18 | 72 | 5 120 | 6 750 | 7 320 | 1.28 |
| 3 | [(NNO) ZnEt] | 100:1:1 | 100 | 24 | 63 | 8950 | 9 360 | 7850 | 1.28 |
| 4 | [(NNO) ZnEt] | 50:1:1 | 100 | 24 | 86 | 6110 | 7 600 | 7800 | 1.43 |
| 5 | [(NNO) ZnEt] | 200:1:1 | 100 | 72 | 76 | 21 600 | 17 000 | 8 650 | 1.35 |
| 6 | [(NNO) ZnEt] | 250:1:5 | 100 | 72 | 82 | 5 830 | 7 500 | 5 400 | 1.39 |
| 7 | [(NNO) ZnEt] | 250:1:1 | 80 | 32 | 89 | 31 600 | 20 600 | 21700 | 1.27 |
| 8 | [(NNO) ZnEt] | 100:1:1 | 80 | 3.75 | 89 | 12 650 | 9800 | 11 000 | 1.64 |
| 9 | [(NNO) ZnEt] | 100:1:1 | 60 | 3 | 91 | 13 100 | 10 300 | 11 400 | 1.33 |
| 10 | [(BDI)Zn{N (TMS)$_2$}] | 50:1:1 | 100 | 76 | 82 | 5 800 | 7 500 | 6100 | 1.27 |
| 11 | DBU | 36:1:1 | 60 | 16 | 5 | 250 | - | Not precipitable | |
| 12 | [(NNO) ZnEt] | 250:1:5 | 60 | 4 | 92 | 6 540 | 6540 | 6 200 | 1.15 |
| 13 | [(NNO) ZnEt] | 500:1:5 | 60 | 4 | 87 | 12 400 | 12 100 | 10 850 | 1.07 |

$^1H$ NMR analysis (Figure 1) of the PCHCs obtained by ROP showed the signals corresponding to the main chain methylene hydrogens (δ 4.63 ppm, $OCH(C_4H_8)CHO$; δ 2.11 ppm, 1.70 ppm, $CH_2CH_2CH$; 8 1.46-1.34 ppm, $CH_2CH_2CH_2$). Benzyloxy resonances (δ 7.35 ppm, $OCH_2C_6H_5$; 5.14 ppm, $OCH_2C_6H_5$, respectively) and PCHC-OH (δ 4.40 ppm $OCH(C_4H_8)CHOH$, δ 3.58 ppm $OCH(C_4H_8)CHOH$, respectively) in terminal positions on the PCHC were also clearly identified. These data confirm that the polymerization was initiated by benzyl alcohol. Remarkably, there was an absence of signals corresponding to the ether bonds (δ 3.4 ppm), proving the perfect chemoselectivity (within the NMR accuracy) of this process.

The $^{13}C\{^1H\}$ NMR spectrum confirmed the chemical structure of PCHC (see Figure 2). The $^{13}C\{^1H\}$ NMR spectrum showed all the same signals in the region of carbonates (δ 153.4 and 153.3 ppm) and in the region of alkanes (δ 29.0, 28.7, 22.5 and 22.3 ppm). These signals were attributed to tetrads. The signal at δ 153.9 ppm was assigned to

the m-centered tetrads [mmm] [mmr] and [rmr] and the two signals at δ 153.4 ppm and δ 153.3 ppm were assigned to tetrads [mrm]/[rrr]. The integral ratio of the former and latter resonances was found to be 61:39. This polymer appears thus to be enriched in the isotactic-rac microstructure (Scheme 2).

The present process also allows to prepare polycarbonates with an isotacticity close to 100%. Differential scanning calorimetry (DSC) analyses were performed on a Setaram DSC 131 apparatus calibrated with indium at a rate of 10°C.min$^{-1}$, under continuous flow of helium (25 mL.min$^{-1}$), using aluminum capsules (typically 10 mg of polymer). The thermograms were recorded according to the following cycles: 30 °C to +230 °C at 10 °C.min$^{-1}$; +230°C to 30 °C at 10 °C.min$^{-1}$. The obtained PCHC had a glass transition temperature (Tg) of 121 °C (Figure 3).

**Example 2: Preparation of pure isotactic (R,R)-PCHC by ring-opening polymerization of *trans* (*R,R*)-CHC**

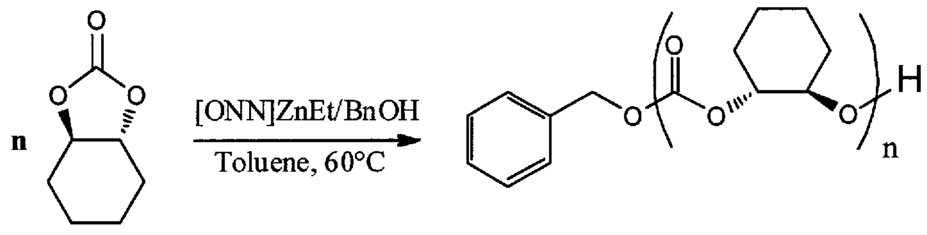


(R,R)-CHC

Isotactic-rac PCHC

Scheme 4

Different ROP of (*R,R*)-CHC (prepared as described in Example 3) were performed with the zinc complex ([(NNO)ZnEt]) (prepared as described in Example 1). Conditions are depicted in Scheme 4 above. Table 2, entries 1-3 were performed in the presence of benzyl alcohol (BnOH) with [(NNO)ZnEt] and PCHCs with a number-average molecular weight (Mn) lower than 21,000 g.mol$^{-1}$ were synthesized. The molecular weights determined by NMR ($Mn_{NMR}$) were in good agreement with the theoretical values ($Mn_{theo}$). The values of molar masses measured by SEC in chloroform (the (*R,R*)PCHCs synthesized were insoluble in THF), calibrated from polystyrene standards and unadjusted for their difference in their hydrodynamic volume, remained coherent with $Mn_{NMR}$ and $Mn_{theo}$ for PCHCs with masses below 21,000 g.mol$^{-1}$.

Table 2

| Entry | $[(R,R)CHC]_0$: [(NNO) $ZnEt]_0$: $[BnOH]_0$ | Temp. (°C) | Time (H) | Conv. CHC (%) | $Mn_{theo}$ (g/mol) | $Mn_{NMR}$ (g/mol) | $Mn_{sec}$ (g/mol) | $\frac{M_W}{M_n}$ |
|---|---|---|---|---|---|---|---|---|
| **1** | 100:1:1 | 60 | 1.5 | 94 | 13 360 | 15 800 | 21 000 | 1.34 |
| **2** | 200:1:1 | 60 | 0.8 | 68 | 19 330 | 17 000 | 21 000 | 1.30 |
| **3** | 120:1:1 | 60 | 0.75 | 87 | 14 500 | 10100 | 12 000 | 1.14 |

$^1H$ NMR analysis of a (*R,R*)-PCHC (Table 2, Entry 1) showed the same signals as an iso-enriched PCHC (Figure 1).

The $^{13}C\{^1H\}$ NMR spectrum (Table 2, Entry 1) confirmed the chemical structure of PCHC (see Figure 6). The $^{13}C\{^1H\}$ NMR spectrum showed only one signal in the region of carbonates (δ 153.95 ppm) which was attributed to m-centered tetrads [mmm] [mmr] and [rmr]. The lack of signals at δ 153.4 ppm and 153.3 ppm indicated that there is no racemo diad in the polymer thus the signal at δ 153.95 ppm in Figure 6 is only due to [mmm] tetrads. The region of alkanes shows only 2 signals (δ 29.84 and 23.21 ppm) as well.

Differential scanning calorimetry (DSC) analyses were performed on a Setaram DSC 131 apparatus calibrated with indium at a rate of 10 °C.min$^{-1}$, under continuous flow of helium (25 mL.min$^{-1}$), using aluminum capsules (typically 10 mg of polymer). The thermograms were recorded according to the following cycles: 30 °C to +280 °C at 10 °C.min$^{-1}$; +280 °C to 30 °C at 10 °C.min$^{-1}$. The obtained isotactic (*R,R*)-PCHC (Table 2, entry 2) had a glass transition temperature ($T_g$) of 130 °C, a crystallization temperature ($T_c$) of 162 °C and a melting temperature ($T_m$) of 250 °C (Figure 7). Once the melting point is reached, the polymer seems to degrade which gives an endothermic peak, and then no signal during

the second heating phase.

**Example 3: Synthesis of a meso or racemo-cyclohexene carbonate (CHC)**

(Meso or racemo) 1.2-cyclohexanediol and ethyl chloroformate were bought from Aldrich and used as received without any purification.

The synthesis of meso or trans-racemic-CHC comprised the transesterification reaction between a cyclohexanediol (meso or trans racemic, respectively) (0.5 M, 1eq) and ethyl chloroformate (2.25 eq), in the presence of triethylamine (2.25 eq), as set out in Scheme 6. The reaction was carried out in 6 h in tetrahydrofuran at room temperature, obtaining isolated CHC with a yield of 90%. Polymer formation was not observed during the reaction.

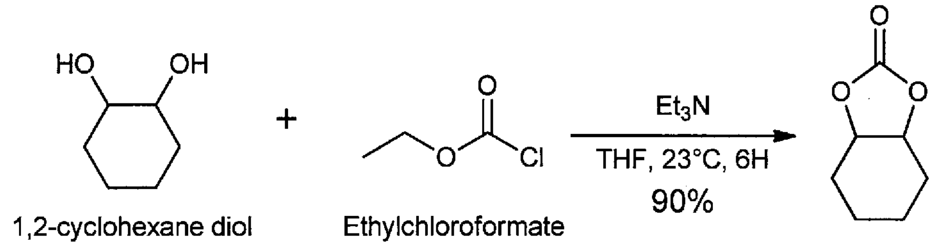


1,2-cyclohexane diol

Ethylchloroformate

CHC

Scheme 6

**Example 4: Preparation of polycarbonate copolymer by ROP copolymerization of (±)-*trans*-CHC with L-lactide**

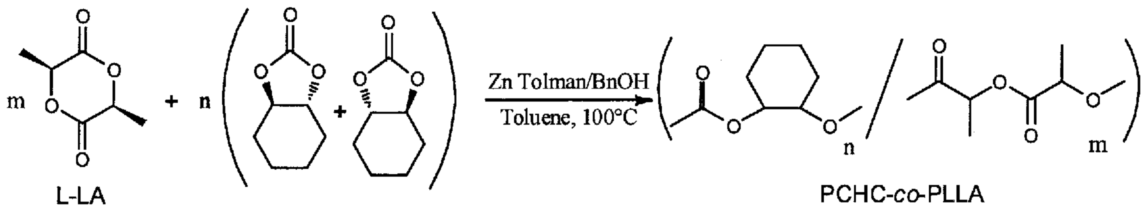


L-LA

(+/-)-transCHC

PCHC-*co*-PLLA

Scheme 5

(±)-*Trans*-CHC was prepared as described in Example 3. L-Lactide (L-LA; technical grade, Total Petrochemicals) was purified by recrystallization from a hot (80 °C) solution of concentrated 2-propanol, followed by two subsequent recrystallizations in hot toluene (105 °C). After purification, L-LA was stored at room temperature under inert atmosphere. The polymerization was performed as illustrated in Scheme 5. The conditions and results of the copolymerization by ROP of (±)-*trans*-CHC with L-lactide are shown in Table 3.

Table 3

| Catalyst | $[L\text{-}LA]_0:[\pm CHC]_0:[Cat]_0:[BnOH]_0$ | T (°C) | Time (h) | Conv. CHC (%) | Conv. L-LA (%) | $Mn_{theo}$ (g/mol) | $Mn_{NMR}$ (g/mol) | $Mn_{sec}$ (g/mol) | $\frac{M_w}{M_n}$ |
|---|---|---|---|---|---|---|---|---|---|
| **[(NNO) ZnEt]** (Zn Tolman) | 100:100:1:1 | 100 | 65 | 78 | 100 | 25 500 | 18 650 | 15 800 | 1.39 |

The $^1H$ NMR spectrum (Figure 4) showed the formation of a copolymer PLLA-co-PCHC, not a mixture of the two homopolymers PLLA and PCHC. Indeed, the signals were intense and could be clearly identified. The formation of a copolymer and not a mixture of homopolymers, was also supported by the narrow dispersity determined by SEC.

Thermal analysis of this copolymer by DSC (Figure 5) also suggested the formation of a random copolymer. The measured $T_g$ for the copolymer PLLA-co-PCHC was 78 °C, between the $T_g$ of PLLA (60 °C) and that of the PCHC

(116 °C). Differential scanning calorimetry (DSC) analyses were performed on a Setaram DSC 131 apparatus calibrated with indium at a rate of 10 °C.min$^{-1}$, under continuous flow of helium (25 mL.min$^{-1}$), using aluminum capsules (typically 10 mg of polymer). The thermograms were recorded according to the following cycles: 30 °C to +230 °C at 10 °C.min$^{-1}$; +230 °C to 30 °C at 10 °C.min$^{-1}$.

**Example 5: Synthesis of 2,3-dimethylcarbonate**

2,3-Butanediol and ethyl chloroformate were bought from Aldrich and used as received without any purification. The synthesis of 2,3-dimethylcarbonate comprised the transesterification reaction between the butane-2,3-diol (0.5 M, 1 eq) and ethyl chloroformate (2.25 eq), in the presence of triethylamine (2.25 eq), as set out in scheme 7. The reaction was carried out in 6 h in THF at room temperature. Polymer formation was not observed during the reaction.

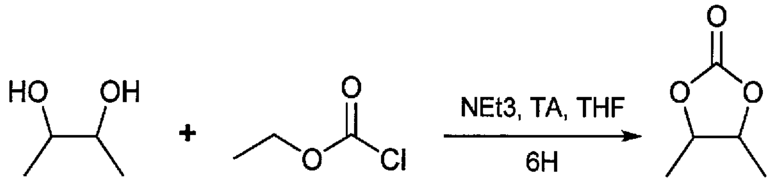


Scheme 7

**Example 6: Preparation of poly(cyclohexene carbonate) (PCHC) by ring-opening polymerization of *rac*-cyclohexene carbonate (*rac*-CHC)**

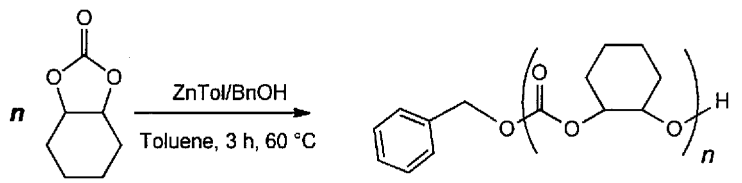


***rac*-CHC**

**PCHC**

Scheme 8

Different ring-opening polymerizations (ROP) of racemic cyclohexene carbonate (rac-CHC) were performed with various catalysts, alcohols and reacting conditions. The conditions and results are listed in Table 4. One combination of specific catalyst and reacting conditions is depicted in Scheme 8. The polymerization was performed using the following catalytic systems:

[(NNO)ZnEt], $Y[N(SiMe_3)_2]_3$, [Y] + L1, [Y] + L2, and TBD.

$Y[N(SiMe_3)_2]_3$: was commercially available from Strem Chemicals.

[Y] + L1, wherein **L1** corresponds to the tetradentate ligand depicted in Scheme 9, was prepared as described in Bouyahyi M, Ajellal N, Kirillov E, Thomas CM, Carpentier J-F. Exploring Electronic versus Steric Effects in Stereoselective Ring-Opening Polymerization of Lactide and β-Butyrolactone with Amino-alkoxy-bis(phenolate)-Yttrium Complexes. Chem Eur J. 2011;17:1872-1883.

[Y] + L2, wherein **L2** corresponds to the tetradentate ligand depicted in Scheme 9, was prepared as described in Kramer JW, Treitler DS, Dunn EW, Castro PM, Roisnel T, Thomas CM, Coates GW. Polymerization of Enantiopure Monomers Using Syndiospecific Catalysts: A New Approach to Sequence Control in Polymer Synthesis. J Am Chem Soc. 2009;131:16042-16044.

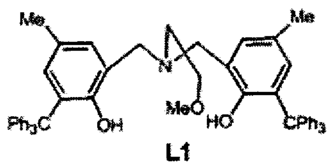
L1

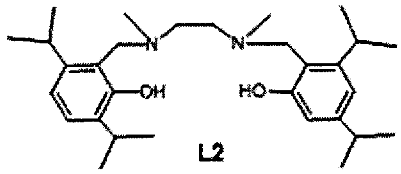
L2

Scheme 9

TBD: (1,5,7-triazobicyclo-[4,4,0]dec-5-ene) was commercially available from Aldrich. Isopropyl alcohol was commercially available from Aldrich.

Benzyl alcohol (BnOH) (from Acros) was further treated as described in Example 1.

Typical polymerization procedure: All polymerizations were performed similarly according to the following typical procedure (Table 4, entry 1): In a dry-box, benzyl alcohol (0.97 μL, 9.34 μmol), [(NNO)ZnEt] (4.0 mg, 9.34 μmol) and dry toluene (0.10 mL) were placed in a Schlenk flask equipped with a magnetic stir-bar. The mixture was stirred 10 min at room temperature to form the benzyloxide catalyst. rac-CHC (0.133 g, 0.934 mmol, prepared as described in Example 3, and dry toluene (0.13 mL) were then added. The flask and its content were placed under argon and immersed in an oil-bath at 60 °C. After stirring over 3 h, the reaction mixture was cooled down to room temperature and three drops of a 1.6 M acetic acid solution in toluene were added to quench the reaction. The solvent was evaporated and an aliquot was taken out to determine the % of CHC conversion by $^{1}H$ NMR. The crude polymer was purified by precipitation in cold methanol. This process was repeated twice to ensure complete removal of the catalyst and unreacted CHC. A white powder was obtained following drying under vacuum (0.110 g, 82% isolated yield).

The isolated yield of polymer and the % of conversion of CHC are in agreement. This result clearly indicates that all the CHC converted was transformed to high molecular weight PCHC. No decarboxylation (as evidenced by the absence of ether signals in $^{1}H$ and $^{13}C$ NMR spectra) or formation of non-precipitable oligomers was observed.

Table 4

| Entry | Catalyst | ROH | [±CHC]$_0$:[Cat]$_0$:[ROH]$_0$ | Temp (°C) | *Time (h)* | CHC Conv.[a] (%) | $Mn_{theo}$[b] (g/mol) | $Mn_{NMR}$[c] (g/mol) | $Mn_{SEC}$[d] (g/mol) | Đ$_M$ $\frac{M_W}{(M_n)}$ |
|---|---|---|---|---|---|---|---|---|---|---|
| **1** | [(NNO)ZnEt] | BnOH | 100:1:1 | 60 | 3 | 88 | 13100 | 10 300 | 11 700 | 1.1 |
| **1'** | [(NNO)ZnEt] | BnOH | 100:1:1 | 60 | 3 | 91 | 13100 | 10 300 | 11 400 | 1.2 |
| **2** | $Y[N(SiMe_3)_2]_3$ | *i*PrOH | 50:1:1 | 40 | 21 | 28 | 570 | | | |
| **3** | [Y] + L1 | *i*PrOH | 100:1:1 | 40 | 16 | 13 | 10100 | | | - |
| **4** | [Y] + L1 | *i*PrOH | 100:1:1 | 40 | 16 | 56 | 10100 | | | - |
| **5** | [Y] + L1 | *i*PrOH | 100:1:1 | 60 | 16 | 70 | 10100 | 10 600 | 13100 | 1.2 |
| **6** | [Y] + L2 | *i*PrOH | 100:1:1 | 60 | 16 | 67 | 9600 | 11 000 | 11 400 | 1.2 |
| **7** | [Y] + L2 | *i*PrOH | 50:1:1 | 40 | 16 | 83 | 6000 | 6700 | 8600 | 1.1 |
| **8** | TBD | BnOH | 100 :1:1 | 60 | 1 | 75 | 10 700 | 8300 | 7800 | 1.1 |
| **9** | TBD | BnOH | 100 :1:1 | 60 | 19 | 93 | 13 200 | 16100 | 10 000 | 1.5 |
| **10** | TBD | BnOH | 250 :1:5 | 60 | 4 | 85 | 6000 | 6100 | 6100 | 1.2 |

[a]Calculated from the $^1H$ NMR of the crude product. [b]Theoretical molar mass value of PCHC calculated from the relation: $M_{n,theo} = M_{rac\text{-}CHC} \times [rac\text{-}CHC]_0/[ROH]_0 \times conversion_{rac\text{-}CHC} + M_{ROH}$ with $M_{rac\text{-}CHC}$ = 142 g.mol$^{-1}$, $M_{iPrOH}$ = 60 g.mol$^{-1}$, $M_{BnoH}$ = 108 g.mol$^{-1}$. [c]NMR molar mass value of PCHC calculated from the integral value ratio of the signals of alkoxide methylene or methine end-group hydrogens to internal methine hydrogens. [d]Experimental number average molar mass and dispersity values determined by SEC in THF at 30 °C using polystyrene standards.

Figure 8 shows the carbonyl regions of the $^{13}C\{^1H\}$ NMR spectra (100 MHz, $CDCl_3$, 25°C) of PCHC samples synthesized by ROP of rac-CHC using two different catalysts: a) [(NNO)ZnEt] (Table 1, entry 1') and b) Yttrium complexes (Table 1, entry 5 (lighter line) and entry 6 (darker line)).

NMR analyses revealed that all the catalytic systems investigated consistently gave the same extent of isotacticity ($P_m$= *ca.* 60-76%) as determined by integration of the *mm*/*mr*/*rr* triads in the methylene region of the $^{13}C\{^1H\}$ NMR spectra. This might reflect a chain-end control mechanism.

**Example 7: Preparation of PCHC-PLLA copolymers by ROP copolymerization of *rac*-CHC with L-lactide**

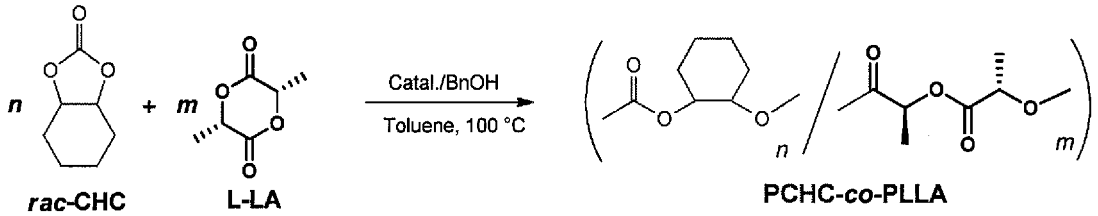


**rac-CHC** **L-LA** **PCHC-co-PLLA**

Scheme 10

Different copolymerizations of racemic cyclohexene carbonate (*rac*-CHC) and L-Lactide (L-LA) (Scheme 10) were performed with various catalysts, monomer concentrations and reacting conditions. The conditions and results are listed in Table 5. The copolymerizations were performed using the following catalytic systems: [(NNO)ZnEt] or TBD.

Typical procedure for CHC/L-LA simultaneous copolymerization was as follows: All copolymerizations were performed similarly to the following typical procedure (Table 5, entry 4). In a dry-box, benzyl alcohol (0.97 μL, 9.34 μmol), [(NNO)ZnEt] (4.0 mg, 9.34 μmol) and dry toluene (0.10 mL) were placed in a Schlenk flask equipped with a magnetic stir-bar. The mixture was stirred 10 min at room temperature to form the corresponding zinc alkoxide catalyst. *rac*-CHC (0.066 g, 0.467 mmol, prepared as described in Example 3), L-lactide (0.202 g, 1.401 mmol, prepared as described in Example 4) and dry toluene (0.37 mL) were then added under argon. The flask and its content were immersed in an oil-bath at 100°C. After stirring over 6 h, the reaction mixture was cooled down to room temperature. Three drops of a 1.6 M acetic acid solution in toluene were added to quench the reaction. The solvent was evaporated and after determination of the monomers' conversions by $^1H$ NMR analysis of the crude product, the resulting mixture was dissolved in $CH_2Cl_2$ (10 mL) and precipitated in cold methanol (50 mL). This process was repeated twice to ensure complete removal of the catalyst and the unreacted monomers. A white copolymer was finally obtained following drying under vacuum.

Table 5

| Entry | Catalyst | [*rac*-CHC]$_0$: [LLA]$_0$: [Catal.]$_0$: [BnOH]$_0$ | Time (H) | CHC Conv.[a] (%) | L-LA Conv.[a] (%) | $Mn_{theo}$[b] (g/mol) | $Mn_{NMR}$[c] (g/mol) | $Mn_{SEC}$[d] (g/mol) | $Đ_M$ |
|---|---|---|---|---|---|---|---|---|---|
| 1 | [(NNO) ZnEt] | 50:150:1:1 | 6 | 80 | 95 | 26200 | nd | 29300 | 1.6 |
| 2 | [(NNO) ZnEt] | 100:100:1:1 | 65 | 78 | 100 | 25 500 | nd | 15 800 | 1.4 |
| 3 | [(NNO) ZnEt] | 150:50:1:1 | 6 | 23 | 80 | 10700 | nd | 11 300 | 1.3 |
| 4 | [(NNO) ZnEt] | 50:150:1:1 | 6 | 91 | 100 | 28200 | 26100 | 30 100 | 1.5 |

(continued)

| Entry | Catalyst | [*rac*-CHC]$_0$: [LLA]$_0$: [Catal.]$_0$: [BnOH]$_0$ | Time (H) | CHC Conv.[a] (%) | L-LA Conv.[a] (%) | $Mn_{theo}$[b] (g/mol) | $Mn_{NMR}$[c] (g/mol) | $Mn_{SEC}$[d] (g/mol) | $Đ_M$ |
|---|---|---|---|---|---|---|---|---|---|
| 5 | [(NNO) ZnEt] | 100:100:1:1 | 24 | 93 | 100 | 27700 | 24300 | 20 300 | 1.5 |
| 6 | [(NNO) ZnEt] | 150:50:1:1 | 6 | 86 | 100 | 25600 | 23100 | 19 200 | 1.4 |
| 7 | [(NNO) ZnEt] | 50:150:1:1 | 6 | 86 | 100 | 27800 | 22700 | 25 900 | 1.5 |
| 8 | [(NNO) ZnEt] | 100:100:1:1 | 24 | 82 | 100 | 26200 | 18300 | 13 300 | 1.5 |
| 9 | [(NNO) ZnEt] | 150:50:1:1 | 6 | 77 | 100 | 23700 | 19900 | 14 100 | 1.4 |
| 10 | TBD | 50:150:1:1 | 6 | 77 | 100 | 27200 | 26400 | 13 800 | 1.4 |
| 11 | TBD | 100:100:1:1 | 24 | 42 | 100 | 20500 | 23500 | 13 800 | 1.3 |
| 12 | TBD | 150:50:1:1 | 6 | 26 | 100 | 12800 | 11800 | 6400 | 1.3 |

Calculated from the H NMR of the crude product. [b]Theoretical molar mass value of PCHC-co-PLLA calculated from the relation: $M_{n,theo} = M_{rac\text{-}CHC} \times [rac\text{-}CHC]_0/[BnOH]_0 \times \text{conversion}_{rac\text{-}CHC} + M_{LLA} \times [LLA]_0/[BnOH]_0 \text{x conversion}_{LLA} + M_{BnOH}$ with $M_{rac\text{-}CHC}$ = 142 g.mol$^{-1}$, $M_{LLA}$ = 144 g.mol$^{-1}$, $M_{BnoH}$ = 108 g.mol$^{-1}$. [c]NMR molar mass value of the copolymer calculated from the integral value ratio of the signals of alkoxide methylene or methine end-group hydrogens to internal methine hydrogens. [d]Experimental number average molar mass and dispersity values determined by SEC in THF at 30 °C using polystyrene standards. The concentrations of CHC and LLA in all experiments are 4 M except for entries 7-9 which are 2 M.

Similarly as in Example 3, using [(NNO)ZnEt] catalyst, yields very high CHC incorporation (up to 93%) into PCHC/PLLA copolymer.

Figures 9 and 10 show the $^1$H NMR (Figure 9) and $^{13}$C{$^1$H} NMR (Figure 10) spectra of the copolymer from entry 8 of Table 5 (wherein x stands for unreacted CHC, and * stands for residual solvent resonances).

**Example 8: Preparation of PCHC-PLLA diblock copolymers by sequential copolymerization of *rac*-CHC and L-lactic acid**

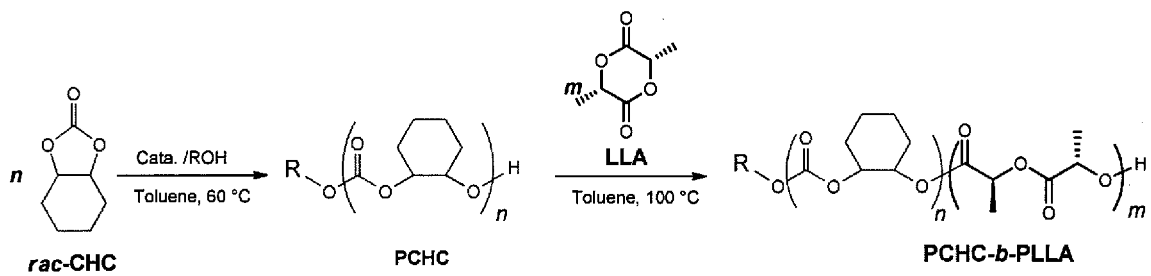


***rac*-CHC** **PCHC** **PCHC-*b*-PLLA**

Scheme 11

The process was performed by sequential copolymerization (addition of CHC prior to LLA), as depicted in Scheme 11. Different copolymerizations were performed with various catalysts, alcohols, monomer concentrations and reaction time during the copolymerization of the *rac*-CHC. The conditions and results are listed in Table 6. The copolymerizations were performed using the following catalytic systems: [(NNO)ZnEt], TBD, or $Y[N(SiMe_3)_2]_3$.

Typical procedure for CHC/L-LA sequential copolymerization (addition of CHC prior to LLA). All copolymerizations were performed similarly to the following typical procedure (Table 6, Entry 2).

The only differences lie in the nature of the catalyst, of the alcohol, in the initial concentrations in monomers,

and in the reaction time during the homopolymerization of the *racemic* cyclohexene carbonate (rac-CHC). In a dry-box, benzyl alcohol (0.97 μL, 9.34 μmol), [(NNO)ZnEt] (4.0 mg, 9.34 μmol) and dry toluene (0.10 mL) were placed in a Schlenk flask equipped with a magnetic stir-bar. The mixture was stirred 10 min at room temperature to form the corresponding alkoxide catalyst. rac-CHC (0.133 g, 0.934 mmol, prepared as described in Example 3) and dry toluene (0.23 mL) were then added under argon. The flask and its contents were immersed in an oil-bath at 60°C. After stirring over 6 h, L-lactide (LLA, 0.135 g, 0.934 mmol, prepared as described in Example 4) and dry toluene (0.24 mL) were added under argon; the reaction mixture was stirred over 2 h at 100°C, and was cooled down to room temperature. Three drops of a 1.6 M acetic acid solution in toluene were added to quench the reaction. The solvent was evaporated and the crude polymer was dissolved in $CH_2Cl_2$ (10 mL) and precipitated in cold methanol (50 mL). This process was repeated twice to ensure complete removal of the catalyst and the unreacted monomers. A white polymer was finally obtained following drying under vacuum.

Table 6

| | | | | | | PCHC | | PCHC-*b*-PLLA | | | |
|---|---|---|---|---|---|---|---|---|---|---|---|
| Entry | Catalyst | ROH | [*rac*-CHC]$_0$:[L-LA]$_0$:[Catalyst]$_0$:[ROH]$_0$ | CHC Conv.[a] (%) | LLA Conv.[a] (%) | $Mn_{theo}$[b] (g/mol) | $Mn_{NMR}$ (g/mol) | $Mn_{theo}$[c] (g/mol) | $Mn_{NMR}$[d] (g/mol) | $Mn_{SEC}$[e] (g/mol) | $Đ_M$ |
| 1 | [(NNO)ZnEt] | BnOH | 50:150:1:1 | 85 | 98 | 6200 | 4200 | 27300 | 31600 | 28000 | 1.7 |
| 2 | [(NNO)ZnEt] | BnOH | 100:100:1:1 | 86 | 97 | 12300 | 11900 | 26300 | 28000 | 25300 | 1.5 |
| 2' | [(NNO)ZnEt] | BnOH | 100:100:1:1 | 86 | 95 | 12300 | nd | 26000 | 25000 | nd | nd |
| 3 | [(NNO)ZnEt] | BnOH | 150:50:1:1 | 77 | 91 | 16500 | 19300 | 23100 | 23400 | 1 100 | 1.2 |
| 4 | TBD[e] | BnOH | 50:150:1:1 | 91 | 96 | 6600 | 6900 | 27300 | nd | 2 600 | 1.5 |
| 5 | TBD[e] | BnOH | 100:100:1:1 | 91 | 94 | 13000 | 16500 | 26600 | 29000 | 17000 | 1.4 |
| 6 | TBD[e] | BnOH | 150:50:1:1 | 91 | 100 | 19500 | 17300 | 26700 | 31700 | 17100 | 1.4 |
| 7 | $Y[N(SiMe_3)_2]_3$[f] | *i*PrOH | 50:150:1:1 | 87 | 97 | 6200 | *nd* | 27200 | nd | 28600 | 1.7 |
| 8 | $Y[N(SiMe_3)_2]_3$[f] | *i*PrOH | 100:100:1:1 | 77 | 97 | 11000 | *nd* | 25000 | 24900 | 22900 | 1.6 |
| 9 | $Y[N(SiMe_3)_2]_3$[f] | *i*PrOH | 150:50:1:1 | 71 | 100 | 15200 | *nd* | 22400 | 23600 | 20300 | 1.6 |

[a]Calculated from the $^1H$ NMR of the crude product. [b]Theoretical molar mass value of PCHC calculated from the relation: $M_{n,theo} = M_{rac\text{-}CHC} \times [rac\text{-}CHC]_0/[ROH]_0 \times conversion_{rac\text{-}CHC} + M_{ROH}$ with $M_{rac\text{-}CHC}$ = 142 g.mol$^{-1}$, $M_{BnOH}$ = 108 g.mol$^{-1}$, $M_{iPrOH}$ = 60 g.mol$^{-1}$. [c]Theoretical $M_n$ value of PCHC-*b*-PLLA calculated from the relation: $M_{n,theo} = \{M_{rac\text{-}CHC} \times [rac\text{-}CHC]_0/[ROH]_0 \times conversion_{rac\text{-}CHC}\} + \{M_{LLA} \times [LLA]_0/[ROH]_0 \times conversion_{LLA}\} + M_{ROH}$. [d] NMR molar mass value of PCHC/PLLA copolymer calculated from the integral value ratio of the signals of alkoxide methylene or methine end-group hydrogens to internal methine hydrogens. [e]Experimental number average molar mass and dispersity values determined by SEC in THF at 30°C using polystyrene standards. [e,f]The homopolymerization reaction time of *rac*-CHC is 24 h and 16 h, respectively.

The $^1H$ NMR spectrum (Figure 11) of the PCHC-*b*-PLLA copolymer prepared according to Table 6, entry 2 showed the signals of the PCHC (CH at δ 4.61 ppm) and the PLLA (CH at δ 5.15 ppm) main chain CH in a 0.68:1.00: = 0.68 ratio, which is quite different from the CHC and LLA conversions ratio (0.86:0.97 = 0.89). This is likely due to the aliquot of PCHC taken out for analysis to determine the CHC conversion prior to the addition LLA. The $^{13}C\{^1H\}$ NMR spectrum of the same copolymer (Figure 12) allowed comparing the ratio between the PCHC and PLLA carbons. This ratio was dependent on the nature of the carbon atoms considered. Table 7 shows the integral ratio between PCHC and PLLA carbons groups.

Table 7

| Carbon groups | | Integral ratio (PCHC/PLLA)* |
|---|---|---|
| PCHC (δ ppm) | PLLA (δ ppm) | |
| C=O (152.1-152.8) | C=O (168.6) | 0.67 |
| CH2 (21.1-28.7) | CH (68.0) | 0.58 |
| CH2 (21.1-28.7) | CH3 (15.6) | 0.51 |
| *Calculated from the integrals of the $^{13}C\{^1H\}$ NMR resonances in the PCHC-*b*-PLLA polymer and the relation: (integral of PCH carbons/number of carbons)/(integral of PLLA carbons/number of carbons). | | |

The same experiment (conditions of Table 6, entry 2) was repeated without any aliquot being taken out for analysis during the copolymerization (Table 6, Entry 2'). The $^1H$ NMR of the crude product (Figure 13) exhibits a *rac*-CHC and LLA conversion of 86 and 95 %, respectively. After precipitation of the copolymer, the $^1H$ NMR of the recovered sample showed (Figure 14) that the integration of the signals corresponding to PCHC (CH at δ 4.61 ppm) and PLLA (C*H* at δ 5.06 ppm) are in a 0.95:1= 0.95 ratio, which is in agreement with the ratio of the conversions (0.86:0.95 = 0.90).

An experiment under the same operating conditions, yet with the reverse order of comonomers' addition (L-LA followed by *rac*-CHC) using [(NNO)ZnEt] catalyst was also performed. The $^1H$ and $^{13}C\{^1H\}$ NMR (Figures 15 and 16) showed spectra similar to those of a random copolymer (as in Figures 9 and 10). This is most likely due to the trans-esterification of PLLA and /or PCHC.

**Example 9: Preparation of PCHC-PLLA diblock copolymers from sequential copolymerization of *(R,R)*-CHC and L-lactic acid**

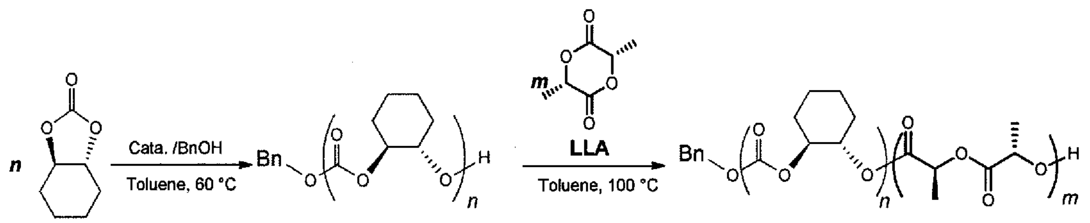


***(R,R)*-CHC** **PCHC** **PCHC-*b*-PLLA**

Scheme 12

The process was performed by sequential copolymerization, wherein the (*R,R*)-cyclohexene carbonate (*(R,R)*-CHC) was added prior to addition of L-lactic acid (LLA). Different copolymerizations were performed using [(NNO)ZnEt] and benzyl alcohol (BnOH), as depicted in Scheme 12. The conditions and results are listed in Table 8. Typical procedure was as described in Example 8.

Table 8

| | | | | | | **PCHC-*b*-PLLA** | | | |
|---|---|---|---|---|---|---|---|---|---|
| **Entry** | **[(*R,R*)-CHC]$_0$: [LLA]$_0$: [Catal.]$_0$: [BnOH]$_0$** | ***(R, R)*-CHC[a] Conv.** (%) | **LLA[a] Conv.** (%) | **PCHC $M_{n,theo}$[b]** (g/mol) | **PCHC $M_{n,NMR}$** (g/mol) | ***M*n, theo[c]** (g/mol) | **$M_{n,NMR}$[d]** (g/mol) | ***M*n, SEC[e]** (g/mol) | **$Đ_M$** |
| 1 | 50:150:1:1 | 97 | 100 | 7000 | 8100 | 28 600 | 20 800 | | |
| 2 | 100:100:1:1 | 97 | 100 | 13 900 | 14 600 | 28 300 | 24 700 | | |

(continued)

| | | | | | | PCHC-*b*-PLLA | | | |
|---|---|---|---|---|---|---|---|---|---|
| **Entry** | **$[(R,R)\text{-CHC}]_0$: $[LLA]_0$: $[Catal.]_0$: $[BnOH]_0$** | ***(R, R)*-CHC[a] Conv.** (%) | **LLA[a] Conv.** (%) | **PCHC $M_{n,theo}$[b]** (g/mol) | **PCHC $M_{n,NMR}$** (g/mol) | ***M*n, theo[c]** (g/mol) | **$M_{n,NMR}$[d]** (g/mol) | ***M*n, SEC[e]** (g/mol) | **$Đ_M$** |
| 3 | 150:50:1:1 | 97 | 100 | 20 800 | 17 000 | 28 000 | 24 600 | | |

[a]Calculated from the $^1H$ NMR of the crude product. [b]Theoretical molar mass value of PCHC calculated from the relation: $M_{n.theo} = M_{(R,R)\text{-CHC}} \times [(R,R)\text{-CHC}]_0/[ROH]_0 \times \text{conversion}_{(R,R)\text{-CHC}} + M_{ROH}$ with $M_{(R,R)\text{-CHC}}$ = 142 g.mol$^{-1}$, $M_{BnoH}$ = 108 g.mol$^{-1}$. [c]theoretical $M_n$ value of PCHC-b-PLLA calculated from the relation: $M_{n,theo} = M_{(R,R)\text{-CHC}} \times [(R,R)\text{-CHC}]_0/[ROH]_0 \times \text{conversion}_{(R,R)\text{-CHC}} + M_{LLA} \times [LLA]_{0/[ROH]0} \times \text{conversion}_{LLA} + M_{BnOH}$. [d]NMR molar mass value of PCHC/copolymer calculated from the integral value ratio of the signals of alkoxide methylene or methine end-group hydrogens to internal methine hydrogens. Experimental *number average molar mass* and dispersity values determined by SEC in THF at 30 °C using polystyrene standards.

The PCHC-*b*-PLLA copolymer (Table 8, entry 8) was characterized by its $^1H$ NMR (Figure 17) and $^{13}C\{^1H\}$ NMR (Figure 18) spectra.

**Example 10: Preparation of PCHC-PTMC copolymers**

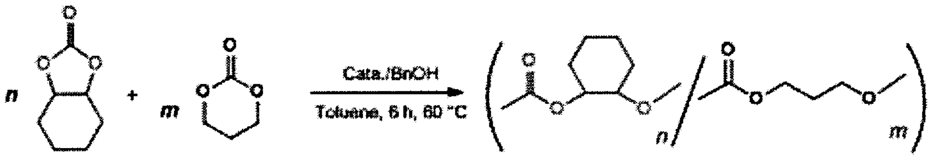


*rac*-CHC TMC PCHC-co-PTMC

Scheme 13

The copolymerization of racemic cyclohexene carbonate (*rac*-CHC) and trimethylene carbonate (TMC) was performed as depicted in Scheme 13. All polymerizations were performed similarly according to the following typical procedure (Table 9, entry 2). The only differences were in the nature of the catalyst and the initial concentrations of the monomers. The conditions and results are listed in Table 9. The copolymerizations were performed using the following catalytic systems:[(NNO)ZnEt] or TBD. The TMC (1,3-dioxane-2-one from Labso Chimie Fine, Blanquefort, France) was purified by first dissolving it in THF, stirring over $CaH_2$ for 2 days before being filtrated and dried *in vacuo,* and finally recrystallized from cold THF.

Typical procedure of CHC/TMC simultaneous copolymerization was as follows. In a dry-box, benzyl alcohol (0.97 µL, 9.34 µmol), [(NNO)ZnEt] (4.0 mg, 9.34 µmol) and dry toluene (0.10 mL) were placed in a Schlenk flask equipped with a magnetic stir-bar. The mixture was stirred 10 min at room temperature to form the corresponding alkoxide catalyst. *rac*-CHC (0.133 g, 0.934 mmol, prepared as in Example 3), TMC (0.095 g, 0.934 mmol, and dry toluene (0.37 mL) were then added. The flask and its contents were placed under argon and immersed in an oil-bath at 60 C. After stirring over 6 h, the reaction mixture was cooled down to room temperature and three drops of 1.6 M acetic acid solution in toluene were added to quench the reaction. The solvent was evaporated and the crude polymer was dissolved in $CH_2Cl_2$ (10 mL) and precipitated in cold methanol (50 mL). This process was repeated twice to ensure complete removal of the catalyst and the unreacted monomers. A white polymer was finally obtained following drying under vacuum.

Table 9

| **Entry** | **Catalyst** | **$[rac\text{-CHC}]_0$:$[TMC]_0$: $[Cat.]_0$:$[BnOH]_0$** | **CHC Conv.[a]** (%) | **TMC Conv.[a]** (%) | **$M_{nth}$[b]** (g/mol) | **$M_{nNMR}$[c]** (g/mol) | **$M_{nSEC}$[d]** (g/mol) | **$Đ_M$** |
|---|---|---|---|---|---|---|---|---|
| 1 | [(NNO) ZnEt] | 50:150:1:1 | 80 | 98 | 20 800 | 20 200 | 17 500 | 1.5 |

(continued)

| Entry | Catalyst | [*rac*-CHC]$_0$:[TMC]$_0$:[Cat.]$_0$:[BnOH]$_0$ | CHC Conv.[a] (%) | TMC Conv.[a] (%) | $M_{nth}$[b] (g/mol) | $M_{nNMR}$[c] (g/mol) | $M_{nSEC}$[d] (g/mol) | $Đ_M$ |
|---|---|---|---|---|---|---|---|---|
| 2 | [(NNO) ZnEt] | 100:100:1:1 | 80 | 98 | 20 800 | 18 900 | 14100 | 1.7 |
| 3 | [(NNO) ZnEt] | 150:50:1:1 | 70 | 97 | 20 000 | 15 700 | 13 600 | 1.5 |
| 4 | TBD | 50:150:1:1 | 93 | 100 | 22 000 | 26 800 | 15 900 | 1.5 |
| 5 | TBD | 100:100:1:1 | 91 | 100 | 23 200 | 29 300 | 17 500 | 1.4 |
| 6 | TBD | 150:50:1:1 | 85 | 100 | 23 300 | 11 500 | 15 300 | 1.4 |

[a]Calculated from the $^1H$ NMR of the crude product. [b]Theoretical $M_n$ value of PCHC-co-PTMC calculated from the relation: $M_{n,theo} = M_{rac\text{-}CHC} \times [rac\text{-}CHC]_0/[ROH]_0 \times conversion_{rac\text{-}CHC} + M_{TMC} \times [TMC]_0/[BnOH]_0 \times conversion_{TMC} + M_{BnOH}$. [c]NMR molar mass value of PCHC calculated from the integral value ratio of the signals of alkoxide methylene end-group hydrogens to internal methine hydrogens. [d]Experimental *number average molar mass* and dispersity values determined by SEC in THF at 30 °C using polystyrene standards.

The $^1H$ NMR of the polymer produced in Entry 2 of Table 9 (Figure 19), shows that the ratio of the integral values of signals of the PCHC (*CH* at δ 4.63 ppm) and the TMC ($CH_2$-O at δ 4.22 ppm) main chain methine and methylene, respectively, is equal to [(1/2)/(2.5/4)] = 0.80, which is similar to the CHC and TMC conversions ratio (0.80:0.98= 0.82; Table 5, entry 2). Analysis of the $^{13}C\{^1H\}$ NMR of the same polymer (Figure 20) (relaxation delay = 10 s) exhibits the methylene groups of PCHC and PTMC in a 1.35:2 = 0.68 ratio, which is different from the $^1H$ NMR (0.80) as $^{13}C\{^1H\}$ NMR is not a quantitative technique for these polymers.

**Example 11: Preparation of PCHC-PTMC copolymers by sequential copolymerization of rac-CHC and TMC**

A similar procedure as that described in Example 8 for the CHC/L-LA sequential copolymerization (PCHC-*b*-PLLA), was used for the preparation of PCHC-PTMC copolymers by sequential copolymerization of racemic cyclohexene carbonate (*rac*-CHC) and trimethylene carbonate (TMC). The process was performed by sequential copolymerization (addition of TMC prior to rac-CHC) at 100°C in toluene. TMC was fully converted before addition of CHC. The copolymerizations were performed using the following catalytic system: [(NNO)ZnEt]. The conditions and results are listed in Table 10.

Table 10

| | | | | | | PTMC-*b*-PCHC | | | |
|---|---|---|---|---|---|---|---|---|---|
| Entry | Catalyst | [TMC]$_0$:[*rac*-CHC]$_0$:[Catal.]$_0$:[BnOH]$_0$ | TMC Conv.[a] (%) | CHC Conv.[a] (%) | PTMC $Mn_{th}$[b] (g/mol) | $Mn_{th}$[c] (g/mol) | $Mn_{NMR}$[d] (g/mol) | $Mn_{SEC}$[e] (g/mol) | $Đ_M$ |
| 1 | [(NNO) ZnEt] | 50:150:1:1 | 100 | 62 | 5200 | 18 400 | 19 400 | 16 500 | 1.4 |
| 2 | [(NNO) ZnEt] | 100:100:1:1 | 100 | 83 | 10 300 | 21 800 | 26 800 | 20 300 | 1.6 |

[a]Calculated from the $^1H$ NMR of the crude product. [b]Theoretical $M_n$ value of PTMC calculated from the relation: $M_{n,theo} = M_{TMC} \times [TMC]_0/[BnOH]_0 \times conversion_{TMC} + M_{BnoH}$ with $M_{TMC}$ = 102 g.mol$^{-1}$, $M_{BnOH}$ = 108 g.mol$^{-1}$. [c]Theoretical $M_n$ value of PTMC-*co*-PCHC calculated from the relation: $M_{n,theo} = M_{rac\text{-}CHC} \times [rac\text{-}CHC]_0/[BnOH]_0 \times conversion_{rac\text{-}CHC} + M_{TMC} \times [TMC]_0/[BnOH]_0 \times conversion_{TMC} + M_{BnOH}$. [d]NMR molar mass value of PTMC-*co*-PCHC copolymer calculated from the integral value ratio of the signals of benzylalkoxide methylene end-group hydrogens to internal methine hydrogens. [e]Experimental *number average molar mass* and dispersity values determined by SEC in THF at 30 °C using polystyrene standards.

The $^{13}C$ NMR spectrum of the copolymer entry 2 of Table 10, (Figure 21), showed the same spectrum as the PCHC-*co*-PTMC random copolymer (Figure 20). This most likely results from extensive trans-esterification of the PTMC and/or PCHC blocks under the reaction conditions.

**Example 12: Preparation of PCHC-PTMC diblock copolymers by sequential copolymerization of *rac*-CHC and TMC**

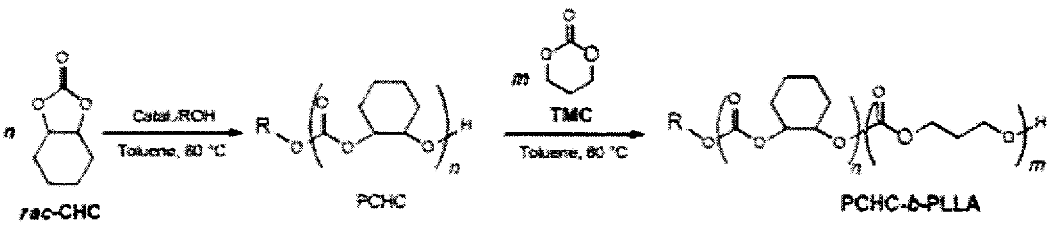


Scheme 15

A similar procedure as that described in Example 8 for the CHC/L-LA sequential copolymerization (PCHC-*b*-PLLA), was used for the preparation of PCHC-PTMC diblock copolymers by sequential copolymerization of racemic cyclohexene carbonate (*rac*-CHC) and trimethylene carbonate (TMC). The process was performed by sequential copolymerization (addition of rac-CHC prior to TMC) at 60°C in toluene as depicted in scheme 15, using various catalysts and different initial concentrations of TMC and *rac*-CHC. The conditions and results are listed in Table 11. The copolymerizations were performed using the following catalytic systems: [(NNO)ZnEt] and TBD.

Table 11

| Entry | Catalyst | [*rac*-CHC]$_0$: [TMC]$_0$ [Catal.]$_0$:[BnOH]$_0$ | CHC Conv.[a] (%) | TMC Conv.[a] (%) | $Mn_{th}$[b] (g/mol) | $Mn_{NMR}$[c] (g/mol) | $Mn_{SEC}$[d] (g/mol) | $Đ_M$ |
|---|---|---|---|---|---|---|---|---|
| 1d | [(NNO) ZnEt] | 50:150:1:1 | 96 | 100 | 22 200 | 16 800 | | |
| 2d | [(NNO) ZnEt] | 100:100:1:1 | 96 | 100 | 24 000 | 18 600 | | |
| 3[d] | [(NNO) ZnEt] | 150:50:1:1 | 96 | 100 | 25 700 | 22 600 | | |
| 4[e] | TBD | 50:150:1:1 | 93 | 100 | 22 000 | 16 500 | | |
| 5[e] | TBD | 100:100:1:1 | 92 | 100 | 23 400 | 21 500 | | |
| 6[e] | TBD | 150:50:1:1 | 91 | 100 | 24 600 | 22 400 | | |

[a] Calculated from the $^1H$ NMR of the crude product. [b]Theoretical $M_n$ value of PCHC-*b*-PTMC calculated from the relation: $M_{n,theo} = M_{rac\text{-}CHC} \times [rac\text{-}CHC]_0/[BnOH]_0 \times conversion_{rac\text{-}CHC} + M_{TMC} \times [TMC]_0/[BnOH]_0 \times conversion_{TMC} + M_{BnOH}$. [c]NMR molar mass value of the copolymer calculated from the integral value ratio of the signals of benzylalkoxide methylene end-group hydrogens to internal methine hydrogens. [d]Experimental *number average molar mass* and dispersity values determined by SEC in THF at 30 °C using polystyrene standards. [d,e]The homopolymerization reaction time of *rac*-CHC is 6 h and 24 h, respectively.

Figures 22 and 23 show respectively the $^1H$ NMR spectrum and 14C NMR spectrum of the copolymer entry 2 of Table 11.

## Claims

1. A process for preparing a polycarbonate or copolymer thereof, said process comprising homo- or copolymerizing in the presence of at least one catalyst, one or more compounds of formula (I), stereoisomers, racemics, or mixtures

thereof; optionally with one or more cyclic esters, stereoisomers, racemics, or mixtures thereof;

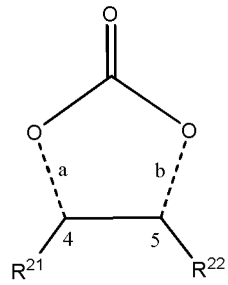 (I)

wherein

each dotted bond "**a**" and "**b**" independently represents a solid bond, a wedged bond, or a hashed wedged bond; and
**$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

**2.** The process according to claim 1, wherein the catalyst is an organometallic catalyst of formula (II) or (III),

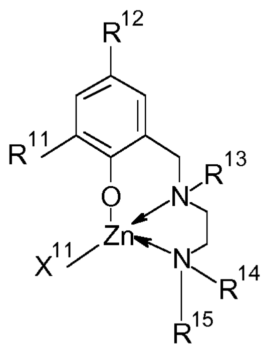

(II)

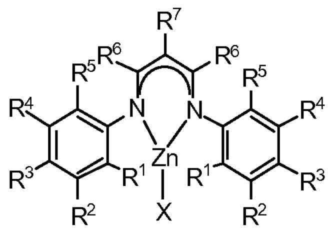

(III)

wherein

**$R^1$, $R^2$, $R^3$, $R^4$, $R^5$, $R^6$,** and **$R^7$** are each independently selected from the group consisting of hydrogen, optionally substituted $C_{1-12}$alkyl, and an inert functional group, and wherein two or more of said groups can be linked together to form one or more rings;
**X** is $-N(SiR^{27}{}_3)_2$, $C_{1-12}$alkyl, $C_{1-12}$alkoxy, $-NR^9R^{10}$ or-$BH_4$;
each **$R^{27}$** is independently selected from hydrogen and $C_{1-6}$alkyl;
each **$R^9$** and **$R^{10}$** is independently selected from hydrogen and $C_{1-6}$alkyl;
**$R^{11}$** and **$R^{12}$** are each independently $C_{1-10}$alkyl;
**$R^{13}$, $R^{14}$,** and **$R^{15}$** are each independently $C_1$-$_{10}$alkyl, or
**$R^{13}$** and **$R^{14}$** are covalently bound to each other and are each a methylene and **$R^{15}$** is $C_{1-10}$alkyl;
**$X^{11}$** is selected from $C_{1-10}$alkyl, $-OR^{16}$, and $-N(SiR^{17}{}_3)_2$;
**$R^{16}$** is $C_{1-10}$alkyl; and
each **$R^{17}$** is independently selected from hydrogen and $C_{1-6}$alkyl; preferably the catalyst is selected from [(NNO)ZnEt], [BDI]Zn($N(SiMe_3)_2$), [BDI]Zn(Et), and {[BDI]Zn($OR^{30}$)}$_2$, wherein **$R^{30}$** is $C_{1-6}$alkyl; preferably the catalyst is [(NNO)ZnEt].

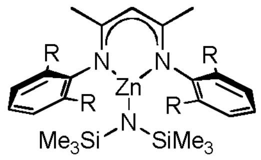

R = $^{i}$Pr

**(BDI)Zn(NTMS$_2$)**

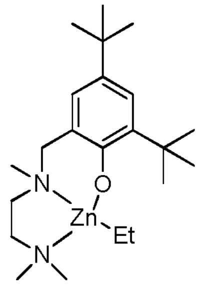

**(NNO)ZnEt** .

3. The process according to claim 1, wherein the catalyst is selected from complexes of formulae $M(OSO_2CF_3)_m$, $M(N(OSO_2CF_3)_2)_m$, $M(R^{23}C(O)CR^{23}{}_2C(O)R^{23})_m$, and $(R^{24}CO_2)_mM$, wherein **M** is a metal of Group 2, 3, including the lanthanide series, or Group 12, 13, 15, wherein each $\mathbf{R^{23}}$ is independently an optionally substituted $C_{1-12}$alkyl, wherein each $\mathbf{R^{24}}$ is independently a perfluorinated $C_{1-12}$alkyl or an aryl, and wherein m is the valence of M.

4. The process according to claim 1, wherein the catalyst is selected from the group comprising dimeric phosphazene bases, or amines or guanidines, organic acids, sparteine, thiourea-amino derivaties, N-heterocyclic carbenes, 4-(di-alkylamino)pyridines, amidines, phosphorus based phospines, phosphazenium derivatives and phosphazenes, metal amides; and compounds of general formula $M^1(Y^1,Y^2, ...Y^p)_q$, in which $\mathbf{M^1}$ is a metal selected from the group comprising the elements of columns 3 to 12 of the periodic table of the elements, and the elements Al, Ga, In, Tl, Ge, Sn, Pb, Sb, Ca, Mg and Bi; whereas $\mathbf{Y^1}$**,** $\mathbf{Y^2}$**, ...** $\mathbf{Y^p}$ are each substituents selected from the group comprising alkyl with 1 to 20 carbon atoms, aryl having from 6 to 30 carbon atoms, alkoxy having from 1 to 20 carbon atoms, aryloxy having from 6 to 30 carbon atoms, oxide, carboxylate, and halide groups, and **p** and **q** are integers of from 1 to 6; preferably the catalyst is selected from the group comprising 1,5,7-triazobicyclo-[4,4,0]dec-5-ene (TBD), 1,8-diazabicyclo[5.4.0]undec-7-ene (DBU), 4-dimethylaminopyridine (DMAP), and *tert*-butylimino-1,3-dimethylperhydro-1,3,2-diazaphosphine (BEMP); $Sn(Oct)_2$, $Al(OiPr)_3$, $Ti(OiPr)_4$, Ti(2-ethylhexanoate)$_4$, Ti(2-ethylhexyloxide)$_4$, $Zr(OiPr)_4$, Bi(neodecanoate)$_3$, (2,4-di-tert-butyl-6-(((2-(dimethylamino)ethyl)(methyl)amino)methyl)phenoxy)(ethoxy)zinc, Zn(lactate)$_2$; $Y[N(SiMe_3)_2]_3$; [Y] + L1; and [Y] + L2, wherein **L1** is a tetradentate ligand of formula L1 and **L2** is a tetradentate ligand of formula L2

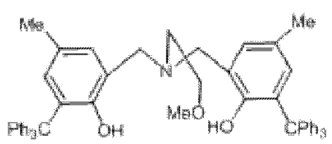

L1

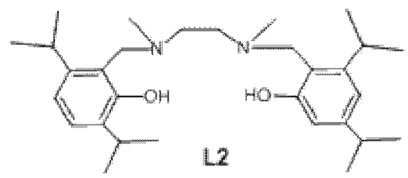

L2 .

5. The process according to any one of claims 1 to 4, wherein the process is performed in the presence of a compound of formula (IV),

$R^8$-OH (IV)

wherein $\mathbf{R^8}$ is selected from the group consisting of $C_{1-20}$alkyl, $C_{6-30}$aryl, and $C_{6-30}$aryl$C_{1-20}$alkyl optionally substituted by one or more substituents selected from the group consisting of halogen, hydroxyl, and $C_{1-6}$alkyl.

6. The process according to any one of claims 1 to 5, wherein the cyclic ester is selected from the group comprising lactones, cyclic carbonates, glycolides and lactides, wherein each group can be unsubstituted or substituted with one or more substituents each independently selected from $C_{1-6}$alkyl and $C_{1-6}$alkoxy, preferably lactones, glycolides and lactides.

7. The process according to any one of claims 1 to 6, wherein the cyclic ester is selected from the group comprising lactide, trimethylene carbonate, glycolide, β-butyrolactone, δ-valerolactone, γ-butyrolactone, γ-valerolactone, 4-methyldihydro-2(3H)-furanone, alpha-methyl-gamma-butyrolactone, and ε-caprolactone, 1,3-dioxolan-2-one, propylene carbonate, 4-methyl-1,3-dioxan-2-one, 1,3-doxepan-2-one, 5-$C_{1-4}$alkoxy-1,3-dioxan-2-one; and mixture

thereof, wherein each group can be unsubstituted or substituted with one or more substituents each independently selected from $C_{1-6}$alkyl and $C_{1-6}$alkoxy, preferably wherein said cyclic ester is selected from the group comprising lactide, glycolide, β-butyrolactone, δ-valerolactone, γ-butyrolactone, γ-valerolactone, 4-methyldihydro-2(3H)-furanone, alpha-methyl-gamma-butyrolactone, and ε-caprolactone, and mixture thereof.

**8.** The process according to any one of claims 1 to 7, wherein the process is performed in the presence or absence (in bulk) of solvent.

**9.** The process according to any one of claims 1 to 8, wherein the polycarbonate or copolymer thereof comprises recurring units of formula (VII3) or of formula (VII4),

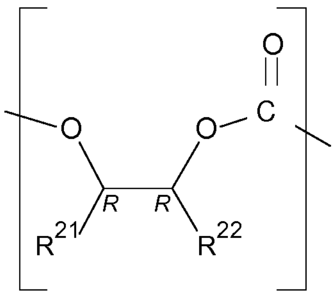
(VII3)
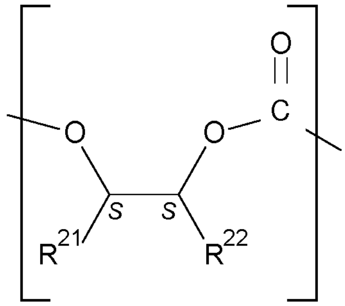
(VII4).

**10.** A process for preparing a polycarbonate copolymer according to any of the claims 1 to 9;

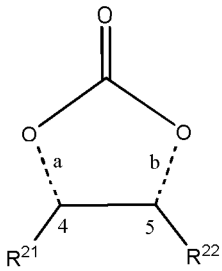
(I)

wherein
**$R^{21}$** and **$R^{22}$** taken together with the carbon atoms 4 and 5 of the cyclic carbonate form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; wherein two of said one or more substituents can join to form a ring fused to said $C_{3-9}$cycloalkylene, $C_{5-9}$cycloalkenylene, or $C_{6-12}$arylene; wherein said ring formed by said two substituents is selected from the group comprising $C_{3-9}$cycloalkyl, $C_{5-9}$cycloalkenyl, $C_{6-12}$aryl, and heterocyclyl, wherein each group can be unsubstituted or substituted with one or more $R^{40}$, wherein each **$R^{40}$** is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

**11.** A process for the synthesis of a compound of formula (I) a stereoisomer or racemate thereof, comprising the step of contacting a compound of formula (V), a stereoisomer, or racemate thereof; with at least one reagent selected from the group consisting of an alkyl- or cycloalkyl-chloroformate of formula (VI);

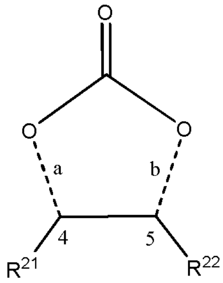 (I)

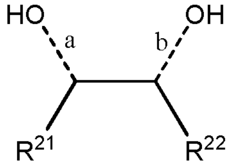 (V) 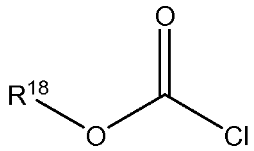 (VI),

wherein

each dotted bond "a" and "b" independently represents a solid bond, a wedged bond, or a hashed wedged bond; **$R^{21}$** and **$R^{22}$** taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and
**$R^{18}$** is $C_{1-6}$alkyl, or $C_{3-6}$cycloalkyl.

**12.** A polycarbonate or copolymer thereof comprising a recurring unit of formula (VII1) and/or (VI12),

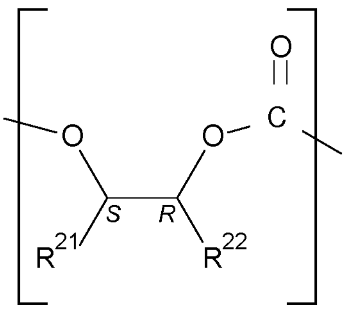 (VII1) 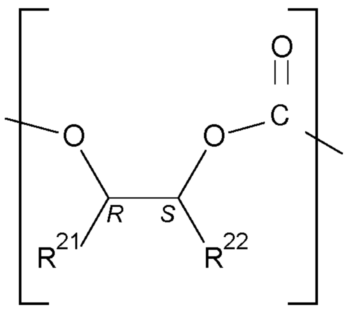 (VII2)

wherein
**$R^{21}$** and **$R^{22}$** taken together with the carbon atoms to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino.

**13.** The polycarbonate or copolymer thereof according to claim 12, comprising recurring units each independently represented by the following formula (VIIA) or (VIIB) or (VIIC);

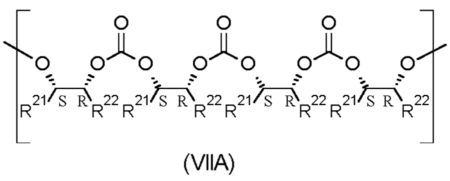

(VIIA)

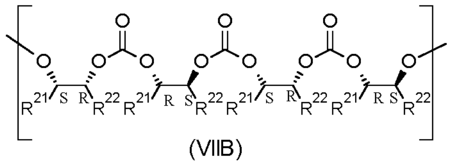

(VIIB)

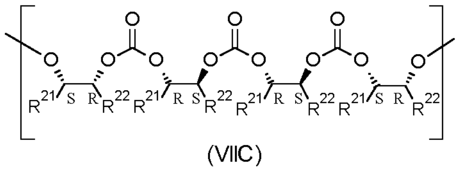

(VIIC)

wherein **$R^{21}$** and **$R^{22}$** have the same meaning as that defined in claim 12.

**14.** The polycarbonate or copolymer thereof according to claim 12 or 13, comprising recurring units each independently represented by the following formula (VIIA1) or (VIIB1) or (VIIC1);

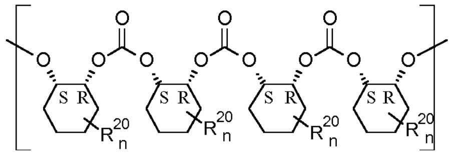

(VIIA1)

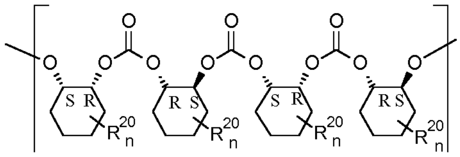

(VIIB1)

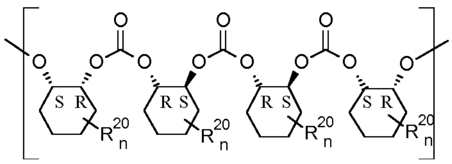

(VIIC1)

each **$R^{20}$** is independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino;
**n** is an integer selected from 0, 1, 2, 3, or 4.

**15.** A polycarbonate or copolymer thereof comprising recurring units of formula (VI13) or of formula (VI14),

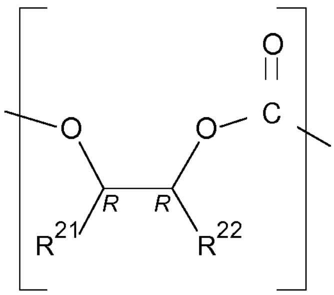
(VII3)
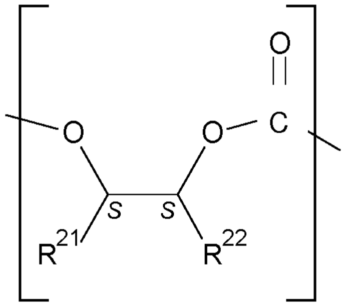
(VII4)

wherein
**$R^{21}$** and **$R^{22}$** taken together with the carbon atoms 4 and 5 to which they are attached form a ring selected from $C_{3-9}$cycloalkylene, $C_{5-9}$cycloalkenylene, and $C_{6-12}$arylene, said ring being optionally substituted with one or more substituents each independently selected from halo, hydroxyl, $C_{1-6}$alkyl, halo$C_{1-6}$alkyl, $C_{1-6}$alkoxy, halo$C_{1-6}$alkoxy, $C_{6-12}$aryl, $C_{1-6}$alkylthio, carboxyl, $C_{1-6}$alkoxycarbonyl, $C_{1-6}$alkylcarbonyloxy, amino; or mono- or di-$C_{1-6}$alkylamino; and
wherein said polycarbonate or copolymer thereof is **characterized by** a $^{13}C\{^1H\}$ NMR spectrum comprising only one signal present in the areas ranging from $\delta$ 150 to 155 ppm.

**16.** An article comprising a polycarbonate or copolymer thereof according to any one of claims 12 to 15 or a polycarbonate or copolymer thereof prepared according to a process according to any of claims 1 to 10.

## Patentansprüche

**1.** Verfahren zum Herstellen eines Polycarbonats oder Copolymers davon, wobei das Verfahren das Homo- oder Copolymerisieren einer oder mehrerer Verbindungen der Formel (I), von Stereoisomeren, Racematen oder Mischungen davon in Gegenwart zumindest eines Katalysators; optional mit einem oder mehreren cyclischen Estern, Stereoisomeren, Racematen oder Mischungen davon;

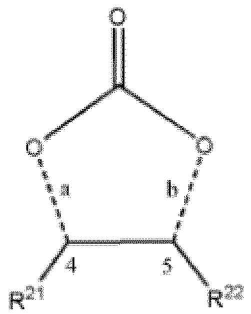
(I)

wobei:

jede gepunktete Bindung "**a**" und "**b**" unabhängig eine feste Bindung, eine verkeilte Verbindung oder eine zerlegte verkeilte Bindung darstellt; und
**$R^{21}$** und **$R^{22}$** mit den Kohlenstoffatomen 4 und 5 des cyclischen Carbonats zusammengenommen einen Ring bilden, der aus $C_{3-9}$-Cycloalkylen, $C_{5-9}$-Cycloalkenylen und $C_{6-12}$-Arylen ausgewählt ist, wobei der Ring optional mit einem oder mehreren Substituenten substituiert ist, die jeweils unabhängig aus Halo, Hydroxyl, $C_{1-6}$-Alkyl, Halo-$C_{1-6}$-alkyl, $C_{1-6}$-Alkoxy, Halo-$C_{1-6}$-alkoxy, $C_{6-12}$-Aryl, $C_{1-6}$-Alkylthio, Carboxyl, $C_{1-6}$-Alkoxycarbonyl, $C_{1-6}$-Alkylcarbonyloxy, Amino; oder Mono- oder Di-$C_{1-6}$-alkylamino ausgewählt sind.

**2.** Verfahren nach Anspruch 1, wobei der Katalysator ein organometallischer Katalysator der Formel (II) oder (III) ist,

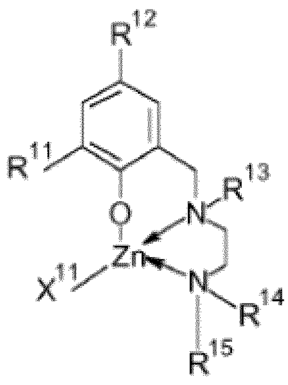

(II)

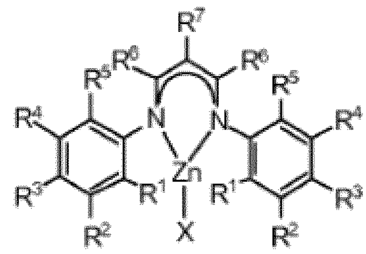

(III)

wobei:

**$R^1$, $R^2$, $R^3$, $R^4$, $R^5$, $R^6$** und **$R^7$** jeweils unabhängig aus der Gruppe ausgewählt sind, bestehend aus Wasserstoff, optional substituiertem $C_{1-12}$-Alkyl und einer inerten funktionellen Gruppe, und wobei zwei oder mehr der Gruppen miteinander verknüpft sein können, um einen oder mehrere Ringe zu bilden;
X $-N(SiR^{27}{}_3)_2$, $C_{1-12}$-Alkyl, $C_{1-12}$-Alkoxy, $-NR^9R^{10}$ oder $-BH_4$ ist;
**$R^{27}$** jeweils unabhängig aus Wasserstoff und $C_{1-6}$-Alkyl ausgewählt ist;
**$R^9$** und **$R^{10}$** jeweils unabhängig aus Wasserstoff und $C_{1-6}$-Alkyl ausgewählt sind;
**$R^{11}$** und **$R^{12}$** jeweils unabhängig $C_{1-10}$-Alkyl sind;
**$R^{13}$**, **$R^{14}$** und **$R^{15}$** jeweils unabhängig $C_{1-10}$-Alkyl sind, oder
**$R^{13}$** und **$R^{14}$** kovalent aneinander gebunden sind und jeweils ein Methylen sind und
**$R^{15}$** $C_{1-10}$-Alkyl ist;
**$X^{11}$** aus $C_{1-10}$-Alkyl, $-OR^{16}$ und $-N(SiR^{17}{}_3)_2$ ausgewählt ist;
**$R^{16}$** $C_{1-10}$-Alkyl ist; und
**$R^{17}$** jeweils unabhängig aus Wasserstoff und $C_{1-6}$-Alkyl ausgewählt ist; vorzugsweise wobei der Katalysator aus [(NNO)ZnEt], [BDI]Zn(N(SiMe$_3$)$_2$), [BDI]Zn(Et) und {[BDI]Zn(OR$^{30}$)}$_2$ ausgewählt ist, wobei **$R^{30}$** $C_{1-6}$-Alkyl ist; vorzugsweise wobei der Katalysator [(NNO)ZnEt] ist.

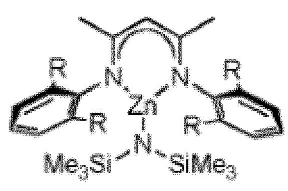

R = $^i$Pr

**(BDI)Zn(NTMS$_2$)**

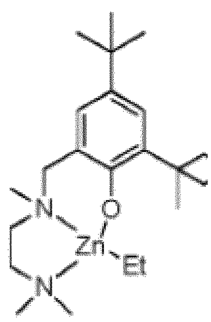

**(NNO)ZnEt**

**3.** Verfahren nach Anspruch 1, wobei der Katalysator aus Komplexen der Formeln $M(OSO_2CF_3)_m$, $M(N(OSO_2CF_3)_2)_m$, $M(R^{23}C(O)CR^{23}{}_2C(O)R^{23})_m$ und $(R^{24}CO_2)_mM$ ausgewählt ist, wobei **M** ein Metall der Gruppe 2, 3, umfassend die Lanthanoidreihe, oder der Gruppe 12, 13, 15 ist, wobei **$R^{23}$** jeweils unabhängig ein optional substituiertes $C_{1-12}$-Alkyl ist, wobei **$R^{24}$** jeweils unabhängig ein perfluoriertes $C_{1-12}$-Alkyl oder ein Aryl ist und wobei **m** die Valenz von M ist.

**4.** Verfahren nach Anspruch 1, wobei der Katalysator aus der Gruppe ausgewählt ist, umfassend dimere Phosphazenbasen oder Amine oder Guanidine, organische Säuren, Spartein, Thioharnstoffaminoderivate, N-heterocyclische Carbene, 4-(Dialkylamino)pyridine, Amidine, phosphorbasierte Phosphine, Phosphazeniumderivate und Phosphazene, Metallamide; und Verbindungen der allgemeinen Formel $M^1(Y^1, Y^2 \ldots Y^p)_q$, wobei **$M^1$** ein Metall ist, das aus

der Gruppe ausgewählt ist, umfassend die Elemente der Spalten 3 bis 12 des Periodensystems der Elemente und die Elemente Al, Ga, In, Tl, Ge, Sn, Pb, Sb, Ca, Mg und Bi; wobei $\mathbf{Y^1}$**,** $\mathbf{Y^2}$ ... $\mathbf{Y^p}$ jeweils Substituenten sind, die aus der Gruppe ausgewählt sind, umfassend Alkyl mit 1 bis 20 Kohlenstoffatomen, Aryl mit 6 bis 30 Kohlenstoffatomen, Alkoxy mit 1 bis 20 Kohlenstoffatomen, Aryloxy mit 6 bis 30 Kohlenstoffatomen, Oxid, Carboxylat und Halidgruppen, und **p** und **q** ganze Zahlen von 1 bis 6 sind; vorzugsweise wobei der Katalysator aus der Gruppe ausgewählt ist, umfassend 1,5,7-Triazobicyclo-[4,4,0]dec-5-en (TBD), 1,8-Diazabicyclo[5.4.0]undec-7-en (DBU), 4-Dimethylaminopyridin (DMAP) und *tert*-Butylimino-1,3-dimethylperhydro-1,3,2-diazaphosphin (BEMP); $Sn(Oct)_2$, $Al(OiPr)_3$, $Ti(OiPr)_4$, Ti(2-Ethylhexanoat)$_4$, Ti(2-Ethylhexyloxid)$_4$, $Zr(OiPr)_4$, Bi(neodecanoat)$_3$, (2,4-Di-tert-butyl-6-(((2-(dimethylamino)ethyl)(methyl)amino)methyl)phenoxy)(ethoxy)zink, Zn(lactat)$_2$; $Y[N(SiMe_3)_2]_3$; [Y] + L1; und [Y] + L2, wobei **L1** ein vierzähniger Ligand der Formel L1 ist und **L2** ein vierzähniger Ligand der Formel L2 ist,

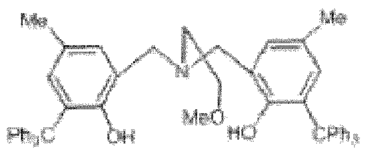
L1

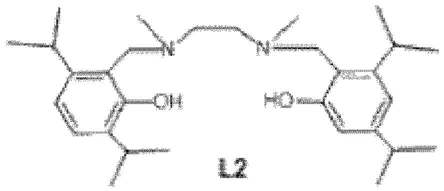
L2

5. Verfahren nach einem der Ansprüche 1 bis 4, wobei das Verfahren in Gegenwart einer Verbindung der Formel (IV) durchgeführt wird,

$R^8$-OH (IV)

wobei $\mathbf{R^8}$ aus der Gruppe ausgewählt ist, bestehend aus $C_{1-20}$-Alkyl, $C_{6-30}$-Aryl und $C_{6-30}$-Aryl-$C_{1-20}$-alkyl, optional mit einem oder mehreren Substituenten substituiert, die aus der Gruppe ausgewählt sind, bestehend aus Halogen, Hydroxyl und $C_{1-6}$-Alkyl.

6. Verfahren nach einem der Ansprüche 1 bis 5, wobei der cyclische Ester aus der Gruppe ausgewählt ist, umfassend Lactone, cyclische Carbonate, Glycolide und Laktide, wobei jede Gruppe unsubstituiert oder mit einem oder mehreren Substituenten substituiert sein kann, die jeweils unabhängig aus $C_{1-6}$-Alkyl und $C_{1-6}$-Alkoxy ausgewählt sind, vorzugsweise Lactonen, Glycoliden und Laktiden.

7. Verfahren nach einem der Ansprüche 1 bis 6, wobei der cyclische Ester aus der Gruppe ausgewählt ist, umfassend Lactid, Trimethylencarbonat, Glycolid, β-Butyrolacton, δ-Valerolacton, γ-Butyrolacton, γ-Valerolacton, 4-Methyldihydro-2(3H)-furanon, α-Methyl-γ-butyrolacton und ε-Caprolacton, 1,3-Dioxolan-2-on, Propylencarbonat, 4-Methyl-1,3-dioxan-2-on, 1,3-Doxepan-2-on, 5-$C_{1-4}$-Alkoxy-1,3-dioxan-2-on; und Mischung davon, wobei jede Gruppe unsubstituiert oder mit einem oder mehreren Substituenten substituiert sein kann, die jeweils unabhängig aus $C_{1-6}$-Alkyl und $C_{1-6}$-Alkoxy ausgewählt sind, vorzugsweise wobei der cyclische Ester aus der Gruppe ausgewählt ist, umfassend Lactid, Glycolid, β-Butyrolacton, δ-Valerolacton, γ-Butyrolacton, γ-Valerolacton, 4-Methyldihydro-2(3H)-furanon, α-Methyl-γ-butyrolacton und ε-Caprolacton und Mischung davon.

8. Verfahren nach einem der Ansprüche 1 bis 7, wobei das Verfahren in Gegenwart oder Abwesenheit (in Bulk) eines Lösungsmittels durchgeführt wird.

9. Verfahren nach einem der Ansprüche 1 bis 8, wobei das Polycarbonat oder Copolymer davon wiederauftretende Einheiten der Formel (VII3) oder der Formel (VII4) umfasst,

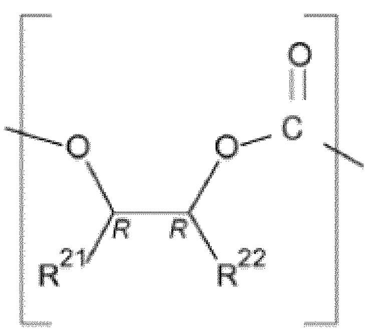 (VII3) 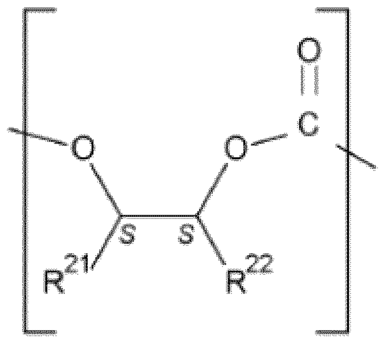 (VII4).

**10.** Verfahren zum Herstellen eines Polycarbonatpolymers nach einem der Ansprüche 1 bis 9;

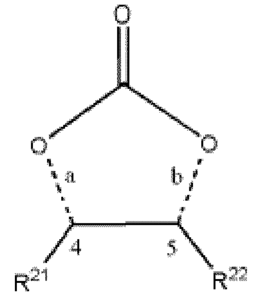 (I)

wobei:

**$R^{21}$** und **$R^{22}$** mit den Kohlenstoffatomen 4 und 5 des cyclischen Carbonats zusammengenommen einen Ring bilden, der aus $C_{3-9}$-Cycloalkylen, $C_{5-9}$-Cycloalkenylen und $C_{6-12}$-Arylen ausgewählt ist, wobei der Ring optional mit einem oder mehreren Substituenten substituiert ist, die jeweils unabhängig aus Halo, Hydroxyl, $C_{1-6}$-Alkyl, Halo-$C_{1-6}$-alkyl, $C_{1-6}$-Alkoxy, Halo-$C_{1-6}$-alkoxy, $C_{6-12}$-Aryl, $C_{1-6}$-Alkylthio, Carboxyl, $C_{1-6}$-Alkoxycarbonyl, $C_{1-6}$-Alkylcarbonyloxy, Amino; oder Mono- oder Di-$C_{1-6}$-alkylamino ausgewählt sind; wobei zwei des einen oder der mehreren Substituenten sich verbinden können, um einen Ring zu bilden, der mit dem $C_{3-9}$-Cycloalkylen, $C_{5-9}$-Cycloalkenylen oder $C_{6-12}$-Arylen fusioniert ist; wobei der von den zwei Substituenten gebildete Ring aus der Gruppe ausgewählt ist, umfassend $C_{3-9}$-Cycloalkyl, $C_{5-9}$-Cycloalkenyl, $C_{6-12}$-Aryl und Heterocyclyl, wobei jede Gruppe unsubstituiert oder mit einem oder mehreren $R^{40}$ substituiert sein kann, wobei **$R^{40}$** jeweils unabhängig aus Halo, Hydroxyl, $C_{1-6}$-Alkyl, Halo-$C_{1-6}$-alkyl, $C_{1-6}$-Alkoxy, Halo-$C_{1-6}$-alkoxy, $C_{6-12}$-Aryl, $C_{1-6}$-Alkylthio, Carboxyl, $C_{1-6}$-Alkoxycarbonyl, $C_{1-6}$-Alkylcarbonyloxy, Amino; oder Mono- oder Di-$C_{1-6}$-alkylamino ausgewählt ist.

**11.** Verfahren zum Synthetisieren einer Verbindung der Formel (I), eines Stereoisomers oder Racemats davon, das den Schritt des Inkontaktbringens einer Verbindung der Formel (V), eines Stereoisomers oder eines Racemats davon; mit zumindest einem Reagens umfasst, das aus der Gruppe ausgewählt ist, bestehend aus einem Alkyl- oder Cycloalkylchlorformiat der Formel (VI);

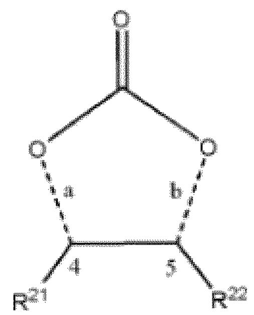 (I)

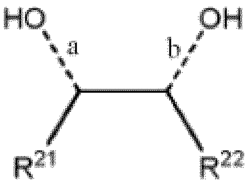 (V) 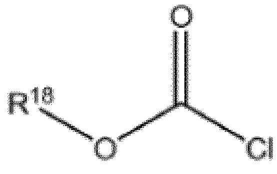 (VI),

wobei:

jede gepunktete Bindung "**a**" und "**b**" unabhängig eine feste Bindung, eine verkeilte Verbindung oder eine zerlegte verkeilte Bindung darstellt; und

**$R^{21}$** und **$R^{22}$** mit den Kohlenstoffatomen, an die sie angebunden sind, zusammengenommen einen Ring bilden, der aus $C_{3-9}$-Cycloalkylen, $C_{5-9}$-Cycloalkenylen und $C_{6-12}$-Arylen ausgewählt ist, wobei der Ring optional mit einem oder mehreren Substituenten substituiert ist, die jeweils unabhängig aus Halo, Hydroxyl, $C_{1-6}$-Alkyl, Halo-$C_{1-6}$-alkyl, $C_{1-6}$-Alkoxy, Halo-$C_{1-6}$-alkoxy, $C_{6-12}$-Aryl, $C_{1-6}$-Alkylthio, Carboxyl, $C_{1-6}$-Alkoxycarbonyl, $C_{1-6}$-Alkylcarbonyloxy, Amino; oder Mono- oder Di-$C_{1-6}$-alkylamino ausgewählt sind; und

**$R^{18}$** $C_{1-6}$-Alkyl oder $C_{3-6}$-Cycloalkyl ist.

**12.** Polycarbonat oder Copolymer davon, das eine wiederauftretende Einheit der Formel (VIII) und/oder (VII2) umfasst,

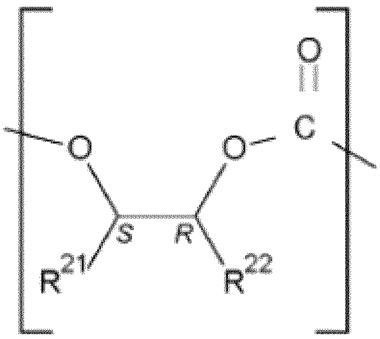 (VII1) 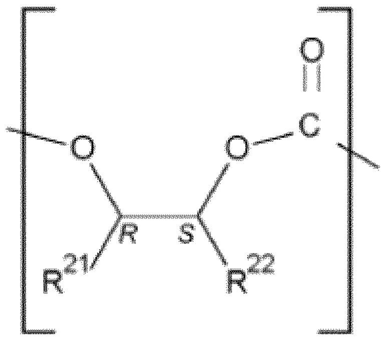 (VII2)

wobei

**$R^{21}$** und **$R^{22}$** mit den Kohlenstoffatomen, an die sie angebunden sind, zusammengenommen einen Ring bilden, der aus $C_{3-9}$-Cycloalkylen, $C_{5-9}$-Cycloalkenylen und $C_{6-12}$-Arylen ausgewählt ist, wobei der Ring optional mit einem oder mehreren Substituenten substituiert ist, die jeweils unabhängig aus Halo, Hydroxyl, $C_{1-6}$-Alkyl, Halo-$C_{1-6}$-alkyl, $C_{1-6}$-Alkoxy, Halo-$C_{1-6}$-alkoxy, $C_{6-12}$-Aryl, $C_{1-6}$-Alkylthio, Carboxyl, $C_{1-6}$-Alkoxycarbonyl, $C_{1-6}$-Alkylcarbonyloxy, Amino; oder Mono- oder Di-$C_{1-6}$-alkylamino ausgewählt sind.

**13.** Polycarbonat oder Copolymer davon nach Anspruch 12, das wiederauftretende Einheiten umfasst, die jeweils unabhängig durch die folgende Formel (VIIA) oder (VIIB) oder (VIIC) dargestellt sind;

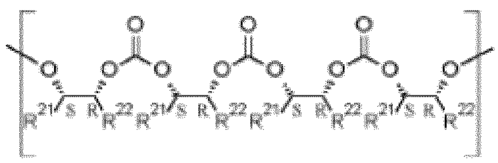

(VIIA)

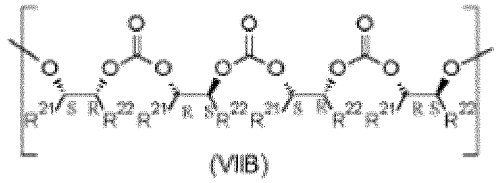

(VIIB)

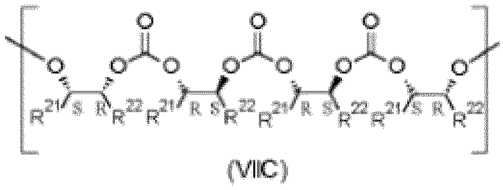

(VIIC)

wobei $R^{21}$ und $R^{22}$ die gleiche Bedeutung wie in Anspruch 12 definiert haben.

**14.** Polycarbonat oder Copolymer davon nach Anspruch 12 oder 13, das wiederauftretende Einheiten umfasst, die jeweils unabhängig durch die folgende Formel (VIIA1) oder (VIIB1) oder (VIIC1) dargestellt sind;

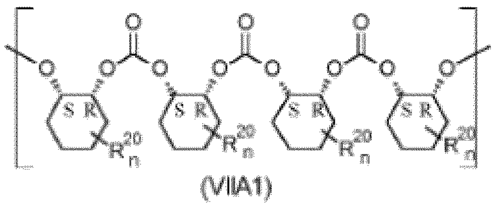

(VIIA1)

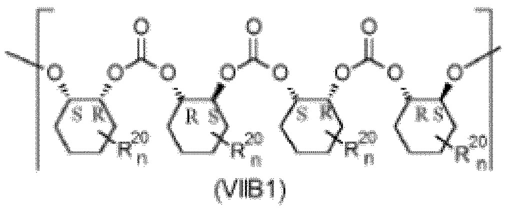

(VIIB1)

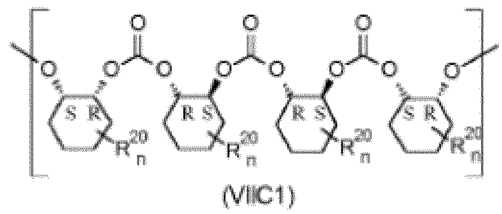

(VIIC1)

wobei $R^{20}$ jeweils unabhängig aus Halo, Hydroxyl, $C_{1-6}$-Alkyl, Halo-$C_{1-6}$-alkyl, $C_{1-6}$-Alkoxy, Halo-$C_{1-6}$-alkoxy, $C_{6-12}$-Aryl, $C_{1-6}$-Alkylthio, Carboxyl, $C_{1-6}$-Alkoxycarbonyl, $C_{1-6}$-Alkylcarbonyloxy, Amino; oder Mono- oder Di-$C_{1-6}$-alkylamino ausgewählt ist;
**n** eine ganze Zahl ist, die aus 0, 1, 2, 3 oder 4 ausgewählt ist.

**15.** Polycarbonat oder Copolymer davon, das ferner wiederauftretende Einheiten der Formel (VII3) oder der Formel (VII4) umfasst,

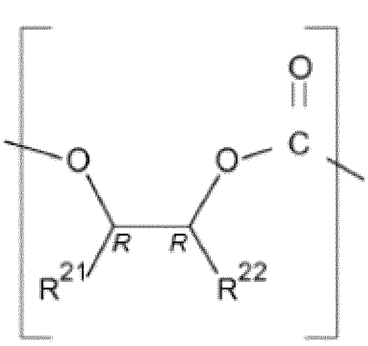

(VII3)

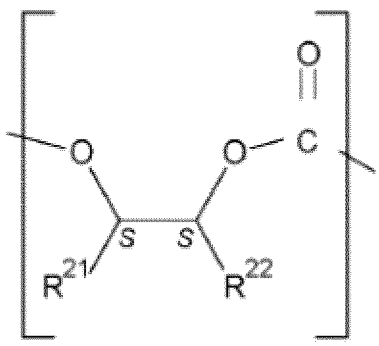

(VII4)

wobei

**$R^{21}$** und **$R^{22}$** mit den Kohlenstoffatomen 4 und 5, an die sie angebunden sind, zusammengenommen einen Ring bilden, der aus $C_{3-9}$-Cycloalkylen, $C_{5-9}$-Cycloalkenylen und $C_{6-12}$-Arylen ausgewählt ist, wobei der Ring optional mit einem oder mehreren Substituenten substituiert ist, die jeweils unabhängig aus Halo, Hydroxyl, $C_{1-6}$-Alkyl, Halo-$C_{1-6}$-alkyl, $C_{1-6}$-Alkoxy, Halo-$C_{1-6}$-alkoxy, $C_{6-12}$-Aryl, $C_{1-6}$-Alkylthio, Carboxyl, $C_{1-6}$-Alkoxycarbonyl, $C_{1-6}$-Alkylcarbonyloxy, Amino; oder Mono- oder Di-$C_{1-6}$-alkylamino ausgewählt sind; und
wobei das Polycarbonat oder Copolymer davon durch ein $^{13}C\{^1H\}$-NMR-Spektrum gekennzeichnet ist, das nur ein Signal umfasst, das in den Bereichen von $\delta$ 150 bis 155 ppm vorhanden ist.

**16.** Gegenstand, der ein Polycarbonat oder Copolymer davon nach einem der Ansprüche 12 bis 15 oder ein Polycarbonat oder Copolymer davon umfasst, das gemäß einem Verfahren nach einem der Ansprüche 1 bis 10 hergestellt wurde.

## Revendications

**1.** Procédé de préparation d'un polycarbonate ou d'un copolymère de celui-ci, ledit procédé comprenant l'homo- ou la copolymérisation en présence d'au moins un catalyseur, d'un ou plusieurs composés de formule (I), des stéréoisomères, des composés racémiques ou des mélanges de ceux-ci ; facultativement avec un ou plusieurs esters cycliques, des stéréoisomères, des composés racémiques ou des mélanges de ceux-ci ;

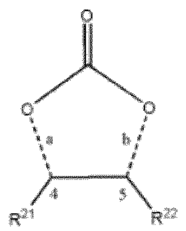
(I)

dans laquelle

chaque liaison pointillée « a » et « b » représente indépendamment une liaison dans le plan, une liaison en avant du plan ou une liaison en arrière du plan ; et
$R^{21}$ et $R^{22}$ ensemble avec les atomes de carbone 4 et 5 du carbonate cyclique forment un cycle choisi parmi les groupes cycloalkylène en $C_3$ à $C_9$, cycloalcénylène en $C_5$ à $C_9$ et arylène en $C_6$ à $C_{12}$, ledit cycle étant facultativement substitué par un ou plusieurs substituants, chacun indépendamment choisi parmi un atome d'halogène, un groupe hydroxyle, alkyle en $C_1$ à $C_6$, halogénoalkyle en $C_1$ à $C_6$, alcoxy en $C_1$ à $C_6$, halogénoalcoxy en $C_1$ à $C_6$, aryle en $C_6$ à $C_{12}$, alkylthio en $C_1$ à $C_6$, carboxyle, (alcoxy en $C_1$ à $C_6$) carbonyle, (alkyle en $C_1$ à $C_6$) carbonyloxy, amino ; ou mono- ou di-(alkyle en $C_1$ à $C_6$)amino.

**2.** Procédé selon la revendication 1, dans lequel le catalyseur est un catalyseur organométallique de formule (II) ou (III)

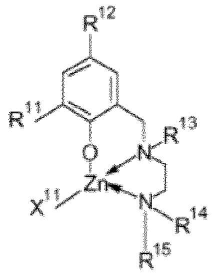
(II)

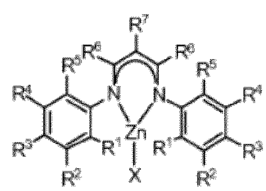
(III)

dans lesquelles

$R^1$, $R^2$, $R^3$, $R^4$, $R^5$, $R^6$ et $R^7$ sont chacun indépendamment choisis dans le groupe constitué par un atome

d'hydrogène, un groupe alkyle en $C_1$ à $C_{12}$ facultativement substitué et un groupe fonctionnel inerte et dans lesquelles deux desdits groupes ou plus peuvent être liés ensemble pour former un ou plusieurs cycles ;
X est $-N(SiR^{27}_3)_2$, un groupe alkyle en $C_1$ à $C_{12}$, un groupe alcoxy en $C_1$ à $C_{12}$, $-NR^9R^{10}$ ou $-BH_4$ ;
chaque $R^{27}$ est indépendamment choisi parmi un atome d'hydrogène et un groupe alkyle en $C_1$ à $C_6$ ;
chaque $R^9$ et $R^{10}$ est indépendamment choisi parmi un atome d'hydrogène et un groupe alkyle en $C_1$ à $C_6$ ;
$R^{11}$ et $R^{12}$ sont chacun indépendamment un groupe alkyle en $C_1$ à $C_{10}$ ;
$R^{13}$, $R^{14}$ et $R^{15}$ sont chacun indépendamment un groupe alkyle en $C_1$ à $C_{10}$ ; ou
$R^{13}$ et $R^{14}$ sont liés de manière covalente l'un à l'autre et sont chacun un groupe méthylène et $R^{15}$ est un groupe alkyle en $C_1$ à $C_{10}$ ;
$X^{11}$ est choisi parmi un groupe alkyle en $C_1$ à $C_{10}$, $-OR^{16}$ et $-N(SiR^{27}_3)_2$ ;
$R^{16}$ est un groupe alkyle en $C_1$ à $C_{10}$ ; et
chaque $R^{17}$ est indépendamment choisi parmi un atome d'hydrogène et un groupe alkyle en $C_1$ à $C_6$ ; de préférence le catalyseur est choisi parmi [(NNO)ZnEt], [BDI]Zn($N(SiMe_3)_2$), [BDI] Zn (Et) et {[BDI]Zn($OR^{30}$)}$_2$, où $R^{30}$ est un groupe alkyle en $C_1$ à $C_6$ ; de préférence le catalyseur est [(NNO)ZnEt]

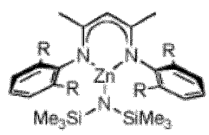

R = $^iPr$

(BDI)Zn($NTMS_2$)

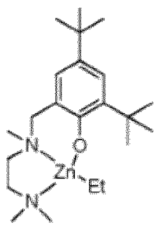

(NNO)ZnEt

3. Procédé selon la revendication 1, dans lequel le catalyseur est choisi parmi les complexes de formules $M(OSO_2CF_3)_m$, $M(N(OSO_2CF_3)_2)_m$, $M(R^{23}C(O)CR^{23}_2C(O)R^{23})_m$ et $(R^{24}CO_2)_mM$ où M est un métal des groupes 2, 3, y compris la série des lanthanides, ou des groupes 12, 13, 15, où chaque $R^{23}$ est indépendamment un groupe alkyle en $C_1$ à $C_{12}$ facultativement substitué, où chaque $R^{24}$ est indépendamment un groupe alkyle en $C_1$ à $C_{12}$ perfluoré ou un groupe aryle, et où m est la valence de M.

4. Procédé selon la revendication 1, dans lequel le catalyseur est choisi dans le groupe comprenant les bases de phosphazène dimérique, ou les amines ou les guanidines, les acides organiques, la spartéine, les dérivés de thiourée-amino, les carbènes N-hétérocycliques, les 4-(dialkylamino)pyridines, les amidines, les phosphines à base de phosphore, les dérivés de phosphazénium et les phosphazènes, les amides métalliques ; et les composés de formule générale $M^1(Y^1, Y^2,...Y^p)_q$ où $M^1$ est un métal choisi dans le groupe comprenant les éléments des colonnes 3 à 12 du tableau périodique des éléments, et les éléments Al, Ga, In, Tl, Ge, Sn, Pb, Sb, Ca, Mg et Bi ; où $Y^1$, $Y^2$,...$Y^P$ sont chacun des substituants choisis dans le groupe comprenant les groupes alkyle ayant de 1 à 20 atomes de carbone, aryle ayant de 6 à 30 atomes de carbone, alcoxy ayant de 1 à 20 atomes de carbone, aryloxy ayant de 6 à 30 atomes de carbone, oxyde, carboxylate et halogénures, et p et q sont des nombres entiers de 1 à 6 ; de préférence le catalyseur est choisi dans le groupe comprenant le 1,5,7-triazobicyclo[4,4,0]déc-5-ène (TBD), le 1,8-diazabicyclo[5.4.0]undéc-7-ène (DBU), la 4-diméthylaminopyridine (DMAP) et la *tert*-butylimino-1,3-diméthylperhydro-1,3,2-diazaphosphine (BEMP) ; $Sn(Oct)_2$, $Al(OiPr)_3$, $Ti(OiPr)_4$, Ti(hexanoate de 2-éthyle)$_4$, Ti(oxyde de 2-éthylhexyle)$_4$, $Zr(OiPr)_4$, Bi(néodécanoate)$_3$, (2,4-di-tert-butyl-6-(((2-(diméthylamino)éthyl)(méthyl)amino)méthyl)phénoxy)-(éthoxy) zinc, Zn(lactate)$_2$ ; $Y[N(SiMe_3)_2]_3$ ; [Y] + L1 ; et [Y] + L2, où L1 est un ligand tétradentate de formule L1 et L2 est un ligand tétradentate de formule L2

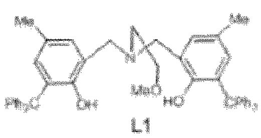

L1

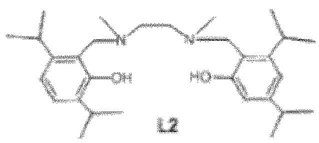

L2

5. Procédé selon l'une quelconque des revendications 1 à 4, ledit procédé étant réalisé en présence d'un composé de formule (IV)

$R^8$-OH (IV)

dans laquelle $R^8$ est choisi dans le groupe constitué par les groupes alkyle en $C_1$ à $C_{20}$, aryle en $C_6$ à $C_{30}$ et (aryle en $C_6$ à $C_{30}$) alkyle en $C_1$ à $C_{20}$ facultativement substitué par un ou plusieurs substituants choisis dans le groupe constitué par un atome d'halogène, un groupe hydroxyle et un groupe alkyle en $C_1$ à $C_6$.

**6.** Procédé selon l'une quelconque des revendications 1 à 5, dans lequel l'ester cyclique est choisi dans le groupe comprenant les lactones, les carbonates cycliques, les glycolides et les lactides, chaque groupe pouvant être non substitué ou substitué par un ou plusieurs substituants choisis chacun indépendamment parmi les groupes alkyle en $C_1$ à $C_6$ et alcoxy en $C_1$ à $C_6$, de préférence les lactones, les glycolides et les lactides.

**7.** Procédé selon l'une quelconque des revendications 1 à 6, dans lequel l'ester cyclique est choisi dans le groupe comprenant le lactide, le carbonate de triméthylène, le glycolide, la β-butyrolactone, la δ-valérolactone, la γ-butyrolactone, la γ-valérolactone, la 4-méthyldihydro-2(3H)-furanone, l'alpha-méthyl-gamma-butyrolactone et l'ε-caprolactone, la 1,3-dioxolane-2-one, le carbonate de propylène, la 4-méthyl-l,3-dioxan-2-one, la 1,3-doxépan-2-one, une 5-alcoxy en $C_1$ à $C_4$-1,3-dioxan-2-one ; et un mélange de ceux-ci, chaque groupe pouvant être non substitué ou substitué par un ou plusieurs substituants choisis chacun indépendamment parmi les groupes alkyle en $C_1$ à $C_6$ et alcoxy en $C_1$ à $C_6$, de préférence dans lequel ledit ester cyclique est choisi dans le groupe comprenant le lactide, le glycolide, la β-butyrolactone, la δ-valérolactone, la γ-butyrolactone, la γ-valérolactone, la 4-méthyldihydro-2(3H)-furanone, l'alpha-méthyl-gamma-butyrolactone et l'ε-caprolactone, et un mélange de ceux-ci.

**8.** Procédé selon l'une quelconque des revendications 1 à 7, dans lequel le procédé est réalisé en présence ou en l'absence (en gros) de solvant.

**9.** Procédé selon l'une quelconque des revendications 1 à 8, dans lequel le polycarbonate ou un copolymère de celui-ci comprend des motifs répétitifs de formule (VII3) ou de formule (VVI4),

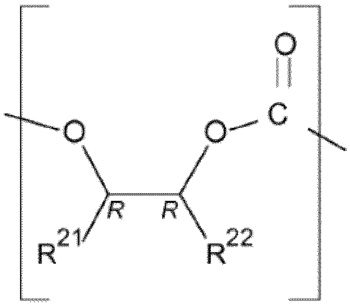
(VII3)

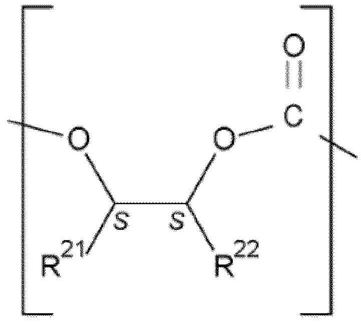
(VII4).

**10.** Procédé de préparation d'un copolymère de polycarbonate selon l'une quelconque des revendications 1 à 9 ;

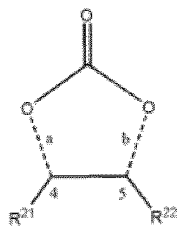
(I)

dans laquelle
$R^{21}$ et $R^{22}$ ensemble avec les atomes de carbone 4 et 5 du carbonate cyclique forment un cycle choisi parmi les groupes cycloalkylène en $C_3$ à $C_9$, cycloalcénylène en $C_5$ à $C_9$ et arylène en $C_6$ à $C_{12}$, ledit cycle étant facultativement substitué par un ou plusieurs substituants, chacun indépendamment choisi parmi un atome d'halogène, un groupe hydroxyle, alkyle en $C_1$ à $C_6$, halogénoalkyle en $C_1$ à $C_6$, alcoxy en $C_1$ à $C_6$, halogénoalcoxy en $C_1$ à $C_6$, aryle en $C_6$ à $C_{12}$, alkylthio en $C_1$ à $C_6$, carboxyle, (alcoxy en $C_1$ à $C_6$) carbonyle, (alkyle en $C_1$ à $C_6$) carbonyloxy, amino ; ou mono- ou di-(alkyle en $C_1$ à $C_6$)amino ; au moins deux desdits substituants pouvant être liés pour former un cycle condensé audit groupe cycloalkylène en $C_3$ à $C_9$, cycloalcénylène en $C_5$ à $C_9$ ou arylène en $C_6$ à $C_{12}$ ; ledit cycle formé par lesdits deux substituants étant choisi dans le groupe comprenant les groupes cycloalkyle en $C_3$ à

$C_9$, cycloalcényle en $C_5$ à $C_9$, aryle en $C_6$ à $C_{12}$ et hétéroaryle, chaque groupe pouvant être non substitué ou substitué par un ou plusieurs $R^{40}$, chaque $R^{40}$ étant indépendamment choisi parmi un atome d'halogène, un groupe hydroxyle, alkyle en $C_1$ à $C_6$, halogénoalkyle en $C_1$ à $C_6$, alcoxy en $C_1$ à $C_6$, halogénoalcoxy en $C_1$ à $C_6$, aryle en $C_6$ à $C_{12}$, alkylthio en $C_1$ à $C_6$, carboxyle, (alcoxy en $C_1$ à $C_6$)carbonyle, (alkyle en $C_1$ à $C_6$)carbonyloxy, amino ; ou mono- ou di-(alkyle en $C_1$ à $C_6$) amino.

**11.** Procédé de synthèse d'un composé de formule (I), d'un stéréoisomère ou d'un racémate de celui-ci, comprenant l'étape de mise en contact d'un composé de formule (V), d'un stéréoisomère ou d'un racémate de celui-ci ; avec au moins un réactif choisi dans le groupe constitué par un chloroformiate d'alkyle ou de cycloalkyle de formule (VI) ;

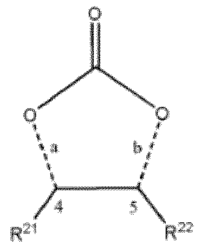 (I)

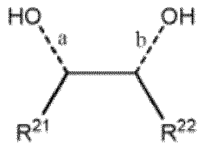 (V) 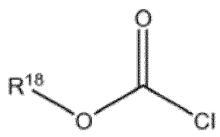 (VI),

dans lesquelles

chaque liaison pointillée « a » et « b » représente indépendamment une liaison dans le plan, une liaison en avant du plan ou une liaison en arrière du plan ;
$R^{21}$ et $R^{22}$ ensemble avec les atomes de carbone auxquels ils sont attachés forment un cycle choisi parmi les groupes cycloalkylène en $C_3$ à $C_9$, cycloalcénylène en $C_5$ à $C_9$ et arylène en $C_6$ à $C_{12}$, ledit cycle étant facultativement substitué par un ou plusieurs substituants, chacun indépendamment choisi parmi un atome d'halogène, un groupe hydroxyle, alkyle en $C_1$ à $C_6$, halogénoalkyle en $C_1$ à $C_6$, alcoxy en $C_1$ à $C_6$, halogénoalcoxy en $C_1$ à $C_6$, aryle en $C_6$ à $C_{12}$, alkylthio en $C_1$ à $C_6$, carboxyle, (alcoxy en $C_1$ à $C_6$) carbonyle, (alkyle en $C_1$ à $C_6$) carbonyloxy, amino ; ou mono- ou di-(alkyle en $C_1$ à $C_6$)amino ; et
$R^{18}$ est un groupe alkyle en $C_1$ à $C_6$ ou cycloalkyle en $C_3$ à $C_6$.

**12.** Polycarbonate ou un copolymère de celui-ci comprenant un motif répétitif de formule (VII1) et/ou (VII2)

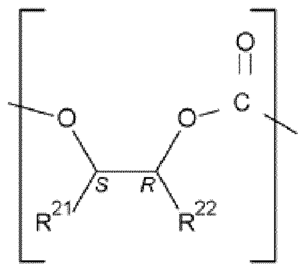 (VII1) 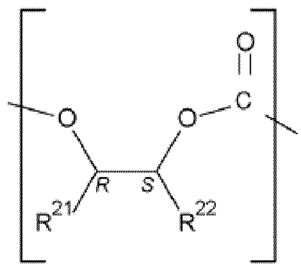 (VII2)

dans lesquelles
$R^{21}$ et $R^{22}$ ensemble avec les atomes de carbone auxquels ils sont attachés forment un cycle choisi parmi les groupes cycloalkylène en $C_3$ à $C_9$, cycloalcénylène en $C_5$ à $C_9$ et arylène en $C_6$ à $C_{12}$, ledit cycle étant facultativement substitué par un ou plusieurs substituants, chacun indépendamment choisi parmi un atome d'halogène, un groupe hydroxyle, alkyle en $C_1$ à $C_6$, halogénoalkyle en $C_1$ à $C_6$, alcoxy en $C_1$ à $C_6$, halogénoalcoxy en $C_1$ à $C_6$, aryle en $C_6$ à $C_{12}$, alkylthio en $C_1$ à $C_6$, carboxyle, (alcoxy en $C_1$ à $C_6$) carbonyle, (alkyle en $C_1$ à $C_6$) carbonyloxy, amino ; ou mono- ou di-(alkyle en $C_1$ à $C_6$)amino.

**13.** Polycarbonate ou un copolymère de celui-ci selon la revendication 12, comprenant des motifs répétitifs, chacun indépendamment représenté par la formule (VIIA) ou (VIIB) ou (VIIC) suivante ;

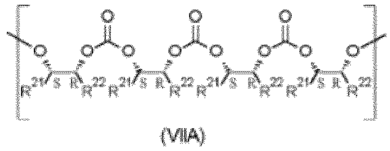

(VIIA)

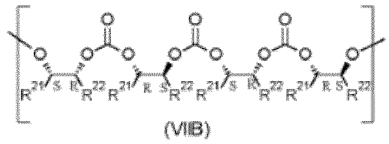

(VIIB)

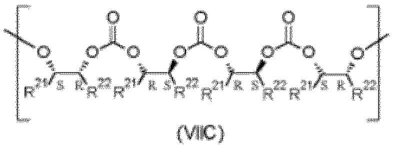

(VIIC)

dans lesquelles $R^{21}$ et $R^{22}$ ont la même signification que définie dans la revendication 12.

**14.** Polycarbonate ou un copolymère de celui-ci selon la revendication 12 ou 13, comprenant des motifs répétitifs, chacun indépendamment représenté par la formule (VIIA1) ou (VIIB1) ou (VIIC1) suivante ;

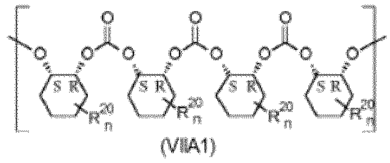

(VIIA1)

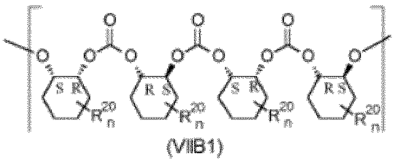

(VIIB1)

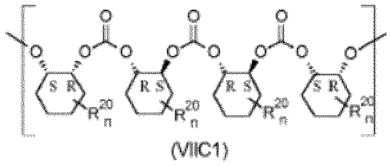

(VIIC1)

chaque $R^{20}$ est indépendamment choisi parmi un atome d'halogène, un groupe hydroxyle, alkyle en $C_1$ à $C_6$, halogénoalkyle en $C_1$ à $C_6$, alcoxy en $C_1$ à $C_6$, halogénoalcoxy en $C_1$ à $C_6$, aryle en $C_6$ à $C_{12}$, alkylthio en $C_1$ à $C_6$, carboxyle, (alcoxy en $C_1$ à $C_6$) carbonyle, (alkyle en $C_1$ à $C_6$) carbonyloxy, amino ; ou mono- ou di-(alkyle en $C_1$ à $C_6$)amino ;

n est un nombre entier choisi parmi 0, 1, 2, 3 ou 4.

**15.** Polycarbonate ou un copolymère de celui-ci comprenant des motifs répétitifs de formule (VII3) ou de formule (VII4)

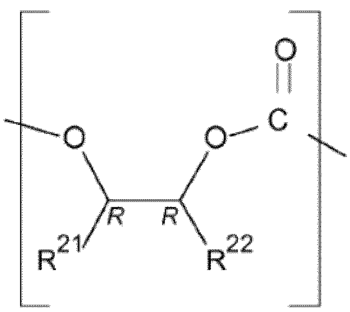 (VII3) 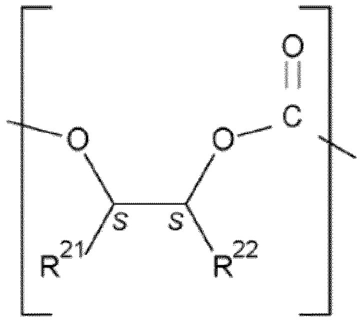 (VII4).

dans lesquelles

$R^{21}$ et $R^{22}$ ensemble avec les atomes de carbone 4 et 5 auxquels ils sont attachés forment un cycle choisi parmi les groupes cycloalkylène en $C_3$ à $C_9$, cycloalcénylène en $C_5$ à $C_9$ et arylène en $C_6$ à $C_{12}$, ledit cycle étant facultativement substitué par un ou plusieurs substituants, chacun indépendamment choisi parmi un atome d'halogène, un groupe hydroxyle, alkyle en $C_1$ à $C_6$, halogénoalkyle en $C_1$ à $C_6$, alcoxy en $C_1$ à $C_6$, halogénoalcoxy en $C_1$ à $C_6$, aryle en $C_6$ à $C_{12}$, alkylthio en $C_1$ à $C_6$, carboxyle, (alcoxy en $C_1$ à $C_6$)carbonyle, (alkyle en $C_1$ à $C_6$)carbonyloxy, amino ; et

ledit polycarbonate ou copolymère de celui-ci étant **caractérisé par** un spectre RMN $^{13}C\{^1H\}$ comprenant uniquement un signal présent dans les plages allant de $\delta$ 150 à 155 ppm.

**16.** Article comprenant un polycarbonate ou un copolymère de celui-ci selon l'une quelconque des revendications 12 à 15 ou un polycarbonate ou un copolymère de celui-ci préparé selon l'une quelconque des revendications 1 à 10.

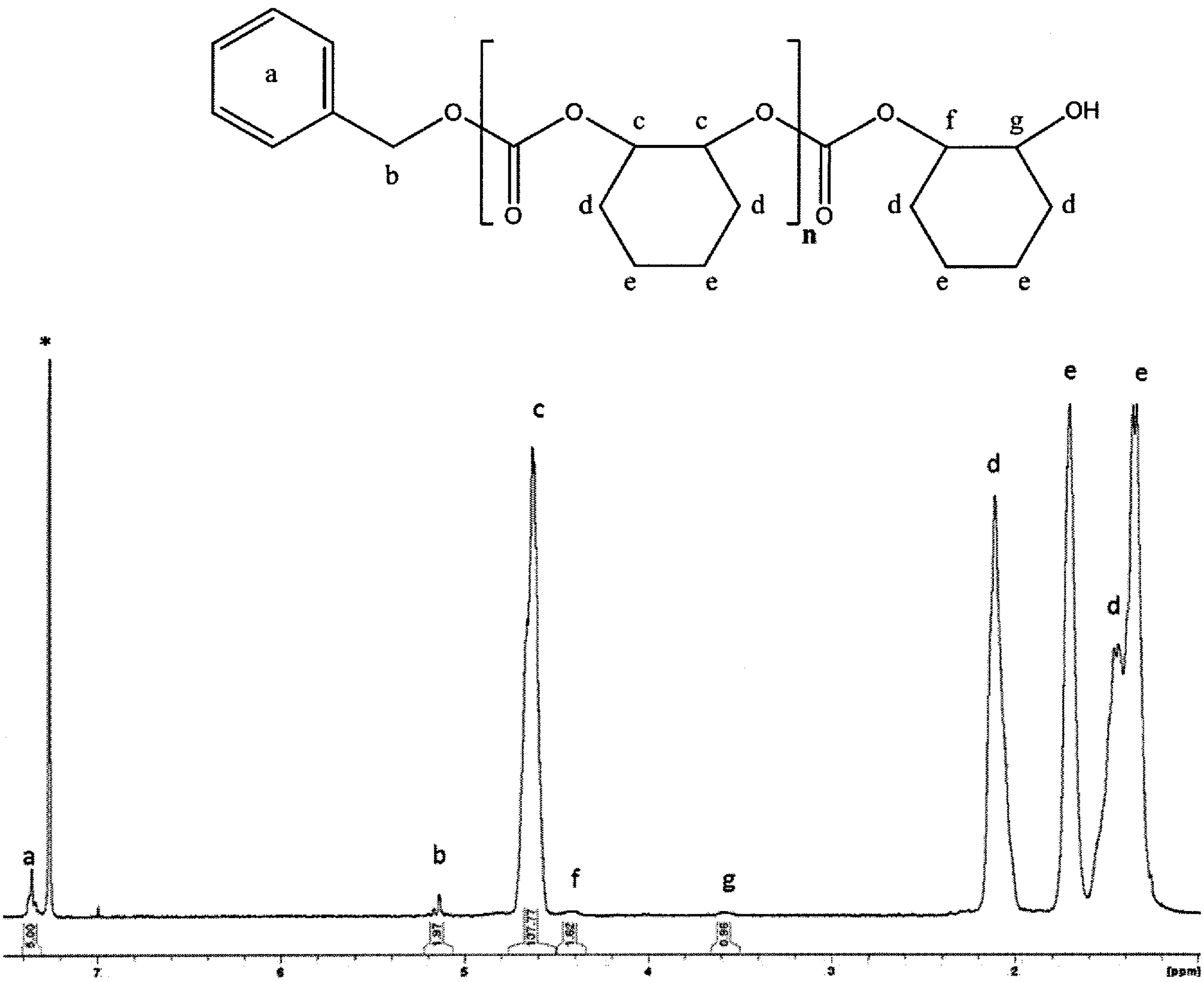


FIG. 1

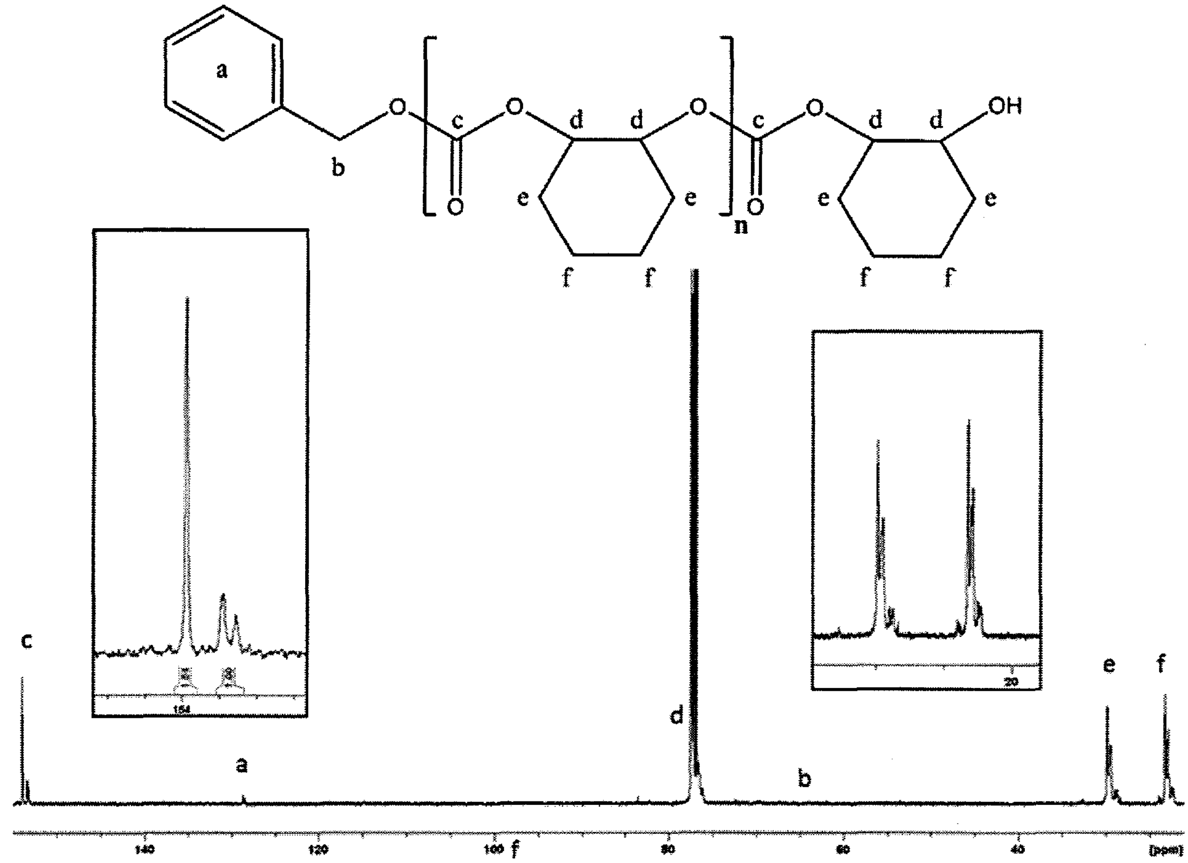
140
120
100
80
60
40
[ppm]

**FIG. 2**

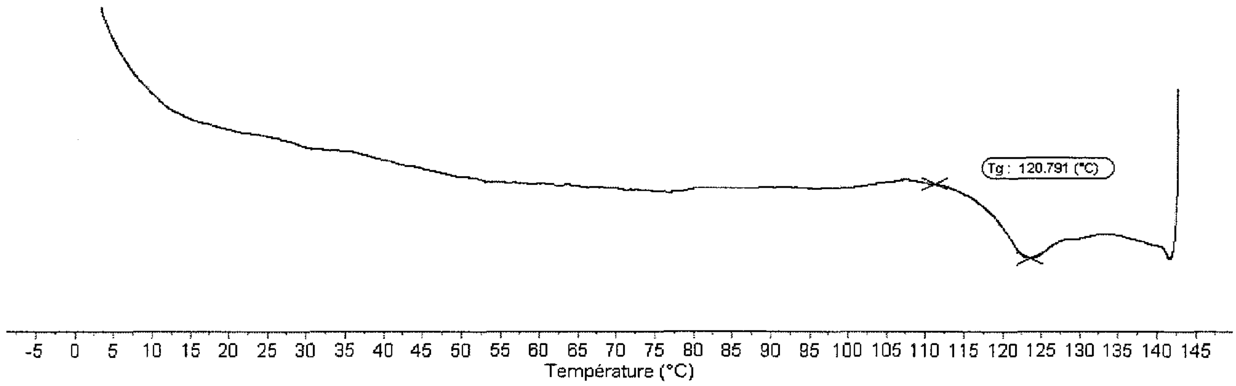
Tg : 120.791 (°C)
-5 0 5 10 15 20 25 30 35 40 45 50 55 60 65 70 75 80 85 90 95 100 105 110 115 120 125 130 135 140 145
Température (°C)

**FIG. 3**

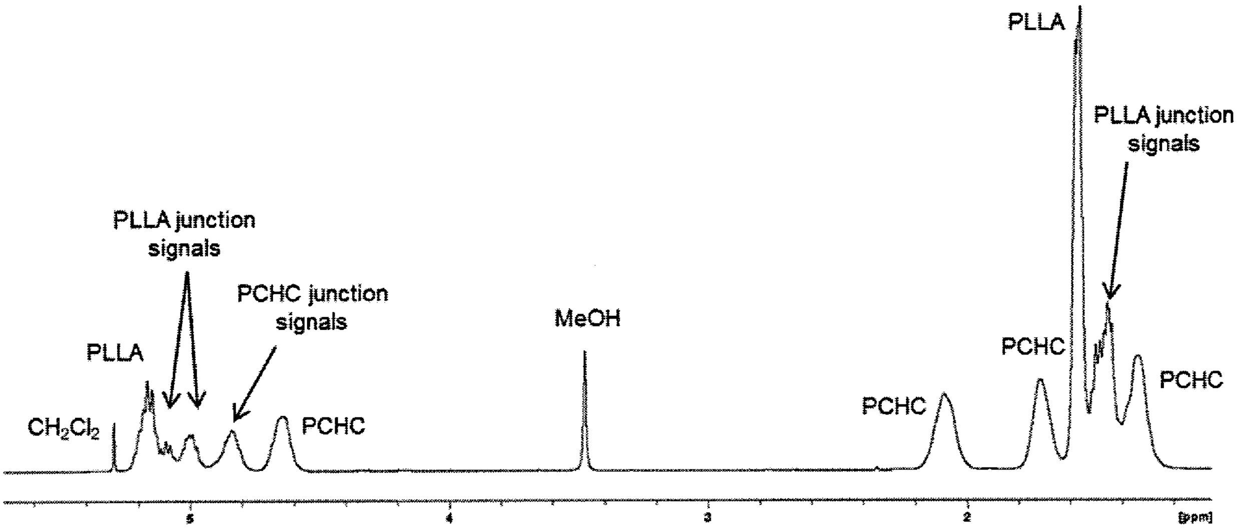
PLLA
PLLA junction signals
PLLA junction signals
PCHC junction signals
MeOH
PLLA
CH$_2$Cl$_2$
PCHC
PCHC
PCHC
PCHC
5
4
3
2
[ppm]

FIG. 4

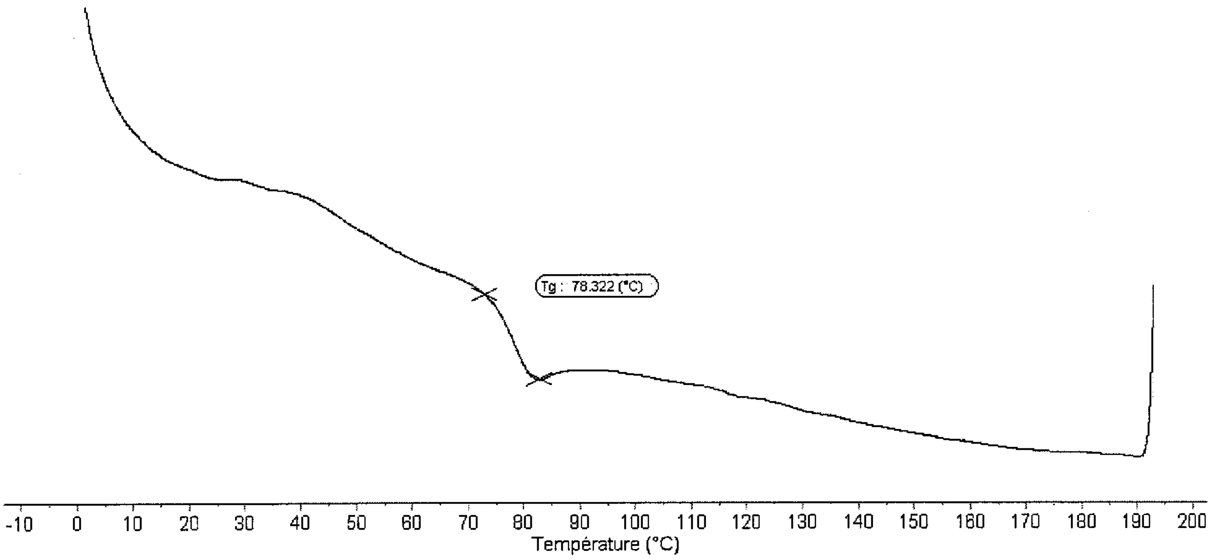
Tg : 78.322 (°C)
-10
0
10
20
30
40
50
60
70
80
90
100
110
120
130
140
150
160
170
180
190
200
Température (°C)

FIG. 5

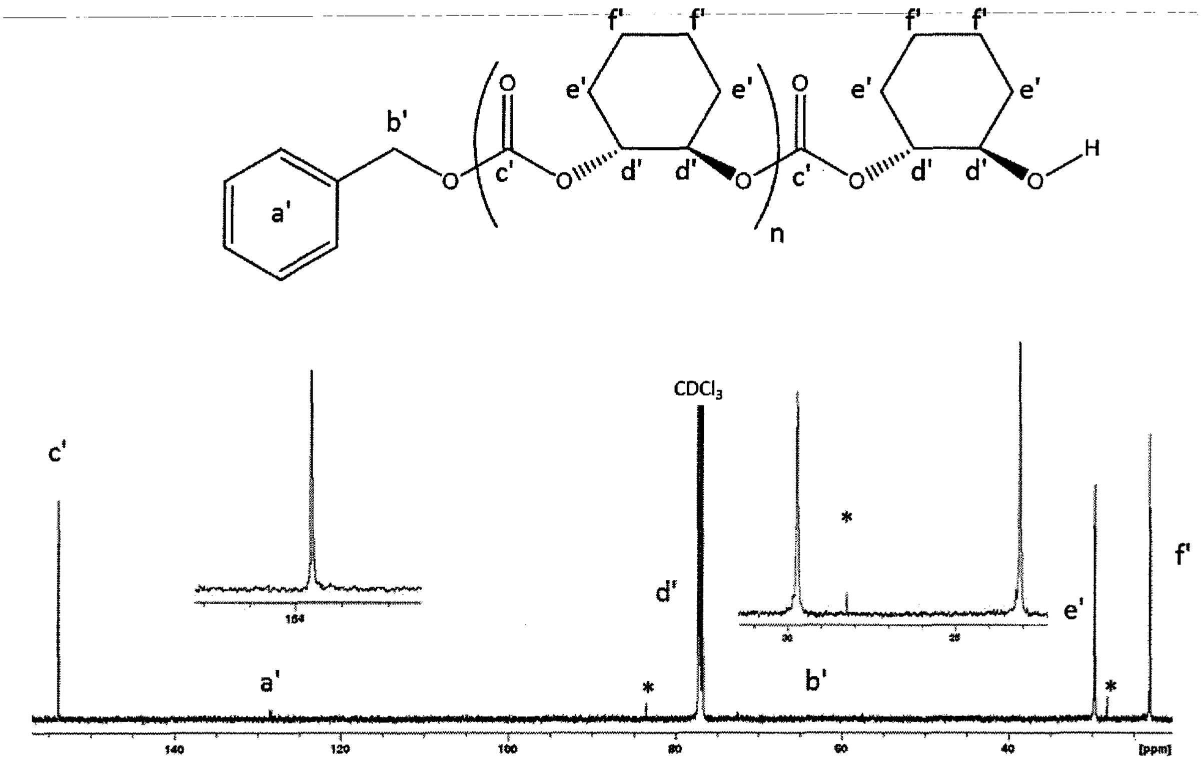

*residual (R,R)CHC

FIG. 6

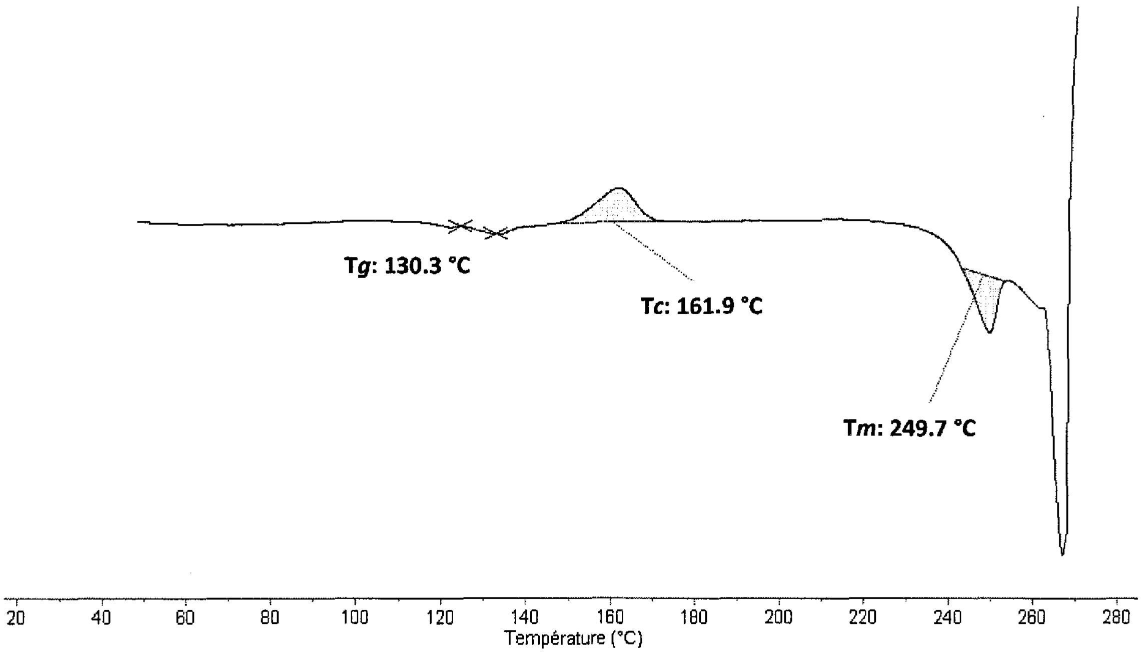


FIG. 7

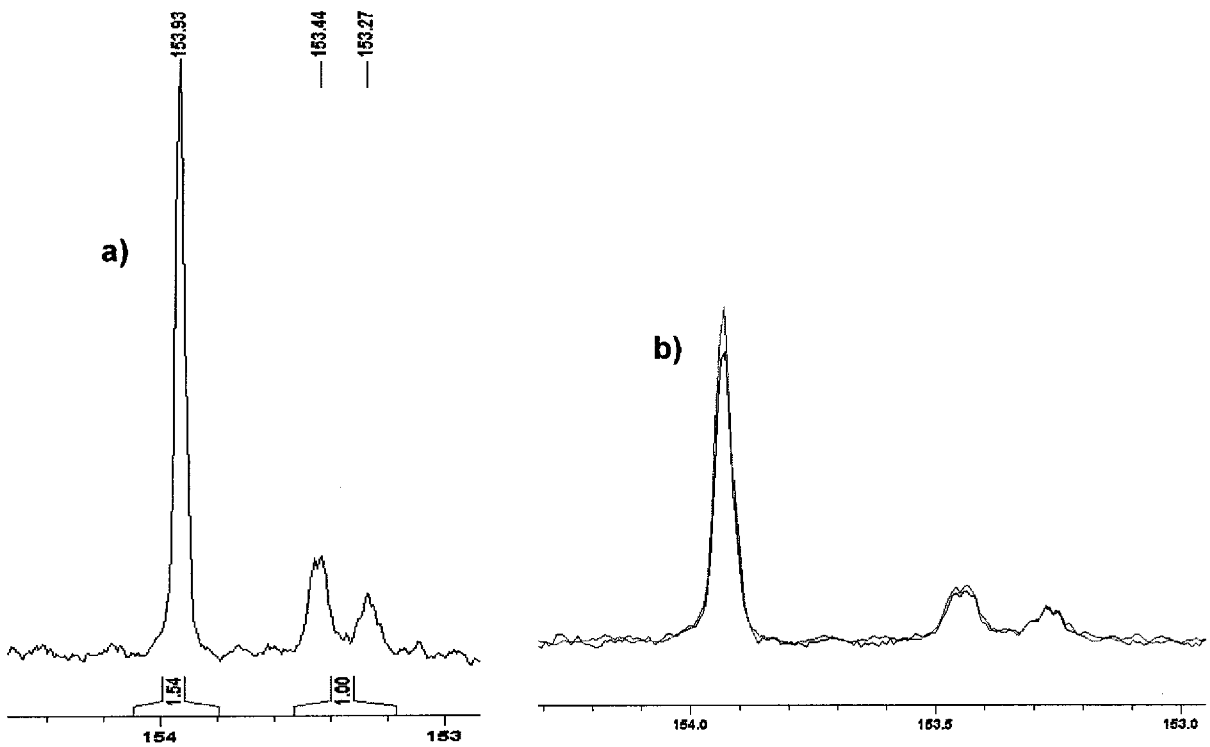
153.93
153.44
153.27
a)
1.54
1.00
154
153
b)
154.0
153.5
153.0

FIG. 8

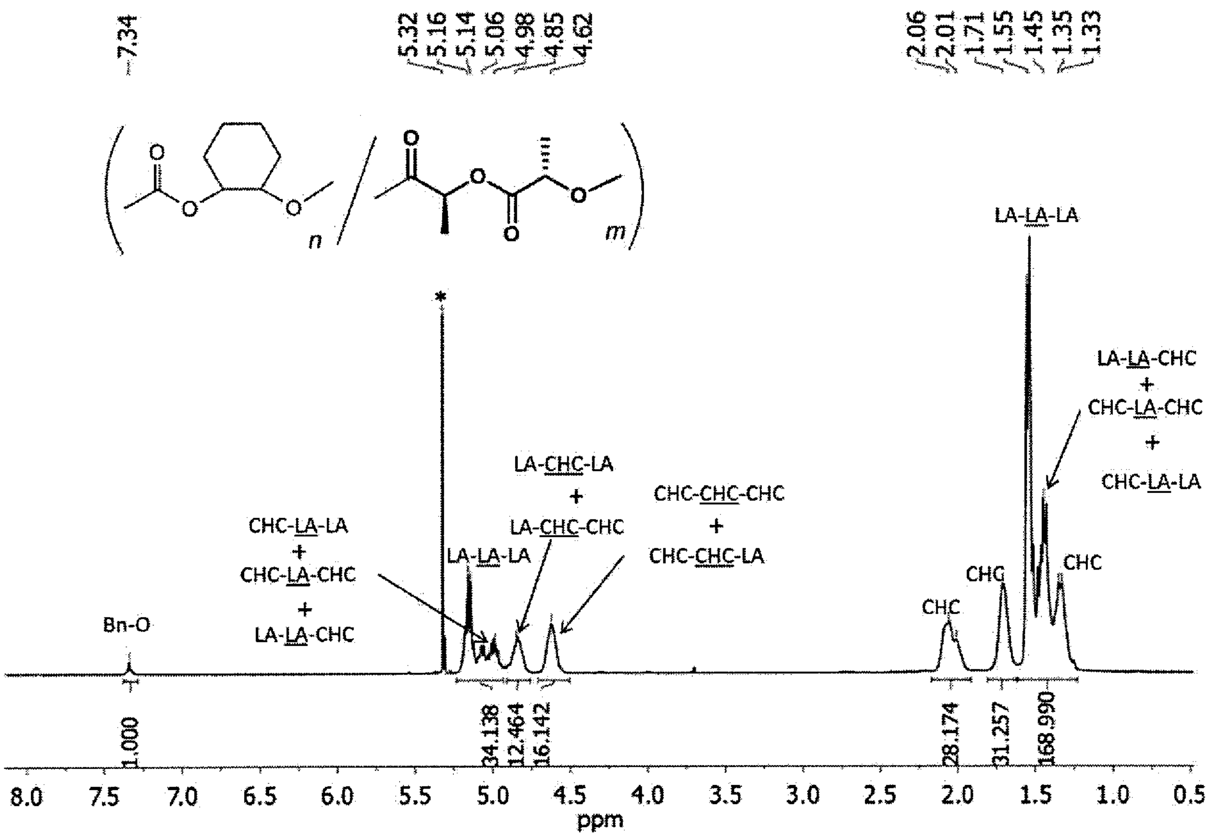
7.34
5.32
5.16
5.14
5.06
4.98
4.85
4.62
2.06
2.01
1.71
1.55
1.45
1.35
1.33
n
m
*
LA-LA-LA
LA-LA-CHC
+
CHC-LA-CHC
+
CHC-LA-LA
LA-CHC-LA
+
LA-CHC-CHC
CHC-CHC-CHC
+
CHC-CHC-LA
CHC-LA-LA
+
CHC-LA-CHC
+
LA-LA-CHC
LA-LA-LA
CHC
CHC
CHC
Bn-O
1.000
34.138
12.464
16.142
28.174
31.257
168.990
8.0
7.5
7.0
6.5
6.0
5.5
5.0
4.5
4.0
3.5
3.0
2.5
2.0
1.5
1.0
0.5
ppm

FIG. 9

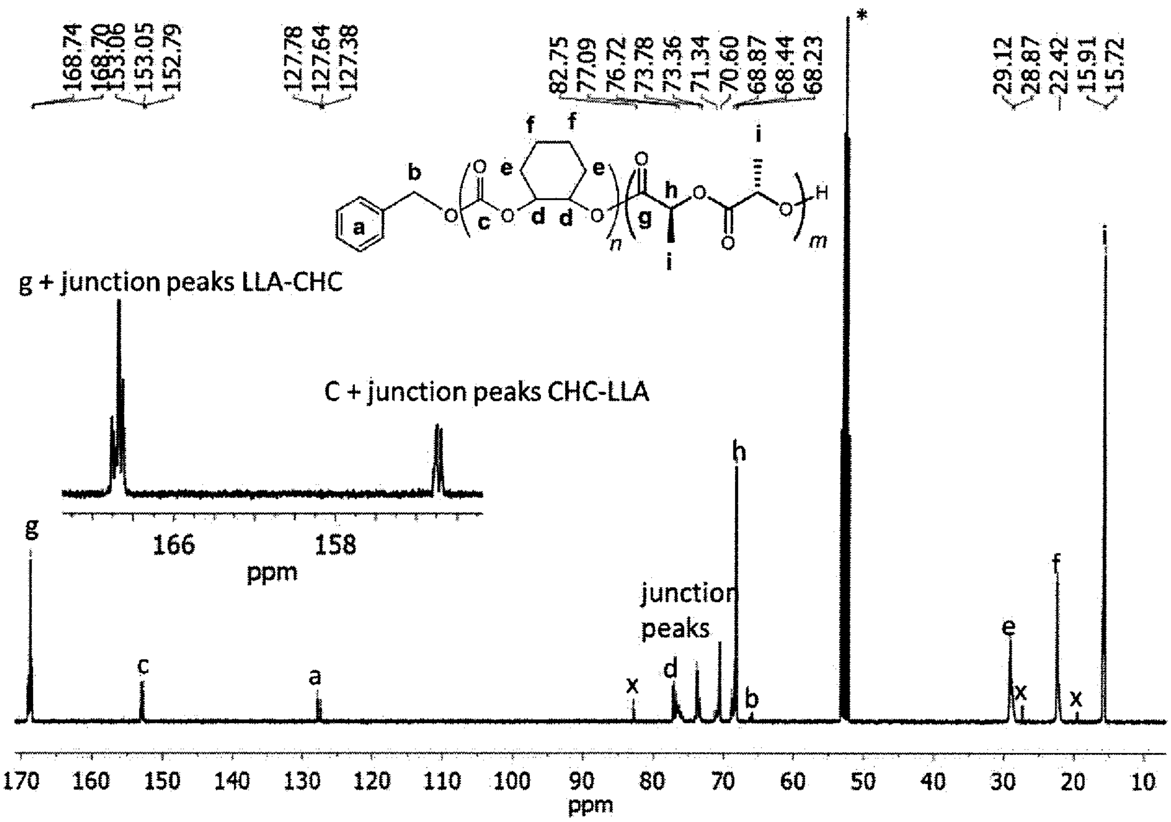
168.74
168.70
153.06
153.05
152.79
127.78
127.64
127.38
82.75
77.09
76.72
73.78
73.36
71.34
70.60
68.87
68.44
68.23
29.12
28.87
22.42
15.91
15.72
g + junction peaks LLA-CHC
C + junction peaks CHC-LLA
166
158
ppm
junction peaks
170 160 150 140 130 120 110 100 90 80 70 60 50 40 30 20 10
ppm

FIG. 10

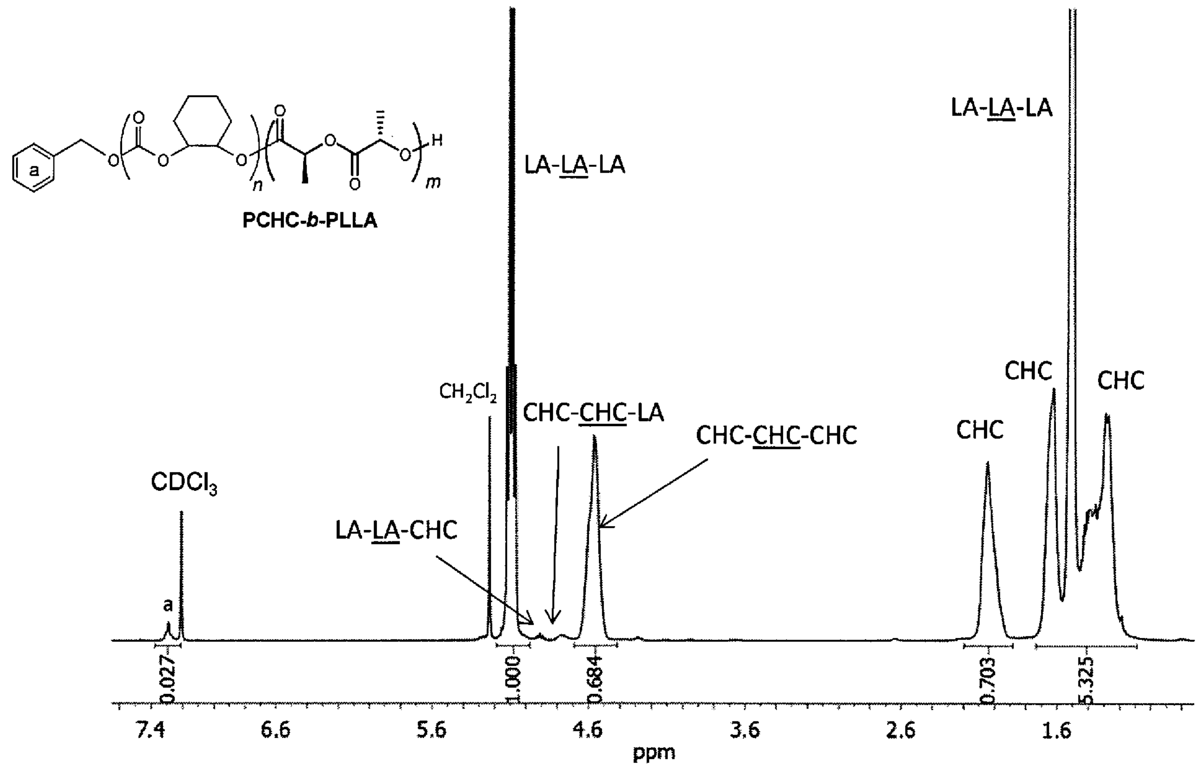
PCHC-*b*-PLLA
LA-LA-LA
LA-LA-LA
CH2Cl2
CHC-CHC-LA
CHC-CHC-CHC
CHC
CHC
CHC
CDCl3
LA-LA-CHC
0.027
1.000
0.684
0.703
5.325
7.4 6.6 5.6 4.6 3.6 2.6 1.6
ppm

FIG. 11

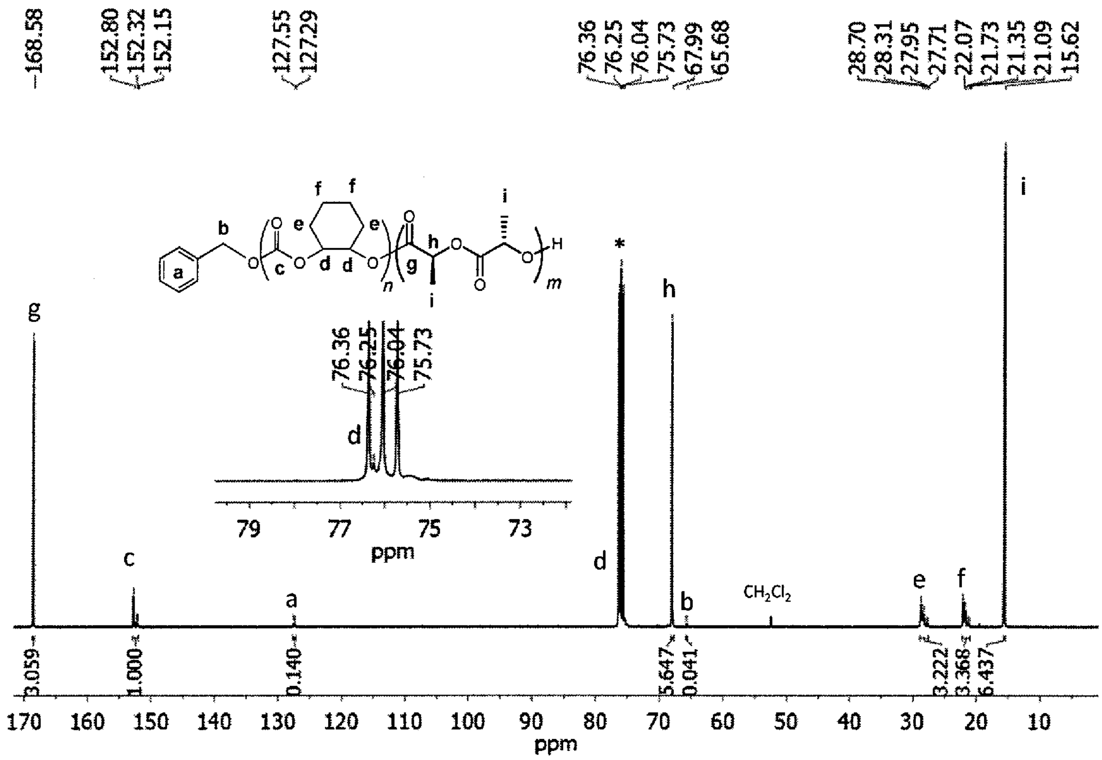

168.58
152.80
152.32
152.15
127.55
127.29
76.36
76.25
76.04
75.73
67.99
65.68
28.70
28.31
27.95
27.71
22.07
21.73
21.35
21.09
15.62
g
c
a
d
h
b
i
e
f
*
$CH_2Cl_2$
3.059
1.000
0.140
5.647
0.041
3.222
3.368
6.437
ppm


FIG. 12

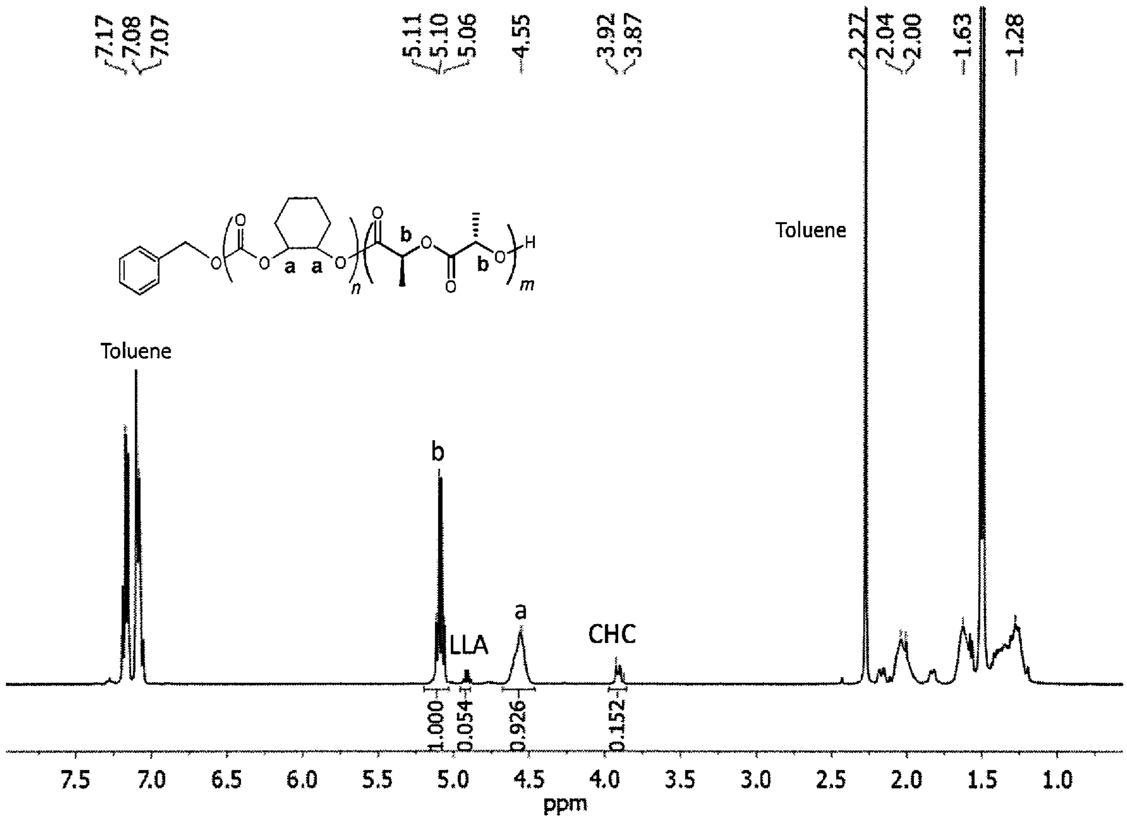

7.17
7.08
7.07
5.11
5.10
5.06
4.55
3.92
3.87
2.27
2.04
2.00
1.63
1.28
Toluene
Toluene
b
LLA
a
CHC
1.000
0.054
0.926
0.152
ppm


FIG. 13

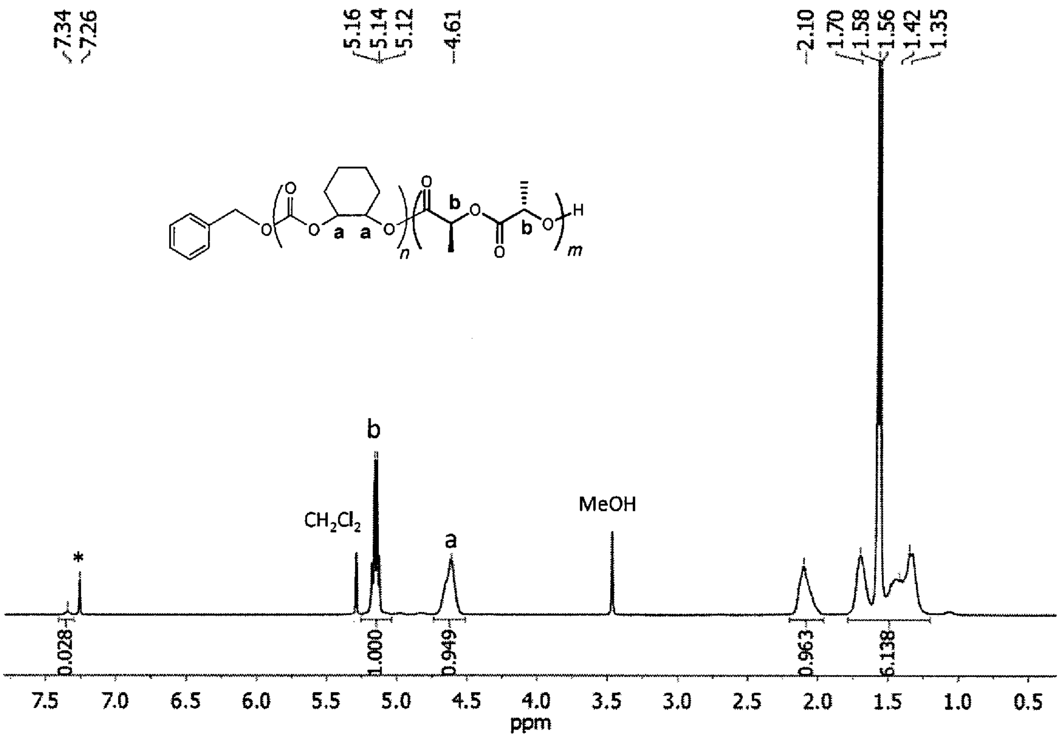
7.34
7.26
5.16
5.14
5.12
4.61
2.10
1.70
1.58
1.56
1.42
1.35
b
a
CH2Cl2
MeOH
*
0.028
1.000
0.949
0.963
6.138
7.5 7.0 6.5 6.0 5.5 5.0 4.5 4.0 3.5 3.0 2.5 2.0 1.5 1.0 0.5
ppm

**FIG. 14**

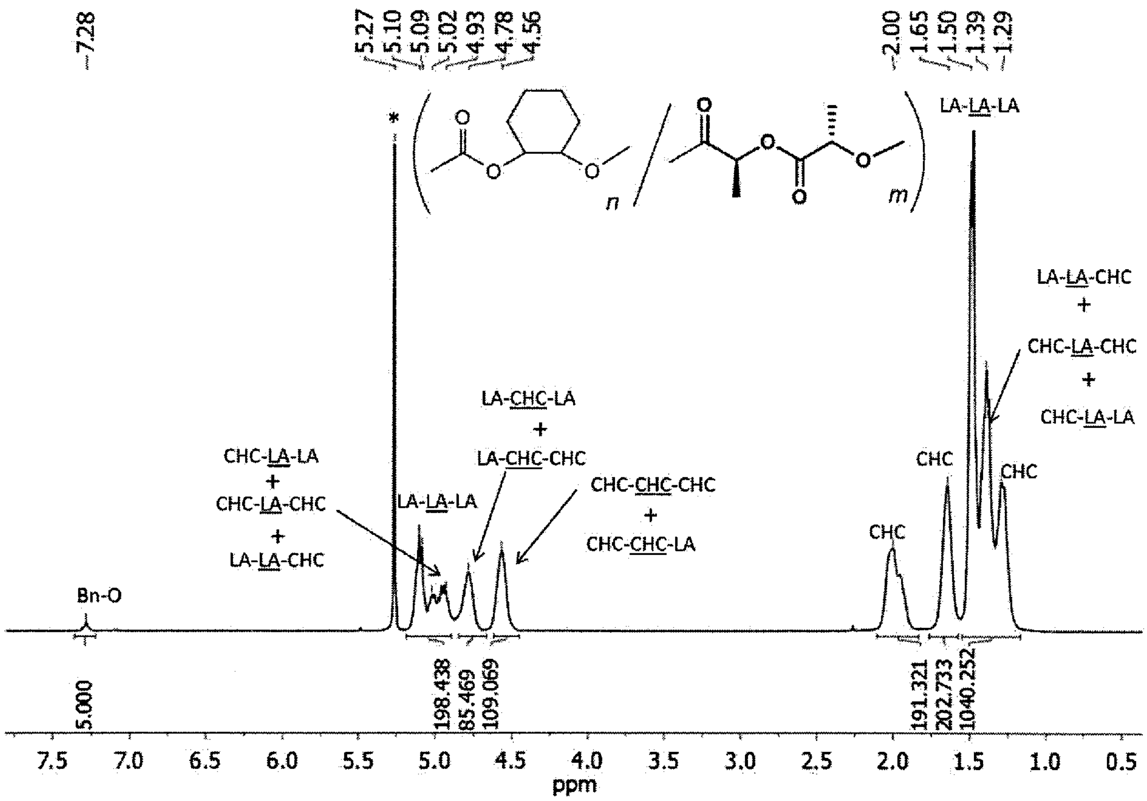
7.28
5.27
5.10
5.09
5.02
4.93
4.78
4.56
2.00
1.65
1.50
1.39
1.29
*
LA-LA-LA
LA-LA-CHC + CHC-LA-CHC + CHC-LA-LA
LA-CHC-LA + LA-CHC-CHC
CHC-CHC-CHC + CHC-CHC-LA
CHC-LA-LA + CHC-LA-CHC + LA-LA-CHC
LA-LA-LA
CHC
CHC
CHC
Bn-O
5.000
198.438
85.469
109.069
191.321
202.733
1040.252
7.5 7.0 6.5 6.0 5.5 5.0 4.5 4.0 3.5 3.0 2.5 2.0 1.5 1.0 0.5
ppm

**FIG. 15**

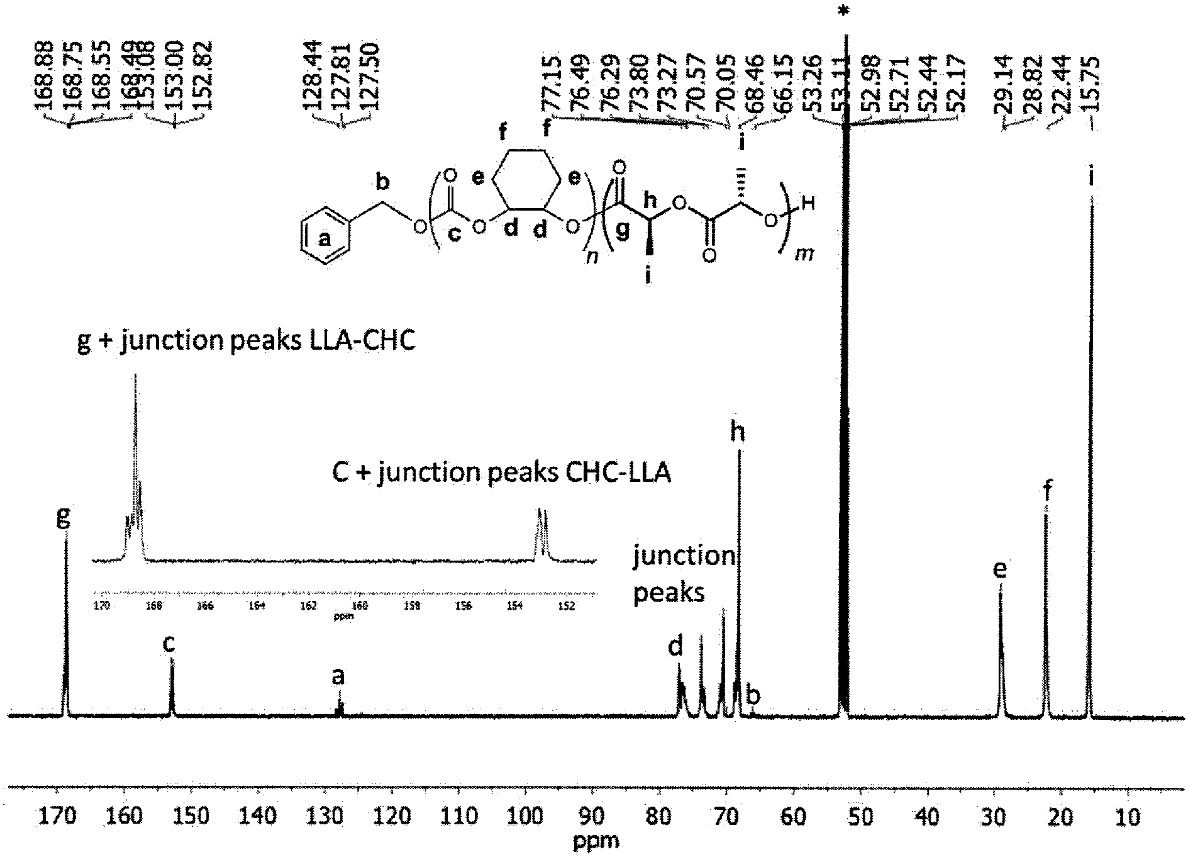
g + junction peaks LLA-CHC
C + junction peaks CHC-LLA
junction peaks
ppm

FIG. 16

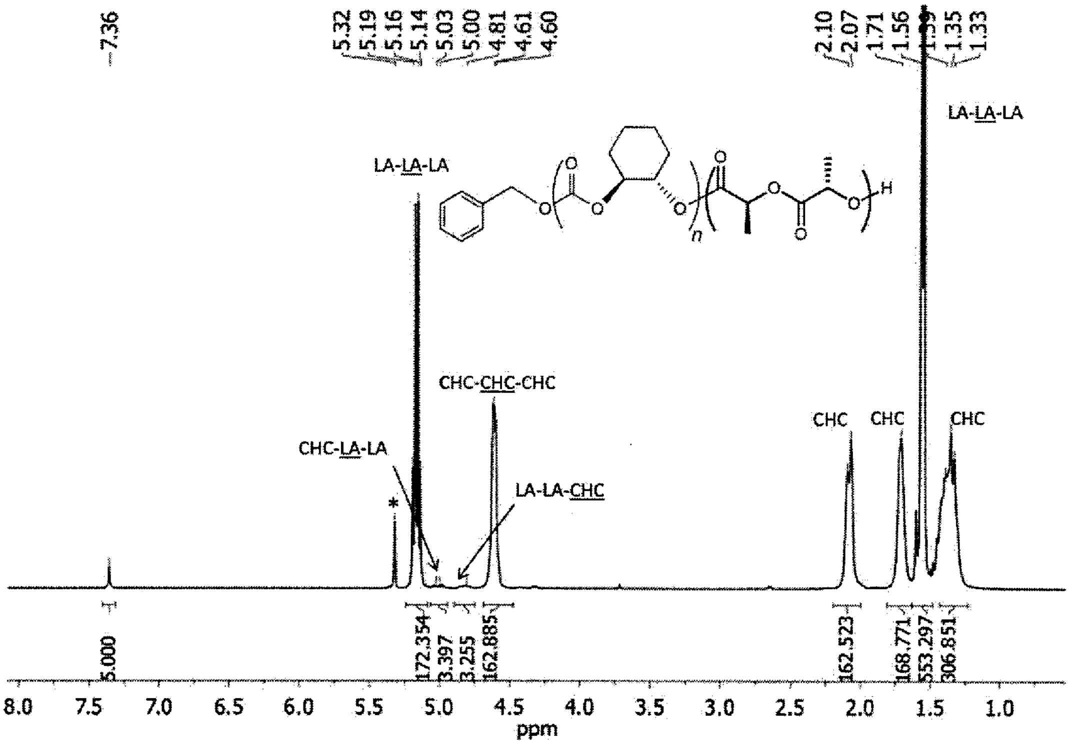
LA-LA-LA
CHC-CHC-CHC
CHC-LA-LA
LA-LA-CHC
CHC
CHC
CHC
LA-LA-LA
ppm

FIG. 17

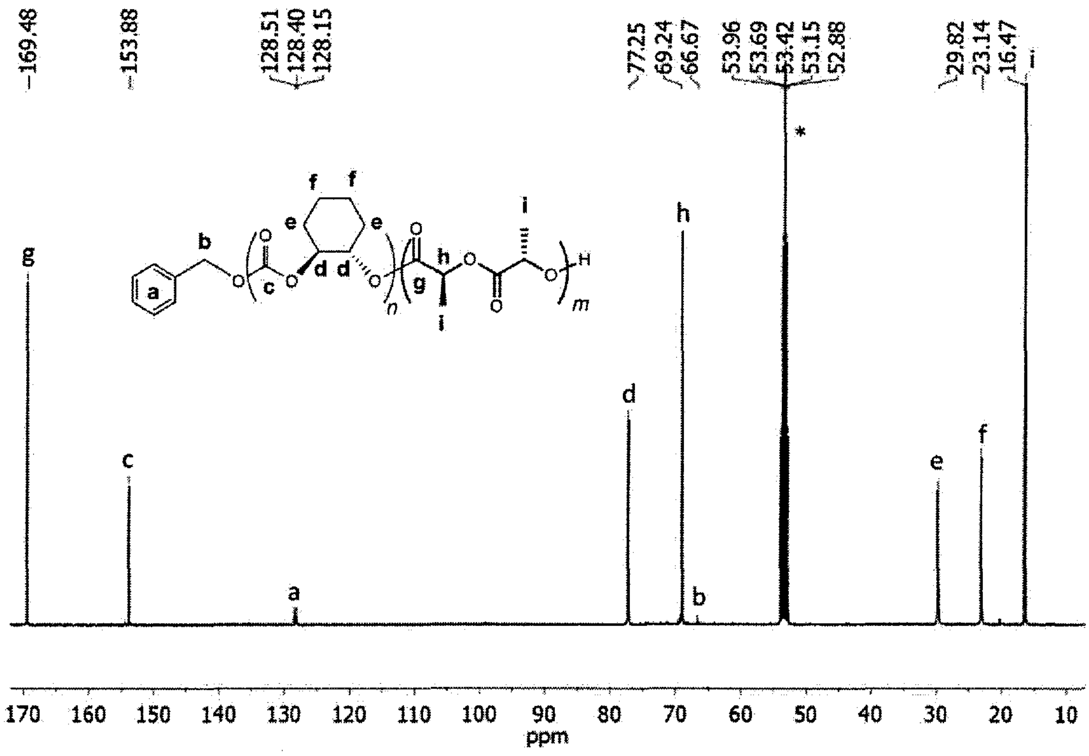

169.48
153.88
128.51
128.40
128.15
77.25
69.24
66.67
53.96
53.69
53.42
53.15
52.88
29.82
23.14
16.47
170 160 150 140 130 120 110 100 90 80 70 60 50 40 30 20 10
ppm


FIG. 18

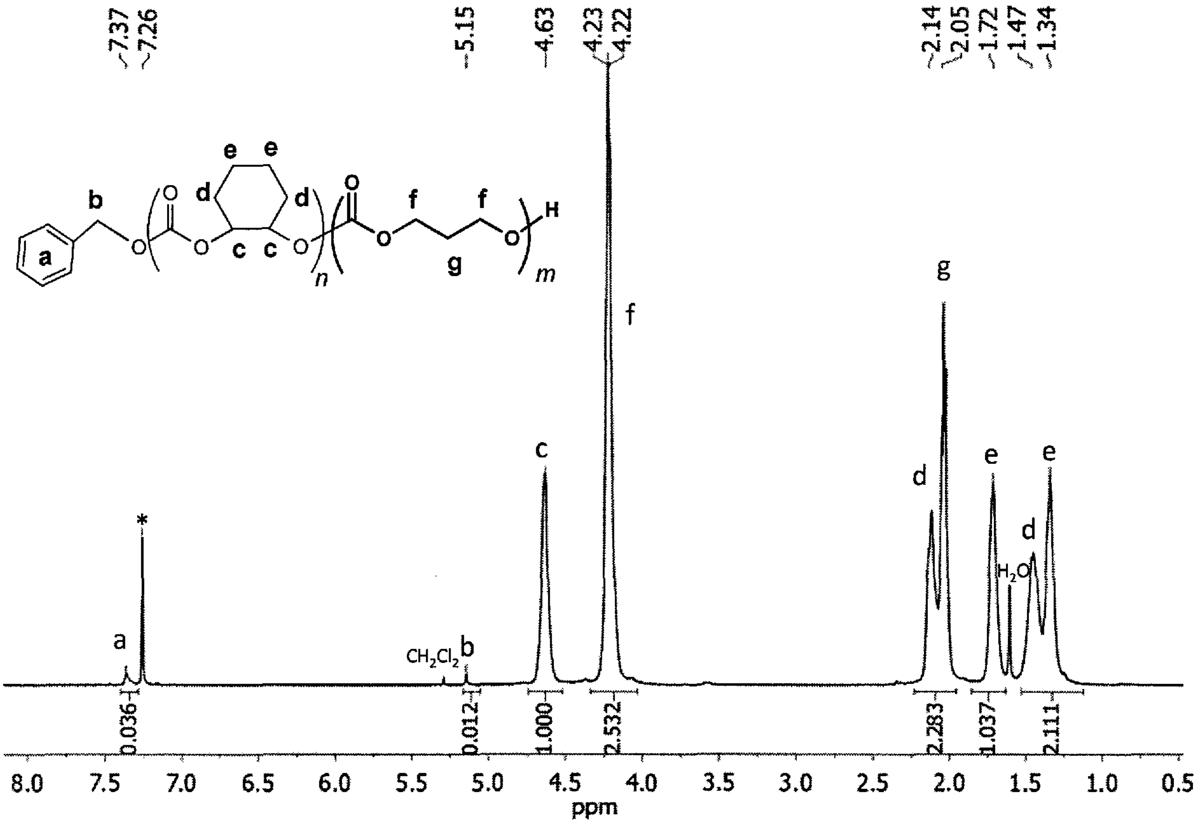

7.37
7.26
5.15
4.63
4.23
4.22
2.14
2.05
1.72
1.47
1.34
$CH_2Cl_2$
$H_2O$
0.036
0.012
1.000
2.532
2.283
1.037
2.111
8.0 7.5 7.0 6.5 6.0 5.5 5.0 4.5 4.0 3.5 3.0 2.5 2.0 1.5 1.0 0.5
ppm


FIG. 19

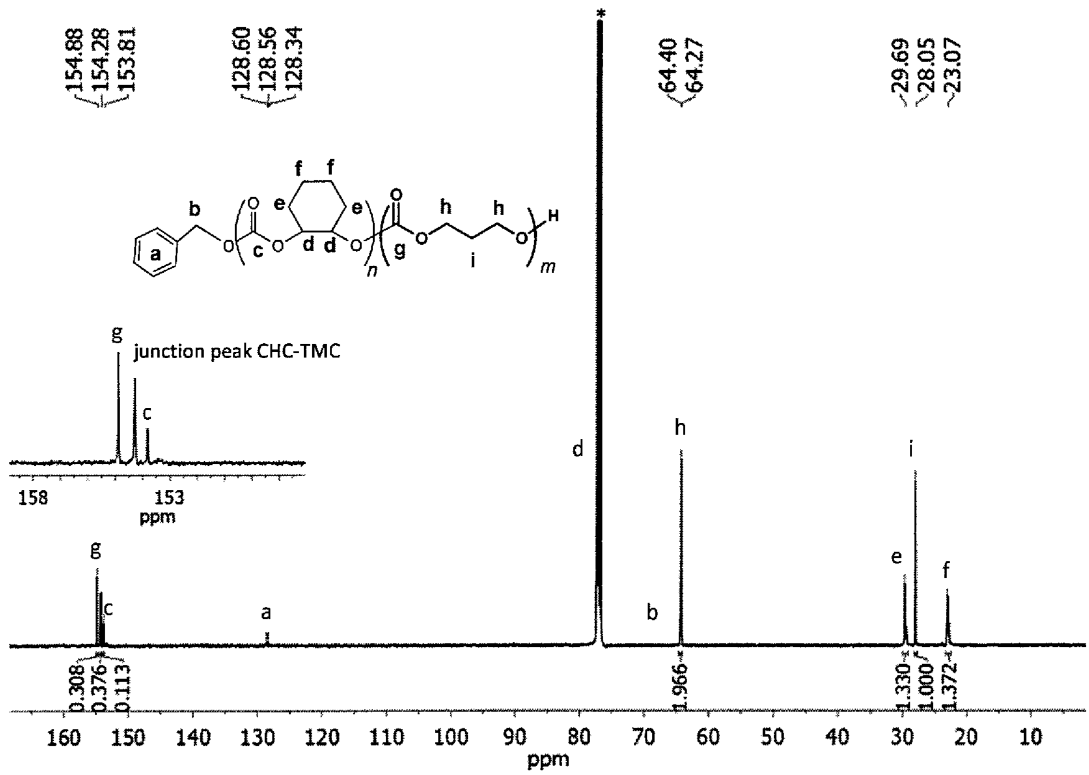
154.88
154.28
153.81
128.60
128.56
128.34
64.40
64.27
29.69
28.05
23.07
junction peak CHC-TMC
158
153
ppm
0.308
0.376
0.113
1.966
1.330
1.000
1.372
160 150 140 130 120 110 100 90 80 70 60 50 40 30 20 10
ppm

**FIG. 20**

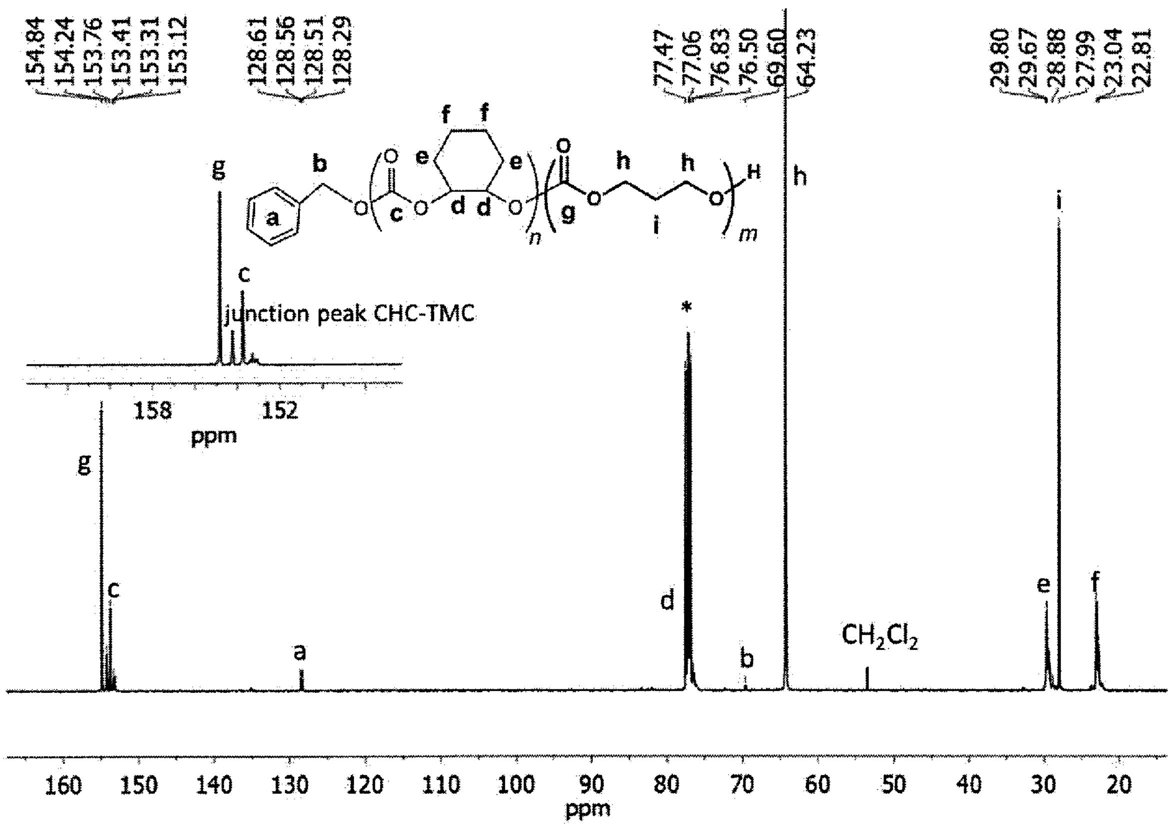
154.84
154.24
153.76
153.41
153.31
153.12
128.61
128.56
128.51
128.29
77.47
77.06
76.83
76.50
69.60
64.23
29.80
29.67
28.88
27.99
23.04
22.81
junction peak CHC-TMC
158
152
ppm
$CH_2Cl_2$
160 150 140 130 120 110 100 90 80 70 60 50 40 30 20
ppm

**FIG. 21**

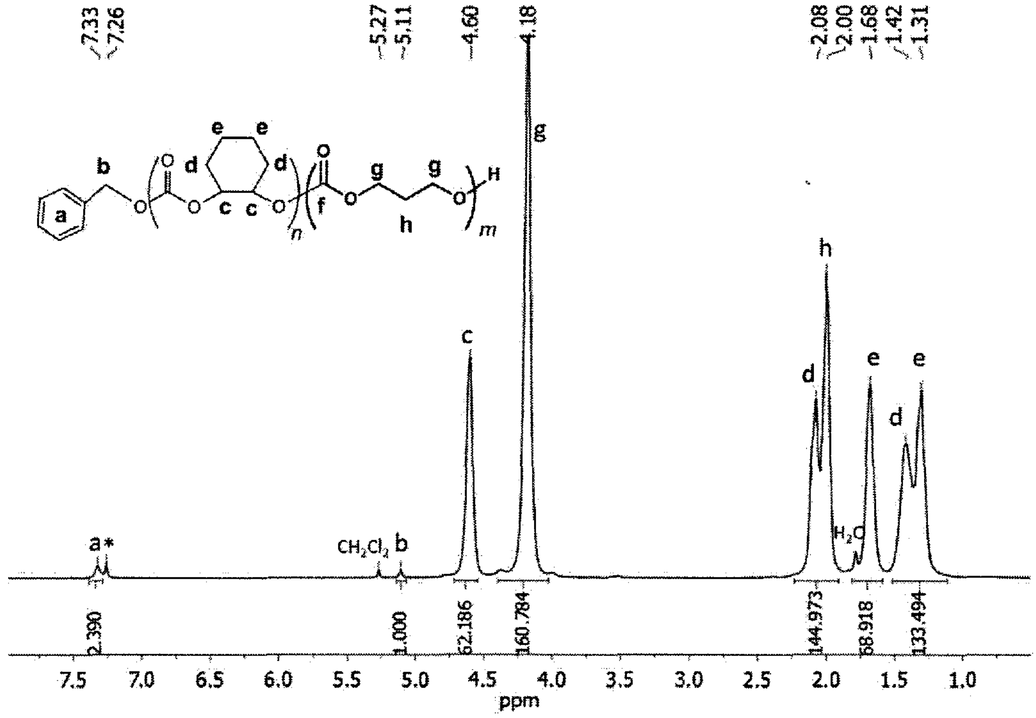
7.33
7.26
5.27
5.11
4.60
4.18
2.08
2.00
1.68
1.42
1.31
CH2Cl2
H2O
2.390
1.000
62.186
160.784
144.973
68.918
133.494
7.5 7.0 6.5 6.0 5.5 5.0 4.5 4.0 3.5 3.0 2.5 2.0 1.5 1.0
ppm

FIG. 22

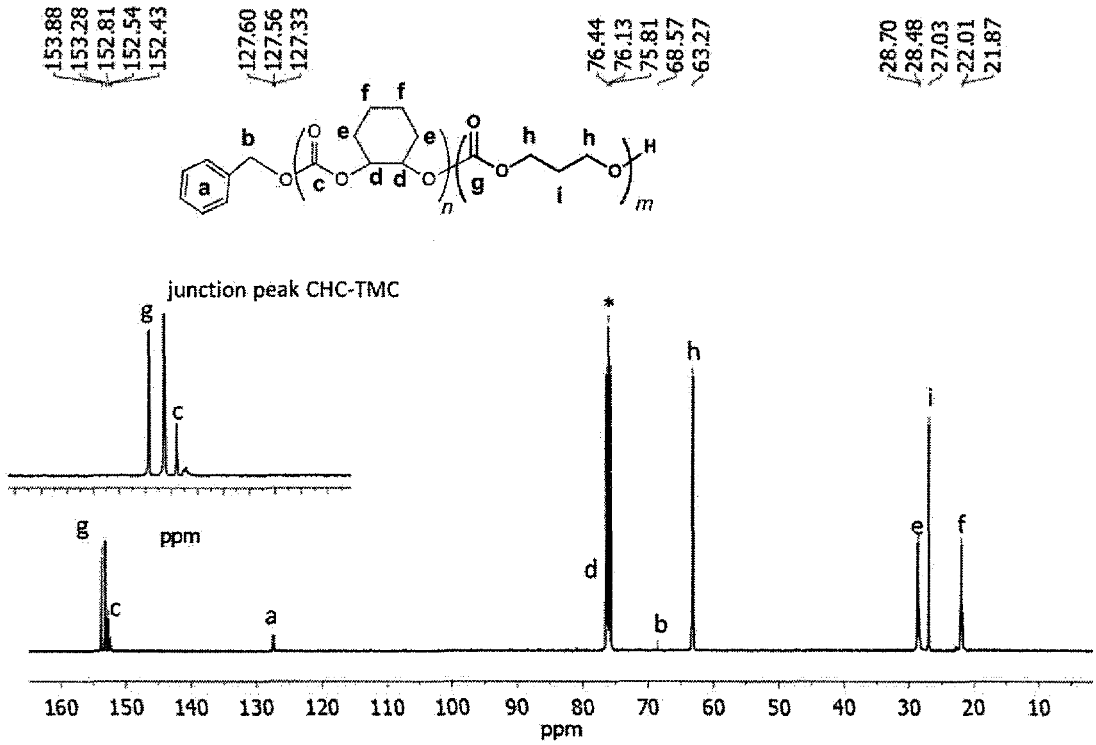
153.88
153.28
152.81
152.54
152.43
127.60
127.56
127.33
76.44
76.13
75.81
68.57
63.27
28.70
28.48
27.03
22.01
21.87
junction peak CHC-TMC
ppm
160 150 140 130 120 110 100 90 80 70 60 50 40 30 20 10
ppm

FIG. 23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PAQUETTE, LEO A.** Principles of Modern Heterocyclic Chemistry. W.A. Benjamin, 1968
- The Chemistry of Heterocyclic Compounds, A series of Monographs. John Wiley & Sons, 1950
- **KATRITZKY, ALAN R. ; REES, C.W. ; SCRIVEN, E.** Comprehensive Heterocyclic Chemistry. Pergamon Press, 1996
- *J. Am. Chem. Soc.,* 1960, vol. 82, 5566
- **M FÈVRE ; J VIGNOLLE ; Y GNANOU ; D TATON.** Organocatalyzed Ring-Opening Polymerizations. Elsevier B.V, 2012
- *Chem. Soc. Rev.,* 2013, vol. 42, 2142
- **NOZAKI, K. ; NAKANO, K. ; HIYAMA, T.** *J. Am. Chem. Soc.,* 1999, vol. 121, 11008-11009
- **CHAMBERLAIN B. M. ; CHENG M. ; MOORE D. R. ; OVITT T. M. ; LOBKOVSKY E. B. ; COATES G. W.** *J. Am; Chem. Soc.,* 2001, vol. 123, 3229-3230
- **WILLIAMS C. K. ; BREYFOGLE L. E. ; CHOI S. K. ; NAM W. ; YOUNG V. G. ; HILLMEYER M. A. ; TOLMAN W. B.** *J. Am. Chem. Soc.,* 2003, vol. 125, 11350-11359
- **BOUYAHYI M ; AJELLAL N ; KIRILLOV E ; THOMAS CM ; CARPENTIER J-F.** Exploring Electronic versus Steric Effects in Stereoselective Ring-Opening Polymerization of Lactide and β-Butyrolactone with Amino-alkoxy-bis(phenolate)-Yttrium Complexes. *Chem Eur J.,* 2011, vol. 17, 1872-1883
- **KRAMER JW ; TREITLER DS ; DUNN EW ; CASTRO PM ; ROISNEL T ; THOMAS CM ; COATES GW.** Polymerization of Enantiopure Monomers Using Syndiospecific Catalysts: A New Approach to Sequence Control in Polymer Synthesis. *J Am Chem Soc.,* 2009, vol. 131, 16042-16044